# EUROPÄISCHE PATENTSCHRIFT

(11) **EP 0 675 172 B1**
(45) Veröffentlichungstag und Bekanntmachung des Hinweises auf die Patenterteilung: **11.04.2001**
(21) Anmeldenummer: 95100784.8
(22) Anmeldetag: 20.01.1995
(51) Int. Cl.: C09B 62/08, C09B 62/507

(54) **Wasserlösliche Azoverbindungen, Verfahren zu ihrer Herstellung und ihre Verwendung als Farbstoffe**
Water-solable azo compounds, process for their preparation and their use as dyestuffs
Composés azoiques solables dans l'eau, procédé pour leur préparation et leur utilisation comme colorants

(30) Priorität: 04.02.1994 DE 4403395; 02.12.1994 DE 4442947
(43) Veröffentlichungstag der Anmeldung: 04.10.1995
(73) Patentinhaber: DyStar Textilfarben GmbH & Co. Deutschland KG, 60318 Frankfurt am Main (DE)
(72) Erfinder: Schumacher, Christian, Dr., D-65929 Frankfurt am Main (DE); Russ, Werner Hubert, Dr., D-65439 Flörsheim (DE)

(56) Entgegenhaltungen:
- EP-A- 0 112 285
- EP-A- 0 493 759
- SZADOWSKI J ET AL: "DIRECT DYES CONTAINING CYCLIC AMIDE GROUPS" DYES AND PIGMENTS, Bd. 21, Nr. 2, 1.Januar 1993, Seiten 123-133, XP000372871
- CHEMICAL ABSTRACTS, vol. 112, no. 2, 8.Januar 1990 Columbus, Ohio, US; abstract no. 8700e, N.M.NAIK ET AL.: "reactive disperse dyes..." Seite 59; XP000154192 & J. INDIAN CHEM. SOC., Bd. 66, Nr. 7, 1989, Seiten 495-497,

## Beschreibung

Die Erfindung liegt auf dem technischen Gebiet der faserreaktiven Farbstoffe.

Die Aufgabe der vorliegenden Erfindung bestand darin, neue faserreaktive Farbstoffe aufzufinden, die den erhöhten Anforderungen der Technik und der Qualität genügen und Färbungen mit brillanten Farbtönen und sehr guten Gebrauchsechtheiten bei hohem Fixiergrad liefern.

Mit der vorliegenden Erfindung wurden neue Azoverbindungen mit solchen guten faserreaktiven Farbstoffeigenschaften gefunden. Die neuen Azoverbindungen entsprechen der allgemeinen Formel (1) 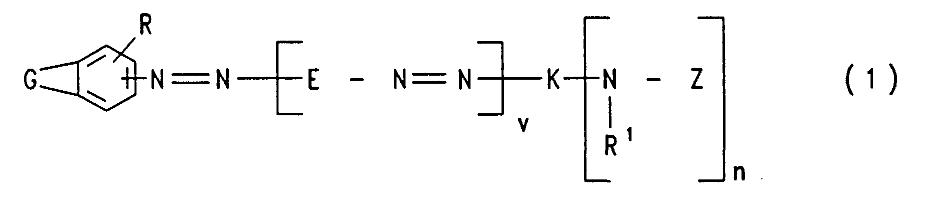 in welcher bedeuten:
- G: bildet zum Benzolring den Rest eines von olefinischen Doppelbindungen freien Heterocyclus, der mindestens eine Carbonamidgruppe der Formel -CO-N(R¹⁰)- enthält, in welcher R¹⁰ Wasserstoff, Alkyl von 1 bis 4 C-Atomen, wie Methyl und Ethyl, oder gegebenenfalls durch 1 bis 3 Substituenten aus der Gruppe Sulfo, Carboxy, Alkoxy von 1 bis 4 C-Atomen, wie Methoxy, Alkyl von 1 bis 4 C-Atomen, wie Methyl, Halogen, wie Chlor, Cyano, Nitro und Amino substituiertes Aryl von 6 bis 10 C-Atomen, wie Phenyl oder Naphthyl, ist, und der gegebenenfalls 1 oder 2 weitere Heterogruppen aus der Reihe -O- , -S- und -N(R¹⁰)- mit R¹⁰ der oben genannten Bedeutung besitzt, und ist bevorzugt eine Gruppe der Formel (2a), (2b), (2c), (2d), (2e), (2f), (2g) oder (2h) 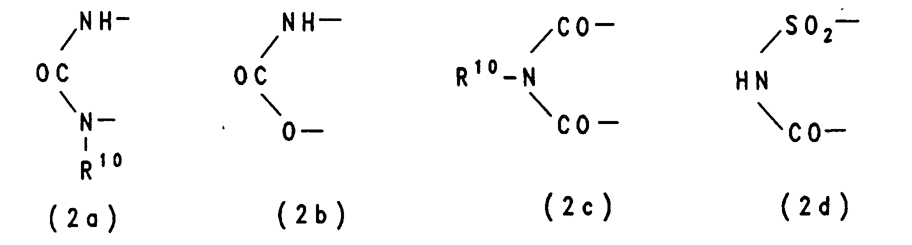 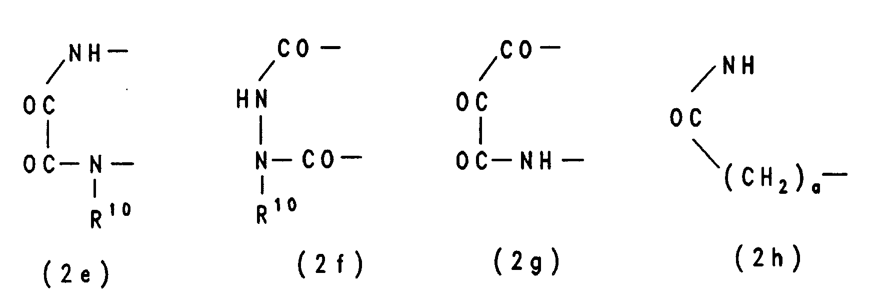 in welchem R¹⁰ die obengenannte Bedeutung hat und in Formel (2h) der Index a für die Zahl 1 oder 2 steht;
- R: ist Wasserstoff, Alkyl von 1 bis 4 C-Atomen, wie Ethyl und insbesondere Methyl, Alkoxy von 1 bis 4 C-Atomen, wie Ethoxy und insbesondere Methoxy, Halogen, wie Chlor oder Brom, oder Sulfo, bevorzugt Wasserstoff;
- R¹: ist Wasserstoff, Alkyl von 1 bis 4 C-Atomen, wie Ethyl und insbesondere Methyl, oder Alkyl von 1 bis 4 C-Atomen, wie insbesondere Ethyl, das durch Hydroxy, Cyano, Alkoxy von 1 bis 4 C-Atomen, wie Ethoxy und Methoxy, Carboxy, Sulfo, Sulfato oder Phosphato substituiert ist, oder ist Phenyl oder Naphthyl, die beide durch 1, 2 oder 3 Substituenten aus der Gruppe Halogen, wie Chlor und Brom, Hydroxy, Cyano, Alkoxy von 1 bis 4 C-Atomen, wie Ethoxy und insbesondere Methoxy, Alkyl von 1 bis 4 C-Atomen, wie Ethyl und insbesondere Methyl, Alkoxycarbonyl von 2 bis 5 C-Atomen, wie Ethoxycarbonyl und Methoxycarbonyl, Carboxy, Sulfamoyl, Sulfo und Alkylsulfonyl mit einem Alkylrest von 1 bis 4 C-Atomen, wie Methylsulfonyl und Ethylsulfonyl, hiervon bevorzugt Alkoxy von 1 bis 4 C-Atomen, Alkyl von 1 bis 4 C-Atomen, Carboxy und Sulfo, substituiert sein können;
- E: ist der bivalente, von der Aminogruppe freie Rest einer kupplungsfähigen und diazotierbaren Verbindung aus der Anilin- oder Naphthylamin-Reihe;
- v: steht für die Zahl Null, 1 oder 2, bevorzugt Null oder 1 und insbesondere bevorzugt Null;
- K: ist der bivalente, von der Aminogruppe freie Rest einer Kupplungskomponente der Anilin- oder Naphthylamin-Reihe oder der bivalente Rest einer Kupplungskomponente der heterocyclischen Reihe;
- n: ist die Zahl 1, 2, 3 oder 4, bevorzugt 1 oder 2 und insbesondere bevorzugt 1;
- Z: ist der faserreaktive Rest einer faserreaktiven Gruppierung oder Gruppe -N(R¹)-Z, wobei im Falle von n größer als 1 die Reste -N(R¹)-Z voneinander verschiedene Bedeutungen haben können;
die Verbindungen der Formel (1) besitzen mindestens eine, bevorzugt mehrere, wie zwei, drei, vier oder fünf, Sulfogruppen.

Bevorzugt ist der Rest R¹⁰ Methyl, Ethyl, Phenyl, Monosulfophenyl, Disulfophenyl, Trisulfophenyl, Naphthyl, Monosulfonaphthyl, Disulfonaphthyl oder Trisulfonaphthyl und insbesondere bevorzugt Wasserstoff.
Von den oben genannten Resten der allgemeinen Formeln (2a) bis (2h) sind als bevorzugt die der Formeln (2a), (2b) und (2e) zu nennen.

Bevorzugt ist Z ein Rest der allgemeinen Formel (3) 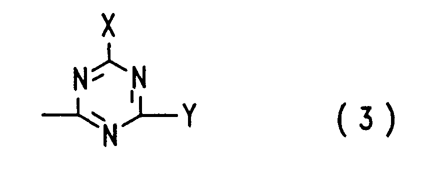 in welcher bedeuten:
- X: ist Halogen, wie Chlor und Fluor, oder eine Gruppe der allgemeinen Formel (4) 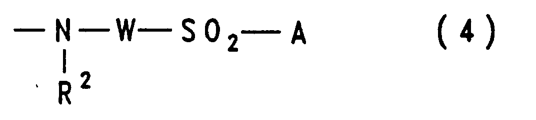 in welcher
R² Wasserstoff, Alkyl von 1 bis 4 C-Atomen, wie Ethyl und insbesondere Methyl, oder Alkyl von 1 bis 4 C-Atomen, wie insbesondere Ethyl, ist, das durch Hydroxy, Cyano, Alkoxy von 1 bis 4 C-Atomen, wie Ethoxy und Methoxy, Carboxy, Sulfo, Sulfato oder Phosphato substituiert ist, oder Phenyl oder Naphthyl ist, die beide durch 1, 2 oder 3 Substituenten aus der Gruppe Halogen, wie Chlor und Brom, Hydroxy, Cyano, Alkoxy von 1 bis 4 C-Atomen, wie Ethoxy und insbesondere Methoxy, Alkyl von 1 bis 4 C-Atomen, wie Ethyl und insbesondere Methyl, Alkoxycarbonyl von 2 bis 5 C-Atomen, wie Ethoxycarbonyl und Methoxycarbonyl, Carboxy, Sulfamoyl, Sulfo und Alkylsulfonyl mit einem Alkylrest von 1 bis 4 C-Atomen, wie Methylsulfonyl und Ethylsulfonyl, hiervon bevorzugt Alkoxy von 1 bis 4 C-Atomen, Alkyl von 1 bis 4 C-Atomen, Carboxy und Sulfo, substituiert sein können,
W Alkylen von 2 bis 4 C-Atomen, bevorzugt von 2 oder 3 C-Atomen, wie 1,2-Ethylen und 1,3-Propylen, ist oder Alkylen von 3 bis 6 C-Atomen, bevorzugt von 4 C-Atomen, ist, das durch 1 oder 2 Heterogruppen aus der Gruppe der Formeln -O- , -NH- , -SO₂- , -CO- und -N(R¹⁰)- mit R¹⁰ der obengenannten Bedeutung unterbrochen ist, wie beispielsweise Gruppen der Formeln -(CH₂)₂-O-(CH₂)₂- und -(CH₂)₂-NH-(CH₂)₂- , oder W Phenylen oder Naphthylen bedeutet, die beide durch 1, 2 oder 3 Substituenten aus der Gruppe Halogen, wie Chlor und Brom, Hydroxy, Cyano, Nitro, Alkoxy von 1 bis 4 C-Atomen, wie Ethoxy und insbesondere Methoxy, Alkyl von 1 bis 4 C-Atomen, wie Ethyl und insbesondere Methyl, Alkoxycarbonyl von 2 bis 5 C-Atomen, wie Ethoxycarbonyl und Methoxycarbonyl, Carboxy, Sulfamoyl und Sulfo, bevorzugt hiervon Alkoxy von 1 bis 4 C-Atomen, Alkyl von 1 bis 4 C-Atomen, Carboxy und Sulfo, substituiert sein können, oder Phenylenalkylen oder Alkylenphenylen mit einem Alkylenrest von jeweils 1 bis 4 C-Atomen, wie insbesondere Ethylen, ist und
A Vinyl ist oder Ethyl bedeutet, das in β-Stellung durch einen mittels Alkali unter Bildung der Vinylgruppe abspaltbaren Substituenten substituiert ist;
Y ist Chlor, Cyanoamino oder eine Gruppe der allgemeinen Formel (5) 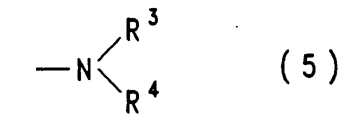 in welcher
R³ Wasserstoff, Alkyl von 1 bis 4 C-Atomen, wie Ethyl und Methyl, oder Alkyl von 1 bis 4 C-Atomen, wie insbesondere Ethyl, ist, das durch Halogen, wie Chlor und Brom, Hydroxy, Cyano, Alkoxy von 1 bis 4 C-Atomen wie Ethoxy und Methoxy, Alkoxycarbonyl von 2 bis 5 C-Atomen, wie Ethoxycarbonyl und Methoxycarbonyl, Carboxy, Sulfo, Sulfato oder Phosphato substituiert ist, oder eine Gruppe der Formel -W-SO₂-A mit W und A einer der obengenannten Bedeutungen ist und
R⁴ Wasserstoff, Alkyl von 1 bis 4 C-Atomen, wie Ethyl und Methyl, oder Alkyl von 1 bis 4 C-Atomen, wie insbesondere Ethyl, ist, das durch Halogen wie Chlor und Brom, Hydroxy, Cyano, Alkoxy von 1 bis 4 C-Atomen, wie Ethoxy und Methoxy, Alkoxycarbonyl von 2 bis 5 C-Atomen, wie Ethoxycarbonyl und Methoxycarbonyl, Carboxy, Sulfo, Sulfato oder Phosphato substituiert ist, oder Cycloalkyl von 5 bis 8 C-Atomen, wie Cyclohexyl, oder eine Gruppe der Formel -W-SO₂-A mit W und A einer der obengennanten Bedeutungen ist oder Phenyl oder Naphthyl ist, die beide durch 1, 2 oder 3 Substituenten aus der Gruppe Halogen, wie Chlor und Brom, Hydroxy, Cyano, Alkoxy von 1 bis 4 C-Atomen, wie Ethoxy und insbesondere Methoxy, Alkyl von 1 bis 4 C-Atomen, wie Ethyl und insbesondere Methyl, Alkoxycarbonyl von 2 bis 5 C-Atomen, wie Ethoxycarbonyl und Methoxycarbonyl, Carboxy, Sulfamoyl, Sulfo und Alkylsulfonyl mit einem Alkylrest von 1 bis 4 C-Atomen, wie Ethylsulfonyl und Methylsulfonyl, substituiert sein können, oder
R³ und R⁴ gemeinsam einen Alkylenrest von 3 bis 6 C-Atomen oder einen durch eine Gruppe -NH- , -O- , -CO- , -S- , -SO₂- oder -N(R⁵)- (worin R⁵ durch Sulfo oder Sulfato substituiertes Alkyl von 1 bis 4 C-Atomen, wie Ethyl, ist) unterbrochenen Alkylenrest von 3 bis 6 C-Atomen darstellen, die zusammen mit dem N-Atom einen heterocyclischen Rest bilden, wie den Pyrrolidino-, Morpholino-, Piperidino- oder Piperazino-Rest.

Weitere bevorzugte faserreaktive Reste Z sind solche aus der halogensubstituierten Pyrimidinreihe, wie ein Difluorchlorpyrimidino-, Trichlorpyrimidino-, Methylsulfonyl-chlor-pyrimidino- und Methyl-fluor-chlorpyrimidino-Rest, und desweiteren der Rest des Dichlorchinoxalins.

In den allgemeinen Formeln (1) bis (5) sowie in den nachfolgenden allgemeinen Formeln können die einzelnen Formelglieder, sowohl verschiedener als auch gleicher Bezeichnung innerhalb einer allgemeinen Formel, im Rahmen ihrer Bedeutung zueinander gleiche oder voneinander verschiedene Bedeutungen haben.

Alkalisch eliminierbare Substituenten, die in ß-Stellung der Ethylgruppe von A stehen, sind beispielsweise Halogenatome, wie Brom und Chlor, Estergruppen organischer Carbon- und Sulfonsäuren, wie von Alkylcarbonsäuren, gegebenenfalls substituierter Benzolcarbonsäuren und gegebenenfalls substituierter Benzolsulfonsäuren, wie die Gruppen Alkanoyloxy von 2 bis 5 C-Atomen, hiervon insbesondere Acetyloxy, Benzoyloxy, Sulfobenzoyloxy, Phenylsulfonyloxy und Toluylsulfonyloxy, des weiteren saure Estergruppen anorganischer Säuren, wie der Phosphorsäure, Schwefelsäure und Thioschwefelsäure (Phosphato-, Sulfato- und Thiosulfatogruppen), ebenso Dialkylaminogruppen mit Alkylgruppen von jeweils 1 bis 4 C-Atomen, wie Dimethylamino und Diethylamino. Bevorzugt ist A ß-Chlorethyl oder Vinyl und insbesondere bevorzugt ß-Sulfatoethyl.

Die Gruppen "Sulfo", "Carboxy", "Thiosulfato", "Phosphato" und "Sulfato" schließen sowohl deren Säureform als auch deren Salzform ein. Demgemäß bedeuten Sulfogruppen Gruppen entsprechend der allgemeinen Formel -SO₃M , Carboxygruppen Gruppen entsprechend der allgemeinen Formel -COOM, Thiosulfatogruppen Gruppen entsprechend der allgemeinen Formel -S-SO₃M , Phosphatogruppen Gruppen entsprechend der allgemeinen Formel -OPO₃M₂ und Sulfatogruppen Gruppen entsprechend der allgemeinen Formel -OSO₃M , in welchen
- M: ein Wasserstoffatom oder ein Alkalimetall, wie Natrium, Kalium oder Lithium, oder ein anderes salzbildendes Metall ist.

Bevorzugt ist R¹ Wasserstoff, Methyl oder Ethyl, insbesondere Wasserstoff. R² ist bevorzugt Wasserstoff, Methyl, Ethyl oder Phenyl. Reste der Formel (5) sind bevorzugt Phenylamino, das durch 1, 2 oder 3, bevorzugt 1 oder 2, Substituenten aus der Gruppe Halogen, wie Chlor und Brom, Hydroxy, Cyano, Ethoxy, Methoxy, Methyl, Ethyl, Carboxy und Sulfo, bevorzugt Sulfo, substituiert sein kann, oder Naphthylamino, bevorzugt Naphth-2-yl-amino, das durch 1, 2 oder 3 Sulfogruppen substituiert ist, oder ist bevorzugt Morpholino oder eine Gruppe der oben angegebenen allgemeinen Formel (4) oder eine Gruppe der allgemeinen Formel (4a) 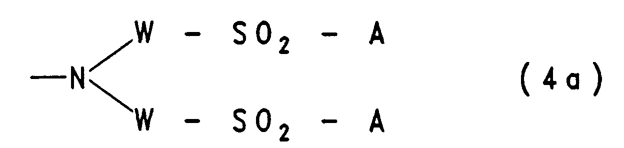 in welchen W und A die oben genannten, insbesondere bevorzugten, Bedeutungen haben.

Aromatische Reste E einer kupplungsfähigen und diazotierbaren Verbindung der allgemeinen Formel H-E-NH₂ sind beispielsweise solche der allgemeinen Formeln (6a), (6b) und (6c) 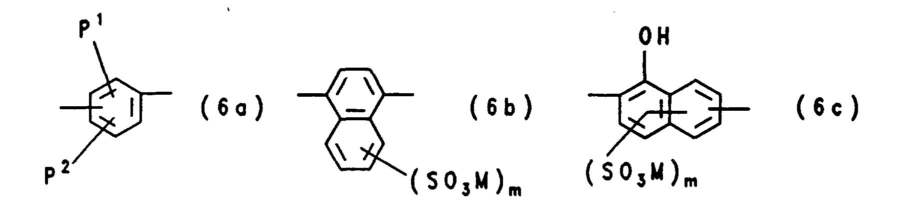 in welchen
- M: die oben angegebenen Bedeutungen hat,
- m: die Zahl Null, 1 oder 2 ist (wobei diese Gruppe im Falle von m gleich Null Wasserstoff bedeutet),
- P¹: Wasserstoff, Alkyl von 1 bis 4 C-Atomen, wie Ethyl und Methyl, Alkoxy von 1 bis 4 C-Atomen, wie Ethoxy und Methoxy, Cyano, Sulfo, Carboxy, Hydroxy, Fluor, Chlor, Brom oder Trifluormethyl ist und
- P²: Wasserstoff, Alkyl von 1 bis 4 C-Atomen, wie Methyl oder Ethyl, Alkoxy von 1 bis 4 C-Atomen, wie Methoxy und Ethoxy, Chlor, Alkanoylamino von 2 bis 5 C-Atomen, wie Acetylamino und Propionylamino, Amino, Benzoylamino, Ureido, Phenylureido, Alkylureido mit 1 bis 4 C-Atomen im Alkylrest, Phenylsulfonyl oder Alkylsulfonyl von 1 bis 4 C-Atomen ist.

Reste -K-N(R¹ )-Z von Kupplungskomponenten der allgemeinen Formel H-K-N(R¹)-Z (bzw. davon abgeleitete Reste der allgemeinen Formel H-K-N(R¹)H , in die der faserreaktive Rest Z nachträglich eingeführt werden muß) sind beispielsweise Reste der allgemeinen Formeln (7a) bis (7f) 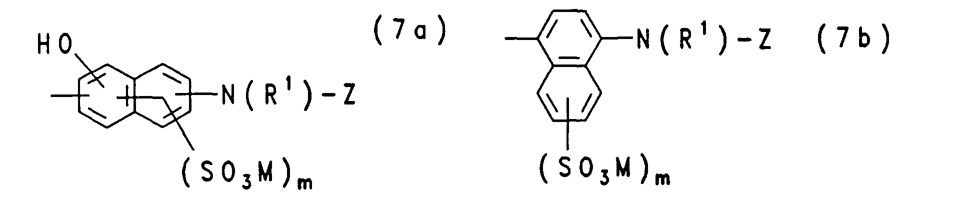 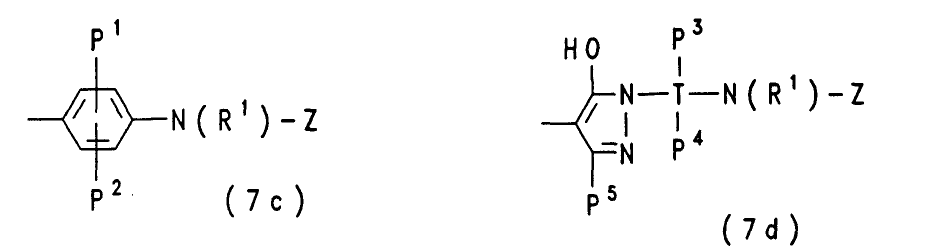 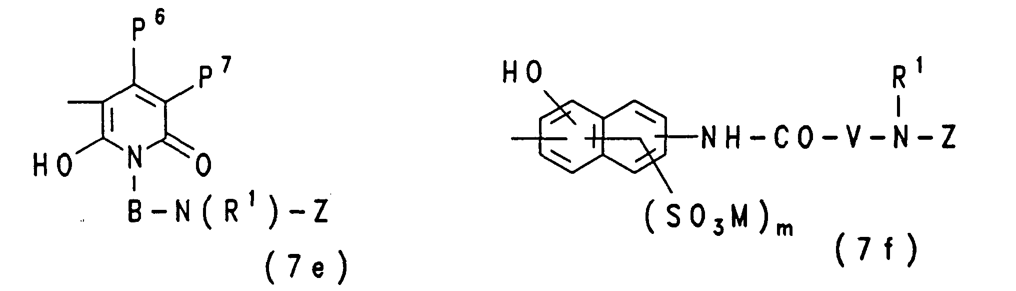 in welchen bedeuten:
P¹, P², M, m, R¹ und Z haben die oben angegebenen, insbesondere bevorzugten, Bedeutungen;

in Formel (7a) steht die freie Bindung, die zur Azogruppe führt, in ortho-Stellung zur Hydroxygruppe an den aromatischen Kern gebunden;
- P³: ist Wasserstoff, Alkyl von 1 bis 4 C-Atomen, wie Ethyl und insbesondere Methyl, Alkoxy von 1 bis 4 C-Atomen, wie Ethoxy und insbesondere Methoxy, Chlor, Brom, Fluor, Sulfo, Carboxy oder Trifluormethyl;
- P⁴: ist Wasserstoff, Alkyl von 1 bis 4 C-Atomen, wie Ethyl und insbesondere Methyl, Alkoxy von 1 bis 4 C-Atomen, wie Ethoxy und insbesondere Methoxy, Chlor oder Sulfo;
- P⁵: ist Wasserstoff, Alkyl von 1 bis 4 C-Atomen, wie Methyl, Cyano, Carboxy, Carbalkoxy von 2 bis 5 C-Atomen, wie Carbomethoxy und Carbethoxy, Carbamoyl oder Phenyl, bevorzugt Methyl, Carboxy, Methoxycarbonyl, Ethoxycarbonyl oder Phenyl und insbesondere Methyl oder Carboxy;
- T: ist ein Benzol- oder Naphthalinring, bevorzugt Benzolring;
- P⁶: ist Wasserstoff oder Alkyl von 1 bis 4 C-Atomen, wie Methyl, oder durch Alkoxy von 1 bis 4 C-Atomen, wie Methoxy, oder Cyano substituiertes Alkyl von 1 bis 4 C-Atomen oder Phenyl, bevorzugt Alkyl von 1 bis 4 C-Atomen oder Phenyl;
- P⁷: ist Wasserstoff, Chlor, Brom, Sulfo, Carbamoyl, Methylsulfonyl, Phenylsulfonyl, Cyano oder Sulfoalkyl von 1 bis 4 C-Atomen, bevorzugt Wasserstoff, Sulfo, Sulfoalkyl mit einem Alkylrest von 1 bis 4 C-Atomen, wie Sulfomethyl, Cyano oder Carbamoyl;
- v: ist Phenylen, bevorzugt meta- oder para-Phenylen, das durch eine Sulfogruppe substituiert sein kann.

Gruppen entsprechend den allgemeinen Formeln (4) und (4a) sind beispielsweise:
N-[γ-(β'-Chlorethylsulfonyl)-propyl]-amino, N-[γ-(β'-Sulfatoethylsulfonyl)-propyl]-amino, N-[γ-(Vinylsulfonyl)-propyl]-amino, N-[β-(β'-Chlorethylsulfonyl)-ethyl]-amino, N-[β-(β'-Sulfatoethylsulfonyl)-ethyl]-amino, N-[β-(Vinylsulfonyl)-ethyl]-amino, N-Methyl-N-[β-(β'-chlorethylsulfonyl)-ethyl]-amino, N-Ethyl-N-[β-(β'-chlorethylsulfonyl)-propyl]-amino, N-n-Propyl-N-[β-(β'-chlorethylsulfonyl)-ethyl]-amino, N-n-Butyl-N-[β-(β'-chlorethylsulfonyl)-ethyl]-amino, N-Carboxymethyl-N-[β-(β'-bromethylsulfonyl)-ethyl]-amino, N-Sulfatomethyl-N-[β-(β'-chlorethylsulfonyl)-ethyl]-amino, N-(β-Carboxyethyl)-N-[β'-(β"-chlorethylsulfonyl)-ethyl]-amino, N-(β-Sulfatoethyl)-N-[β'-(β"-chlorethylsulfonyl)-ethyl]-amino, N-(β-Ethoxyethyl)-N-[β'-(β"-chlorethylsulfonyl)-ethyl]-amino, N-Phenyl-N-[β-(β'-chlorethylsulfonyl)-ethyl]-amino, N-(4-Chlorphenyl)-N-[β-(β'-chlorethylsulfonyl)-ethyl]-amino, N-(2-Methylphenyl)-N-[β-(β'-chlorethylsulfonyl)-ethyl]-amino, N-(4-Methoxyphenyl)-N-[β-(β'-chlorethylsulfonyl)-ethyl]-amino, N-(3-Sulfophenyl)-N-[β-(β'-chlorethylsulfonyl)-ethyl]-amino, N-(4-Sulfophenyl)-N-[β-(β'-chlorethylsulfonyl)-ethyl]-amino, N-(β-Cyanoethyl)-N-[β'-(β"-chlorethylsulfonyl)-ethyl]-amino, N-Phenyl-N-(β-vinylsulfonyl-ethyl)-amino, N-(4-Chlorphenyl)-N-(β-vinylsulfonyl-ethyl)-amino, N-(2-Methylphenyl)-N-(β-vinylsulfonyl-ethyl)-amino, N-(4-Methoxyphenyl)-N-(β-vinylsulfonyl-ethyl)-amino, N-(3-Sulfophenyl)-N-(β-vinylsulfonyl-ethyl)-amino, N-(4-Sulfophenyl)-N-(β-vinylsulfonyl-ethyl)-amino, N-Phenyl-N-(β-(β'-sulfatoethylsulfonyl)-ethyl]-amino, N-(4-Chlorphenyl)-N-[β-(β'-sulfatoethylsulfonyl)-ethyl]-amino, N-(2-Methylphenyl)-N-[β-(β'-sulfatoethylsulfonyl)-ethyl]-amino, N-(4-Methoxyphenyl)-N-[β-(β'-sulfatoethylsulfonyl)-ethyl]-amino, N-(3-Sulfophenyl)-N-[β-(β'-sulfatoethylsulfonyl)-ethyl]-amino, N-(4-Sulfophenyl)-N-[β-(β'-sulfatoethylsulfonyl)-ethyl]-amino, N-Methyl-N-[γ-(β'-chlorethylsulfonyl)-propyl]-amino, N-Ethyl-N-[γ-(β'-chlorethylsulfonyl)-propyl]-amino, N-n-Propyl-N-(γ-(β'-chlorethylsulfonyl)-propyl]-amino, N-n-Butyl-N-[γ-(β'-chlorethylsulfonyl)-propyl]-amino, N-Carboxymethyl-N-[γ-(β'-bromethylsulfonyl)-propyl]-amino, N-Sulfatomethyl-N-[γ-(β'-chlorethylsulfonyl)-propyl]-amino, N-(β-Carboxyethyl)-N-[y'-(β"-chlorethylsulfonyl)-propyl]-amino, N-(β-Sulfatoethyl)-N-[γ'-(β"-chlorethylsulfonyl)-propyl]-amino, N-(β-Sulfatoethyl)-N-[δ'-(β"-chlorethylsulfonyl)-butyl]-amino, N-(β-Ethoxyethyl)-N-[δ'-(β"-chlorethylsulfonyl)-butyl]-amino, N-(β-Ethoxyethyl)-N-[γ'-(β"-chlorethylsulfonyl)-propyl]-amino, N-Phenyl-N-[γ-(β'-chlorethylsulfonyl)-propyl]-amino, N-(4-Chlorphenyl)-N-[γ-(β'-chlorethylsulfonyl)-propyl]-amino, N-(2-Methylphenyl)-N-(γ-(β'-chlorethylsulfonyl)-propyl]-amino, N-(4-Methoxyphenyl)-N-[γ-(β'-chlorethylsulfonyl)-propyl]-amino, N-(3-Sulfophenyl)-N-[γ-(β'-chlorethylsulfonyl)-propyl]-amino, N-(4-Sulfophenyl)-N-[γ-(β'-chlorethylsulfonyl)-propyl]-amino, N-(β-Cyanoethyl)-N-[γ'-(β"-chlorethylsulfonyl)-propyl]-amino, N-Phenyl-N-(γ-vinylsulfonyl-propyl)-amino, N-(4-Chlorphenyl)-N-(γ-vinylsulfonyl-propyl)-amino, N-(2-Methylphenyl)-N-(γ-vinylsulfonyl-propyl)-amino, N-(4-Methoxyphenyl)-N-(γ-vinylsulfonyl-propyl)-amino, N-(3-Sulfophenyl)-N-(γ-vinylsulfonyl-propyl)-amino, N-(4-Sulfophenyl)-N-(γ-vinylsulfonyl-propyl)-amino, N-Phenyl-N-[γ-(β'-sulfatoethylsulfonyl)-propyl]-amino, N-(4-Chlorphenyl)-N-[γ-(β'-sulfatoethylsulfonyl)-propyl]-amino, N-(2-Methylphenyl)-N-[γ-(β'-sulfatoethylsulfonyl)-propyl]-amino, N-(4-Methoxyphenyl)-N-[γ-(β'-sulfatoethylsulfonyl)-propyl]-amino, N-(3-Sulfophenyl)-N-[γ-(β'-sulfatoethylsulfonyl)-propyl]-amino, N-(4-Sulfophenyl)-N-[γ-(β'-sulfatoethylsulfonyl)-propyl]-amino, N-Phenyl-N-[α-carboxy-γ-(β'-chlorethylsulfonyl)-propyl]-amino, N-Phenyl-N-[α-ethoxycarbonyl-γ-(β'-chlorethylsulfonyl)-propyl]-amino, N-Phenyl-N-[α-methoxycarbonyl-γ-(β'-chlorethylsulfonyl)-pröpyl]-amino, N-Phenyl-N-[β-methyl-γ-(β'-chlorethylsulfonyl)-propyl]-amino, N-Phenyl-N-[β-ethyl-γ-(β'-chlorethylsulfonyl)-propyl]-amino, N-Phenyl-N-[δ-(β'-chlorethylsulfonyl)-butyl]-amino, N-Phenyl-N-[ε-(β'-chlorethylsulfonyl)-pentyl]-amino und N-Phenyl-N-[β-(β'-chlorethylsulfonyl)-hexyl]-amino, Bis-N,N-[γ-(β'-chlorethylsulfonyl)-propyl]-amino, Bis-N,N-[γ-(β'-sulfatoethylsulfonyl)-propyl]-amino, Bis-N,N-[γ-(vinylsulfonyl)-propyl]-amino, Bis-N,N-[β-(β'-chlorethylsulfonyl)-ethyl]-amino, Bis-N,N-[β-(β'-sulfatoethylsulfonyl)-ethyl]-amino, Bis-N,N-[β-(vinylsulfonyl)-ethyl]-amino, 4-(β-Sulfatoethylsulfonyl)-phenylamino, 3-(β-Sulfatoethylsulfonyl)-phenylamino, 2-Methoxy-5-(β-sulfatoethylsulfonyl)-phenylamino, 2,5-Dimethoxy-4-(β-sulfatoethylsulfonyl)-phenylamino, 2-Methoxy-5-methyl-4-(β-sulfatoethylsulfonyl)-phenylamino, 2-Sulfo-4-(β-sulfatoethylsulfonyl)-phenylamino, 2-Hydroxy-5-(β-sulfatoethylsulfonyl)-phenylamino, 2-Brom-5-(β-sulfatoethylsulfonyl)-phenylamino, 4-[β-(β'-Sulfatoethylsulfonyl)-ethyl]-phenylamino, 1-Sulfo-6-(β-sulfatoethylsulfonyl)-naphth-2-ylamino, 8-Sulfo-6-(β-sulfatoethylsulfonyl)-naphth-2-ylamino, 6-Sulfo-8-(β-sulfatoethylsulfonyl)-naphth-2-ylamino, 6-(β-Sulfatoethylsulfonyl)-naphth-2-ylamino und 8-(β-Sulfatoethylsulfonyl)-naphth-2-ylamino.

Weitere faserreaktive Reste Z sind beispielsweise: 2,4-Dichlor-1,3,5-triazin-6-yl, 2,4-Difluor-5-chlor-pyrimidin-6-yl, 2,4,5-Trichlor-pyrimidin-6-yl, 2-Methylsulfonyl-5-chlor-4-methyl-pyrimidin-6-yl, 4-Methyl-2-fluor-5-chlorpyrimidin-6-yl, 2,3-Dichlor-chinoxalin-6-carbonyl und 2,4-Difluor-pyrimidin-6-yl, des weiteren Reste entsprechend der allgemeinen Formel (8a) 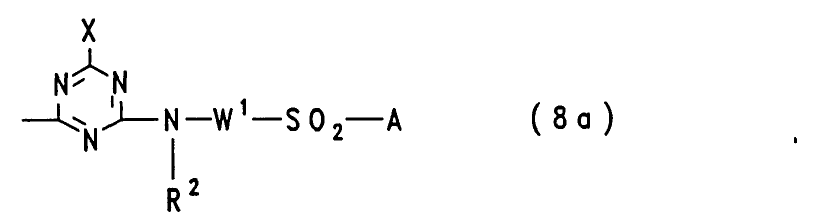 in welcher X Chlor, Fluor oder Cyanoamino ist, A eine der oben genannten, insbesonders bevorzugten Bedeutungen hat, R² Wasserstoff, Ethyl, Methyl, β-Hydroxyethyl, β-Sulfatoethyl, Phenyl, 3-Sulfophenyl oder 4-Sulfophenyl ist und W¹ 1,2-Ethylen, 1,3-Propylen, 2-Methyl-1,2-ethylen, 2-Methyl-1,3-propylen, 1,4-Phenylen, 1,3-Phenylen, 2-Methoxy-1,5-phenylen, 2,5-Dimethoxy-1,4-phenylen, 2-Methoxy-5-methyl-1,4-phenylen, 2-Sulfo-1,4-phenylen, 2-Hydroxy-1,5-phenylen, 2-Brom-1,5-phenylen oder 4-(2'-Eth)-phen-1,1'-ylen ist, wobei in diesen Gruppen die 1-Position mit dem N-Atom verbunden ist, oder 1-Sulfo-naphth-2,6-ylen, 6-Sulfo-naphth-2,8-ylen, Naphth-2,6-ylen und Naphth-2,8-ylen ist, wobei in diesen Gruppen die 2-Position mit dem N-Atom verbunden ist,
weiterhin Reste entsprechend der allgemeinen Formel (8b) 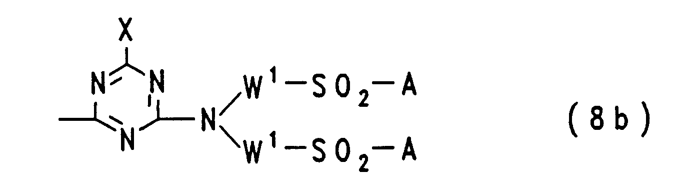 in welcher X und A eine der oben genannten, insbesondere bevorzugten, Bedeutungen haben und W¹ eine der oben genannten Bedeutungen besitzt, bevorzugt 1,3-Propylen oder 1,2-Ethylen ist,
desweiteren Gruppen entsprechend der allgemeinen Formel (8c) 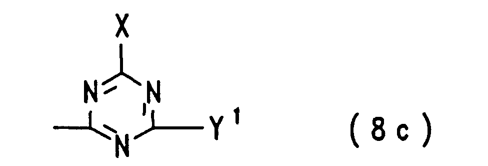 in welcher X Chlor oder Fluor bedeutet und Y¹ Amino, Methylamino, Ethylamino, Dimethylamino, Diethylamino, Bis-(β-hydroxyethyl)-amino, β-Hydroxy-ethylamino, Phenylamino, 3-Sulfo-phenyl-amino, 4-Sulfo-phenyl-amino, 2-Sulfo-phenyl-amino, 2,5-Disulfo-phenylamino, 2,4-Disulfo-phenylamino, 2-Carboxy-phenylamino, 4-Carboxy-phenylamino, 2-Sulfo-4-methoxy-phenylamino, 2-Sulfo-4-methyl-phehylamino, 3-Sulfo-4-methyl-phenylamino, 2-Methylphenylamino, 3-Methyl-phenylamino, 4-Methyl-phenylamino, 2,5-Dimethylphenylamino, 2,4-Dimethyl-phenylamino, 2-Methoxy-phenylamino, 3-Methoxyphenylamino, 4-Methoxy-phenylamino, 2- oder 3- oder 4-Ethoxy-phenylamino, N-Ethyl-phenylamino, N-Methyl-phenylamino, N-(β-Hydroxyethyl)-phenylamino, 2-Chlor-phenylamino, 3- oder 4-Chlor-phenylamino, 2,5-Dichlor-phenylamino, Naphth-2-yl-amino, 1-Sulfo-naphth-2-yl-amino, 3,6,8-Trisulfo-naphth-2-yl-amino, 4,6,8-Trisulfo-naphth-2-yl-amino, 4,8-Disulfo-naphth-2-yl-amino, 1,5-Disulfonaphth-2-yl-amino, N-Morpholino, N-Piperidino, N-Piperazino, N-Pyrrolidino, N'-(β-Sulfatoethyl)-N-piperazino oder N'-(β-Hydroxyethyl)-N-piperazino ist.

Von den erfindungsgemäßen Azoverbindungen der allgemeinen Formel (1) sind insbesondere diejenigen der allgemeinen Formel (1A), (1B), (1C), (1D), (1E), (1F), (1G) und (1H) 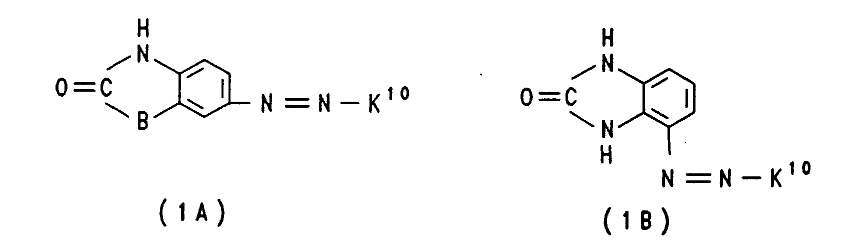 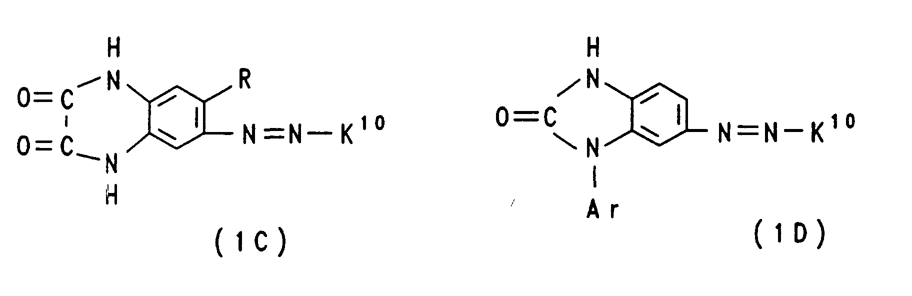 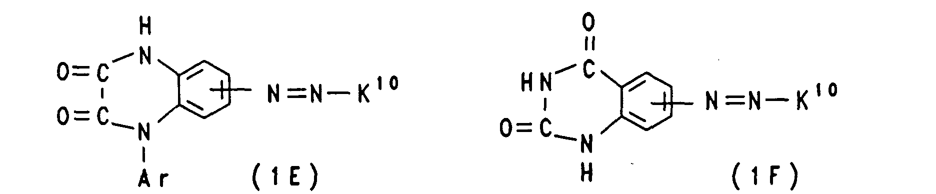 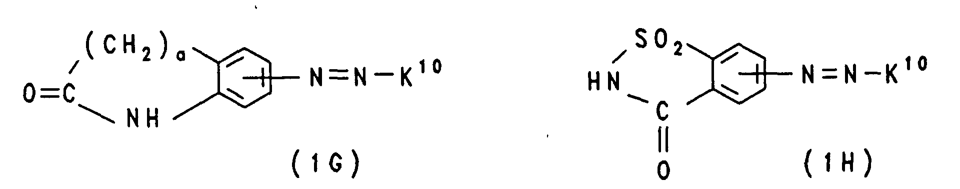 bevorzugt, in welchen R Wasserstoff, Methyl oder Methoxy ist, B für -0- oder -N(R¹⁰)- mit R¹⁰ der obengenannten, insbesondere bevorzugten Bedeutung steht, a die Zahl 1 oder 2 ist, Ar durch 1 oder 2 Sulfogruppen substituiertes Phenyl ist oder durch 1, 2 oder 3 Sulfogruppen substituiertes Naphthyl, hiervon bevorzugt Mono- oder Disulfonaphthyl, bedeutet und der Rest K¹⁰ eine Gruppe der allgemeinen Formel (8A) 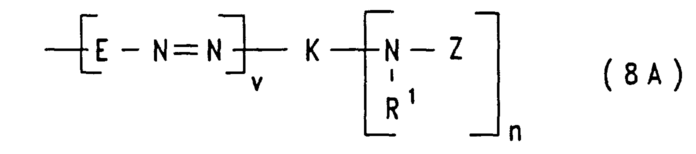 ist, in welchen E, v, K, R¹, Z und n eine der oben genannten, insbesondere bevorzugten, Bedeutungen haben.

Hiervon sind insbesondere diejenigen Azoverbindungen hervorzuheben, die den alligemeinen Formeln (9a), (9b), (9c) und (9d)

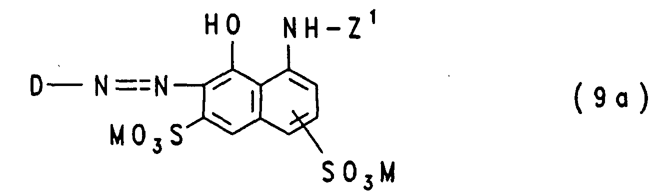

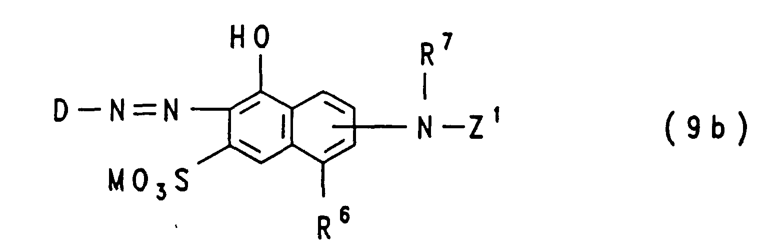

D - N = N - E¹ - NH - Z² (9c)

D - N = N - E¹ - N = N - E² - NH - Z² (9d)

entsprechen, in welchen bedeuten:
- D: ist der aus den Formeln (1A) bis (1H) ersichtliche, vom Rest -N=N-K¹⁰ freie Rest;
- M: hat eine der oben genannten Bedeutungen;
- R⁶: ist Wasserstoff oder Sulfo;
- R⁷: Wasserstoff, Alkyl von 1 bis 4 C-Atomen, wie Methyl oder Ethyl, das durch 1 oder 2 Substituenten aus der Gruppe Hydroxy, Sulfo, Carboxy und Sulfato substituiert sein kann, oder ist Phenyl, das durch 1 , 2 oder 3 Substituenten aus der Gruppe Sulfo und Carboxy substituiert sein kann, und ist bevorzugt Wasserstoff;
die Gruppe -SO₃M steht in Formel (9a) in para- oder bevorzugt in meta-Stellung meta-Stellung zur Gruppe -N(R⁷)-Z¹ und in Formel (9b) in ortho- oder zu R⁶ gebunden;
- E¹: ist 1,4-Phenylen, das durch 1 oder 2 Substituenten aus der Gruppe Sulfo, Methyl, Methoxy, Ureido und Acetylamino substituiert sein kann, oder ist 1,4-Naphthylen, das durch eine Sulfogruppe in 6-, 7- oder 8-Stellung, bevorzugt 6- oder 7-Stellung, substituiert sein kann;
- E²: ist 1,4-Phenylen, das durch 1 oder 2 Substituenten aus der Gruppe Sulfo, Methyl, Methoxy, Ureido und Acetylamino substituiert sein kann, oder ist 1,4-Naphthylen, das durch eine Sulfogruppe in 6-, 7- oder 8-Stellung, bevorzugt 6- oder 7-Stellung, substituiert sein kann;
- Z¹: ist ein Rest der nachstehend angegebenen und definierten allgemeinen Formel (10A) und
- Z²: ist ein Rest der nachstehend angegebenen und definierten allgemeinen Formel (10B) 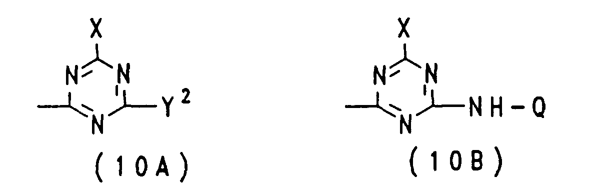 in welchen
X Chlor oder Fluor ist,
Y² Phenylamino ist, das durch 1 oder 2 Sulfogruppen oder eine Gruppe der allgemeinen Formel -SO₂-A mit A der oben genannten Bedeutung oder durch 1 oder 2 Sulfogruppen und eine dieser Gruppen -SO₂-A substituiert sein kann, oder Naphthylamino, bevorzugt Naphth-2-yl-amino, ist, das durch 1, 2 oder 3 Sulfogruppen oder durch eine Gruppe der allgemeinen Formel -SO₂-A mit A der oben genannten Bedeutung oder durch 1 oder 2 Sulfogruppen und diese Gruppe -SO₂-A substituiert sein kann, oder eine Gruppe der allgemeinen Formel (11a), (11b) oder (11c)

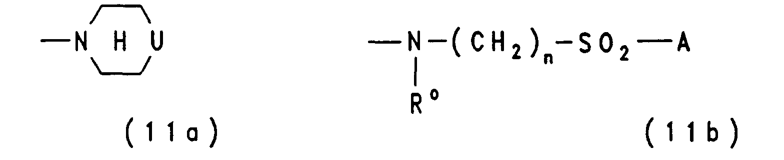

-NH - W² - SO₂ - A (11c)

ist, in welchen
U für -O- , -S- , -SO₂- oder -NH- steht oder eine Gruppe der Formel 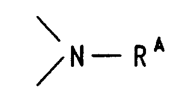 ist, worin R^{A} Wasserstoff, β-Hydroxyethyl oder β-Sulfatoethyl bedeutet,
n für die Zahl 2, 3 oder 4, bevorzugt 2 oder 3, steht,
A eine der oben genannten Bedeutungen hat,
R⁰ Wasserstoff, Methyl, Ethyl, Phenyl, Monosulfophenyl, Disulfophenyl oder ein Rest der Formel -(CH₂)ₙ-SO₂-A mit n und A der oben genannten Bedeutungen ist,
W² Alkylen von 3 bis 6 C-Atomen, bevorzugt von 4 C-Atomen, ist, das durch 1 oder 2 Heterogruppen aus der Gruppe der Formel -O- , -NH- , -SO₂- und -CO- unterbrochen ist, wie beispielsweise eine Gruppe der Formel -(CH₂)₂-O-(CH₂)₂-oder -(CH₂)₂-NH-(CH₂)₂- , und
Q Phenyl ist, das durch 1, 2 oder 3 Sulfogruppen oder durch eine Gruppe der allgemeinen Formel -SO₂-A mit A der obengenannten Bedeutung oder durch 1 oder 2 Sulfogruppen, bevorzugt 1 Sulfogruppe, und diese Gruppe -SO₂-A substituiert sein kann, oder Naphthyl, bevorzugt 2-Naphthyl ist, das durch 1, 2 oder 3 Sulfogruppen oder durch 1 Gruppe der allgemeinen Formel -SO₂-A mit A der obengenannten Bedeutung oder durch 1 oder 2 Sulfogruppen und diese Gruppe -SO₂-A substituiert sein kann.

Bevorzugt ist Y Morpholino, Monosulfophenylamino, Disulfophenylamino, durch Methoxy, Methyl, Carboxy, Sulfo, Hydroxy, Chlor oder Brom substituiertes Monosulfophenylamino, eine Gruppe der allgemeinen Formel (11c) obengenannter Bedeutung oder eine Gruppe der allgemeinen Formel (11b), in welcher n und A die genannten Bedeutungen haben und R⁰ Phenyl oder Monosulfophenyl ist oder in welcher R⁰ ein Rest der Formel -(CH₂)ₙ-SO₂-A mit n und A der obengenannten Bedeutung ist.

Die vorliegende Erfindung betrifft weiterhin Verfahren zur Herstellung der erfindungsgemäßen Azoverbindungen der allgemeinen Formel (1), die dadurch gekennzeichnet sind, daß man eine Verbindung der allgemeinen Formel (12) 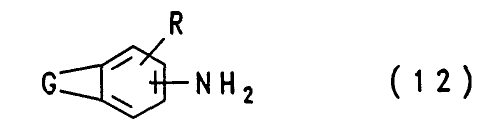 in welcher R und G eine der oben genannten, insbesondere bevorzugten, Bedeutungen haben, diazotiert und mit einer Verbindung der allgemeinen Formel (13) 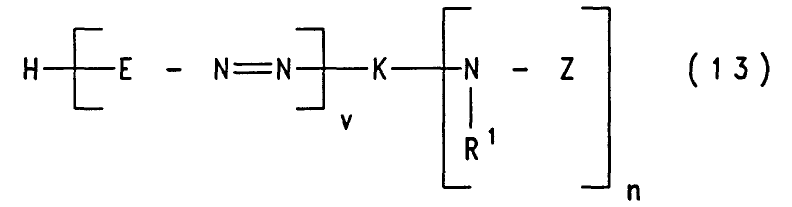 in welcher E, v, K, R¹, Z und n eine der oben genannten, insbesondere bevorzugten, Bedeutungen haben, kuppelt,
oder daß man eine Verbindung der allgemeinen Formel (14) 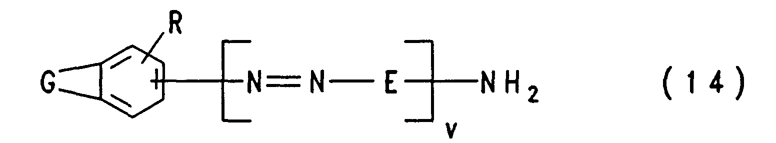 mit G, R, E und v einer der oben genannten, insbesondere bevorzugten, Bedeutungen diazotiert und mit einer Verbindung der allgemeinen Formel H-K-Z mit K und Z der oben genannten Bedeutung kuppelt, oder daß man eine Verbindung der allgemeinen Formel (15) 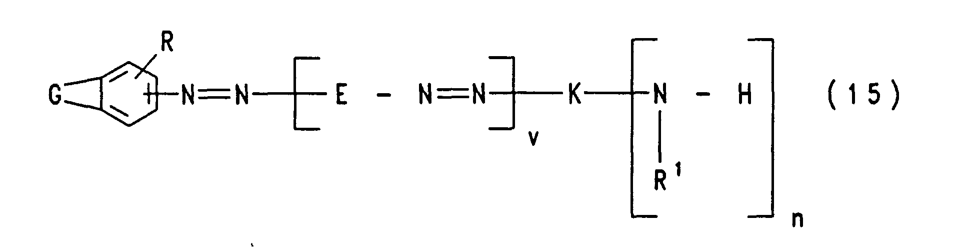 in welcher G, R, v, K, R¹ und n eine der oben genannten, insbesondere bevorzugten, Bedeutungen haben, mit einer Verbindung der allgemeinen Formel X⁰-Z , in welcher X⁰ für Chlor, Brom oder Fluor steht und Z die oben genannte Bedeutung besitzt, bevorzugt hiervon mit einer Verbindung der allgemeinen Formel (16) 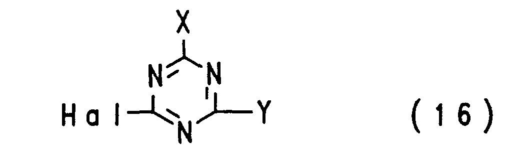 in welcher X und Y die oben genannten, insbesondere bevorzugten, Bedeutungen haben und Hal für Chlor oder Fluor steht, umsetzt.

Die Diazotierungsreaktionen erfolgen in an und für sich bekannter Verfahrensweise in wäßrigem Medium bei einem pH-Wert von kleiner als 2 und bei einer Temperatur zwischen -5°C und + 15°C. Die Kupplungsreaktionen erfolgen ebenfalls in an und für sich bekannter Verfahrensweise in wäßriger Lösung oder in wäßrigem-organischem Medium bei einem pH-Wert zwischen 4 und 9, bevorzugt zwischen 5 und 7, und bei einer Temperatur zwischen 0°C und 40°C, bevorzugt zwischen 5°C und 25°C.

Die Kondensationsreaktionen zwischen den Verbindungen der allgemeinen Formel (15) und der faserreaktiven Ausgangsverbindung der allgemeinen Formel X⁰-Z mit X⁰ und Z der oben genannten Bedeutung, wie bevorzugt der oben angegeben definierten allgemeinen Formel (16), erfolgen ebenfalls in wäßrigem oder wäßrig-organischem Medium in Suspension oder Lösung. Führt man diese Umsetzungen in einem wäßrig-organischen Medium durch, so ist das organische Medium beispielsweise Azeton, Dimethylformamid, Dimethylsulfoxid oder N-Methyl-pyrrolidon. Vorteilhaft wird der bei der Umsetzung freiwerdende Halogenwasserstoff laufend durch Zugabe wäßriger Alkali- oder Erdalkalihydroxide, -carbonate oder -bicarbonate neutralisiert. In der Regel erfolgt die Umsetzung bei einer Temperatur zwischen -5°C und +95°C und bei einem pH-Wert zwischen 3 und 11, bevorzugt zwischen 4 und 6, im Falle von X⁰ bzw. Hal gleich Fluor bevorzugt bei einer Temperatur zwischen 20 und 40°C und bei einem pH-Wert zwischen 4 und 10, insbesondere bevorzugt zwischen 4 und 7.

Die Ausgangsverbindungen der allgemeinen Formel (12) sind literaturbekannt (s. bspw. Dyes and Pigments 21 (1993), 123, und Ann. Chim. 49 (1959), 1809); sofern einzelne Verbindungen nicht beschrieben sind, können sie analog der Verfahrensweise des Standes der Technik hergestellt werden. Beispielsweise können Verbindungen der allgemeinen Formel (12) in welcher G für einen Rest der allgemeinen Formel (2a) oder (2e) mit R¹⁰ einer anderen der angegebenen Bedeutungen als Wasserstoff hergestellt werden, indem man von Verbindungen der nachstehend angegebenen allgemeinen Formel (a) mit R¹⁰ der obengenannten Bedeutung ausgeht (die beispielsweise aus Fierz-David & Blangley, "Farbenchemie", 8. Aufl. (1952), Springer Verlag, Tabelle 5 der Anlage, bekannt sind) und diese mit Phosgen, Dialkylcarbonat oder Chlorameisensäurealkylestern oder mit Oxalylchlorid bei einer Temperatur zwischen 0 und 100°C und einem pH-Wert zwischen 6 und 10 umsetzt und in der so erhaltenen heterocyclischen Verbindung der allgemeinen Formel (b) 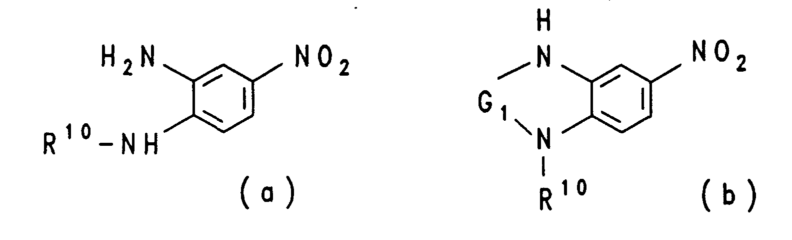 in welcher R¹⁰ die obengenannte Bedeutung besitzt und G₁ für die Gruppe -CO- oder -CO-CO- steht, die Nitrogruppe in bekannter Weise zur Aminogruppe reduziert, wie beispielsweise mittels Wasserstoff und einem Nickel-, Palladium- oder Platinkatalysator.

Ebenso sind die übrigen Ausgangsverbindungen, wie die Verbindungen der allgemeinen Formeln H-E-NH₂ und H-K-N(R¹)H sowie die faserreaktiven Ausgangsverbindungen der allgemeinen Formel X⁰-Z zahlreich in der Literatur beschrieben und vielfach zur Herstellung von faserreaktiven Farbstoffen eingesetzt (s. bspw. die europäischen Patentanmeldungs-Veröffentlichungen Nrs. 0 541 057, 0 542 214 und 0 548 795) oder lassen sich analog den dort angegebenen Verfahrensweisen mit entsprechenden Ausgangsverbindungen herstellen.

Ausgangsverbindungen der allgemeinen Formel (12) sind beispielsweise 4-Amino-benzimidazolin-2-on, 5-Amino-benzimidazolin-2-on, 1-N-(4'-Sulfophenyl)-5-amino-benzimidazolin-2-on, 1-N-(3'-Sulfophenyl)-5-amino-benzimidazolin-2-on, 1-N-(6',8'-Disulfo-naphth-2'-yl)-5-aminobenzimidazolin-2-on, 6-Amino-chinoxalin-2,3-dion, 7-Methyl-6-amino-chinoxalin-2,3-dion, 7-Methoxy-6-amino-chinoxalin-2,3-dion, 7-Ethoxy-6-amino-chinoxalin-2,3-dion, 4-N-(4'-Sulfophenyl)-6-amino-chinoxalin-2,3-dion, 4-N-(3'-Sulfophenyl)-6-amino-chinoxalin-2,3-dion, 4-N-(2',5'-Disulfophenyl)-6-aminochinoxalin-2,3-dion, 4-N-(6',8'-Disulfo-naphth-2'-yl)-6-amino-chinoxalin-2,3-dion, 4-N-(4',6',8'-Trisulfo-naphth-2'-yl)-6-amino-chinoxalin-2,3-dion, 5-Aminooxazolin-2-on, 6-Chlor-5-amino-oxazolin-2-on, 6-Amino-chinazolin-2,4-dion, 7-Amino-chinazolin-2,4-dion, 7-Amino-3,4-dihydro-chinolin-2-on, 6-Amino-1,3-dihydro-indol-2-on, 5-Amino-1,2-benzisothiazolin-3-on-1,1-dioxid, 6-Amino-1,2-benzisothiazolin-3-on-1,1-dioxid, 5-Amino-phthalimid, 5-Amino-N'-(4'-Sulfophenyl)-phthalimid, 5-Amino-N'-(3'-sulfophenyl)-phthalimid, 5-Amino-N'-(5',7'-disulfo-naphth-2'-yl)-phthalimid und 5-Amino-N'-(6',8'-disulfo-naphth-2'-yl)-phthalimid.

Die Ausgangsverbindungen entsprechend der allgemeinen Formel (17) 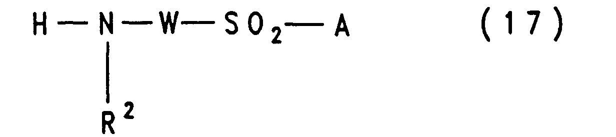 mit R², W und A der oben genannten Bedeutungen, die als Aminoreste in den Ausgangsverbindungen der allgemeinen Formeln (13) und (16) enthalten sind und zu deren Synthese dienen, sind teilweise bekannt, so beispielsweise aus den europäischen Patentanmeldungs-Veröffentlichungen Nrs. 0 070 806, 0 070 807, 0 374 758, 0 499 588, 0 541 057 und 0 568 860 sowie aus den U.S.-Patentschriften 4 908 436 und 5 138 041 und der britischen Patentschrift 1 576 237. Die Aminoverbindungen der allgemeinen Formel (17), in welchen R² für einen gegebenenfalls substituierten Phenylrest steht und W der 1,3-Propylen-Rest ist, sind dagegen bisher per se nicht beschrieben. Sie lassen sich beispielsweise in der Weise herstellen, daß man N-Allyl-N-acetyl-anilin (s. J. Org. Chem. 14, 1099 (1949)) analog der in der deutschen Offenlegungsschrift Nr. 41 06 106 beschriebenen Verfahrensweise mit 2-Mercapto-ethanol in Gegenwart eines Radikalinitiators umsetzt, die erhaltene N-[γ-(ß'-Hydroxyethylthio)-propyl]-N-acetyl-anilin-Verbindung zur Sulfonylverbindung oxidiert, beispielsweise mittels Wasserstoffperoxid in Gegenwart einer katalytischen Menge einer Übergangsmetallverbindung, wie beispielsweise Wolframoxid. Aus der so erhaltenen Sulfonylverbindung wird die Acetylgruppe im alkalischen oder sauren Bereich, vorzugsweise in salzsaurer wäßriger Lösung, wie beispielsweise in 5 bis 30 %iger wäßriger Salzsäure, bei einer Temperatur zwischen 80 und 100°C hydrolytisch abgespalten.
Das so erhaltene N-Phenyl-N-[γ-(ß'-hydroxyethylsulfonyl)-propyl]-amin kann aus der neutral gestellten wäßrigen Syntheselösung von der wäßrigen Phase abgetrennt werden. Dessen ß-Hydroxyethylsulfonyl-Gruppe läßt sich nach üblichen Methoden verestern, so beispielsweise mittels konzentrierter Schwefelsäure bei einer Temperatur zwischen 10 und 30°C in die ß-Sulfatoethylsulfonyl-Verbindung oder mit Thionylchlorid oder gasförmigem Chlorwasserstoff in die ß-Chlorethylsulfonyl-Verbindung überführen.

Ausgangsverbindungen der allgemeinen Formel (17) sind beispielsweise N-[γ-(β'-Chlorethylsulfonyl)-propyl]-amin, N-[γ-(β'-Sulfatoethylsulfonyl)-propyl]-amin, N-[γ-(Vinylsulfonyl)-propyl]-amin, N-[β-(β'-Chlorethylsulfonyl)-ethyl]-amin, N-[β-[β'-Sulfatoethylsulfonyl)-ethyl]-amin, N-[β-(Vinylsulfonyl)-ethyl]-amin, N-Methyl-N-[β-(β'-chlorethylsulfonyl)-ethyl]-amin, N-Ethyl-N-[β-(β'-chlorethylsulfonyl)-propyl]-amin, N-n-Propyl-N-[β-(β'-chlorethylsulfonyl)-ethyl]-amin, N-n-Butyl-N-[β-(β'-chlorethylsulfonyl)-ethyl]-amin, N-Carboxymethyl-N-[β-(β'-bromethylsulfonyl)-ethyl]-amin, N-Sulfatomethyl-N-[β-(β'-chlorethylsulfonyl)-ethyl]-amin, N-(β-Carboxyethyl)-N-[β'-(β"-chlorethylsulfonyl)-ethyl]-amin, N-(β-Sulfatoethyl)-N-[β'-(β"-chlorethylsulfonyl)-ethyl]-amin, N-(β-Ethoxyethyl)-N-[β'-(β"-chlorethylsulfonyl)-ethyl]-amin, N-Phenyl-N-[β-(β'-chlorethylsulfonyl)-ethyl]-amin, N-(4-Chlorphenyl)-N-[β-(β'-chlorethylsulfonyl)-ethyl]-amin, N-(2-Methylphenyl)-N-[β-(β'-chlorethylsulfonyl)-ethyl]-amin, N-(4-Methoxyphenyl)-N-[β-(β'-chlorethylsulfonyl)-ethyl]-amin, N-(3-Sulfophenyl)-N-[β-(β'-chlorethylsulfonyl)-ethyl]-amin, N-(4-Sulfophenyl)-N-[β-(β'-chlorethylsulfonyl)-ethyl]-amin, N-(β-Cyanoethyl)-N-[β'-(β"-chlorethylsulfonyl)-ethyl]-amin, N-Phenyl-N-(β-vinylsulfonyl-ethyl)-amin, N-(4-Chlorphenyl)-N-(β-vinylsulfonyl-ethyl)-amin, N-(2-Methylphenyl)-N-(β-vinylsulfonyl-ethyl)-amin, N-(4-Methoxyphenyl)-N-(β-vinylsulfonyl-ethyl)-amin, N-(3-Sulfophenyl)-N-(β-vinylsulfonyl-ethyl)-amin, N-(4-Sulfophenyl)-N-(β-vinylsulfonyl-ethyl)-amin, N-Phenyl-N-[β-(β'-sulfatoethylsulfonyl)-ethyl]-amin, N-(4-Chlorphenyl)-N-[β-(β'-sulfatoethylsulfonyl)-ethyl]-amin, N-(2-Methylphenyl)-N-[β-(β'-sulfatoethylsulfonyl)-ethyl]-amin, N-(4-Methoxyphenyl)-N-[β-(β'-sulfatoethylsulfonyl)-ethyl]-amin, N-(3-Sulfophenyl)-N-[β-(β'-sulfatoethylsulfonyl)-ethyl]-amin, N-(4-Sulfophenyl)-N-[β-(β'-sulfatoethylsulfonyl)-ethyl]-amin, N-Methyl-N-[γ-(β'-chlorethylsulfonyl)-propyl]-amin, N-Ethyl-N-[γ-(β'-chlorethylsulfonyl)-propyl]-amin, N-n-Propyl-N-[γ-(β'-chlorethylsulfonyl)-propyl]-amin, N-n-Butyl-N-[γ-(β'-chlorethylsulfonyl)-propyl]-amin, N-Carboxymethyl-N-[γ-(β'-bromethylsulfonyl)-propyl]-amin, N-Sulfatomethyl-N-[γ-(β'-chlorethylsulfonyl)-propyl]-amin, N-(β-Carboxyethyl)-N-[γ'-(β"-chlorethylsulfonyl)-propyl]-amin, N-(β-Sulfatoethyl)-N-[γ'-(β"-chlorethylsulfonyl)-propyl]-amin, N-(β-Sulfatoethyl)-N-[δ'-(β"-chlorethylsulfonyl)-butyl]-amin, N-(β-Ethoxyethyl)-N-[δ'-(β"-chlorethylsulfonyl)-butyl]-amin, N-(β-Ethoxyethyl)-N-(γ'-(β"-chlorethylsulfonyl)-propyl]-amin, N-Phenyl-N-[γ-(β'-chlorethylsulfonyl)-propyl]-amin, N-(4-Chlorphenyl)-N-[γ-(β'-chlorethylsulfonyl)-propyl]-amin, N-(2-Methylphenyl)-N-[γ-(β'-chlorethylsulfonyl)-propyl]-amin, N-(4-Methoxyphenyl)-N-[γ-(β'-chlorethylsulfonyl)-propyl]-amin, N-(3-Sulfophenyl)-N-[γ-(β'-chlorethylsulfonyl)-propyl]-amin, N-(4-Sulfophenyl)-N-[γ-(β'-chlorethylsulfonyl)-propyl]-amin, N-(β-Cyanoethyl)-N-[γ'-(β"-chlorethylsulfonyl)-propyl]-amin, N-Phenyl-N-(γ-vinylsulfonyl-propyl)-amin, N-(4-Chlorphenyl)-N-(γ-vinylsulfonyl-propyl)-amin, N-(2-Methylphenyl)-N-(γ-vinylsulfonyl-propyl)-amin, N-(4-Methoxyphenyl)-N-(γ-vinylsulfonyl-propyl)-amin, N-(3-Sulfophenyl)-N-(γ-vinylsulfonyl-propyl)-amin, N-(4-Sulfophenyl)-N-(γ-vinylsulfonyl-propyl)-amin, N-Phenyl-N-[γ-(β'-sulfatoethylsulfonyl)-propyl]-amin, N-(4-Chlorphenyl)-N-[γ-(β'-sulfatoethylsulfonyl)-propyl]-amin, N-(2-Methylphenyl)-N-[γ-(β'-sulfatoethylsulfonyl)-propyl]-amin, N-(4-Methoxyphenyl)-N-[γ-(β'-sulfatoethylsulfonyl)-propyl]-amin, N-(3-Sulfophenyl)-N-[γ-(β'-sulfatoethylsulfonyl)-propyl]-amin, N-(4-Sulfophenyl)-N-[γ-(β'-sulfatoethylsulfonyl)-propyl]-amin, N-Phenyl-N-[α-carboxy-γ-(β'-chlorethylsulfonyl)-propyl]-amin, N-Phenyl-N-[α-ethoxycarbonyl-γ-(β'-chlorethylsulfonyl)-propyl]-amin, N-Phenyl-N-[α-methoxycarbonyl-γ-(β'-chlorethylsulfonyl)-propyl]-amin, N-Phenyl-N-[β-methyl-γ-(β'-chlorethylsulfonyl)-propyl]-amin, N-Phenyl-N-[β-ethyl-γ-(β'-chlorethylsulfonyl)-propyl]-amin, N-Phenyl-N-[δ-(β'-chlorethylsulfonyl)-butyl]-amin, N-Phenyl-N-[ε-(β'-chlorethylsulfonyl)-pentyl]-amin, N-Phenyl-N-[β-(β'-chlorethylsulfonyl)-hexyl]-amin, 3- und 4-(β-Sulfatoethylsulfonyl)-anilin, 2-Methoxy-5-(β-sulfatoethylsulfonyl)-anilin, 2,5-Dimethoxy-4-(β-sulfatoethylsulfonyl)-anilin, 2-Methoxy-5-methyl-4-(β-sulfatoethylsulfonyl)-anilin, 2-Sulfo-4-(β-sulfatoethylsulfonyl)-anilin, 2-Hydroxy- und 2-Brom-5-(β-sulfatoethylsulfonyl)-anilin, 4-[β-(β'-Sulfatoethylsulfonyl)-ethyl]-anilin, 1-Sulfo-6-(β-sulfatoethylsulfonyl)-2-naphthylamin, 8-Sulfo-6-(β-sulfatoethylsulfonyl)-2-napthylamin, 6-Sulfo-8-(β-sulfatoethylsulfonyl)-2-naphthylamin und 8- und 6-(β-Sulfatoethylsulfonyl)-2-naphthylamin.

Ausgangsverbindungen, die zur Herstellung von erfindungsgemäßen Dis- und Trisazoverbindungen der allgemeinen Formel (1) zunächst als Kupplungskomponente und sodann, in Form der gebildeten AminoAzoverbindung als Diazokomponente dienen und der allgemeinen Formel H-E-NH₂ entsprechen, sind beispielsweise Anilin, 3-Methyl-anilin, 2-Methoxy-5-methyl-anilin, 2,5-Dimethyl-anilin, 3-Ureido-anilin, 3-Acetylamino-anilin, 3-Propionylamino-anilin, 3-Butyrylamino-anilin, 3-Methoxy-anilin, 2-Methyl-5-acetylamino-anilin, 2-Methoxy-5-acetylamino-anilin, 2-Methoxy-5-methyl-anilin, 3-(Hydroxyacetylamino)-anilin, 1,3-Diaminobenzol, 1,3-Diaminobenzol-4-sulfonsäure, 2- und 3-Sulfo-anilin, 3-Hydroxy-anilin, 1-Amino-naphthalin-6-, -7-oder -8-sulfonsäure, 1-Amino-2-methoxy-naphthalin-6-sulfonsäure, 2-Amino-5-hydroxy-naphthalin-7-sulfonsäure, 2-Amino-5-hydroxy-naphthalin-1,7-disulfonsäure, 2-Amino-8-hydroxy-naphthalin-6-sulfonsäure, 2-(4'-Amino-benzoylamino)-5-naphthol-7-sulfonsäure, 1-(4'-Amino-2'-sulfophenyl)-3-methyl-5-pyrazolon, 1-(4'-Amino-2'-sulfo-phenyl)-3-carboxy-5-pyrazolon und N-(Acetoacetyl)-3-sulfo-4-amino-anilid.

Kupplungskomponenten entsprechend der allgemeinen Formel H-K-N(R¹)H, die zum Aufbau der erfindungsgemäßen Azoverbindungen dienen können und in deren Aminogruppen der Rest der faserreaktiven Gruppierung eingeführt werden kann, sind beispielsweise Anilin, 3-Methyl-anilin, 2,5-Dimethyl-anilin, 2,5-Dimethoxy-anilin, 3-Methoxy-anilin, 3-Acetylamino-anilin, 3-Propionylaminoanilin, 3-Butyrylamino-anilin, 3-Benzoylamino-anilin, 3-(Hydroxyacetylamino)-anilin, 3-Ureido-anilin, 2-Methyl-5-acetylamino-anilin, 2-Methoxy-5-acetylaminoanilin, 2-Methoxy-5-methyl-anilin, 1 -Amino-naphthalin-6-sulfonsäure, 1-Aminonaphthalin-7-sulfonsäure, 4-Sulfo-1,3-diamino-benzol, 6-Sulfo-2-methoxy-1-amino-naphthalin, 5,7-Disulfo-2-aminonaphthalin, 1-Amino-8-hydroxynaphthalin-4-sulfonsäure, 1-Amino-8-hydroxynaphthalin-6-sulfonsäure, 1-Amino-8-hydroxynaphthaln-2,4-disulfonsäure, 1-Amino-8-naphthol-3,6-disulfonsäure, 1-Amino-8-hydroxy-4,6-disulfonsäure, 1-Amino-8-hydroxy-naphthalin-2,4,6-trisulfonsäure, 2-(Methylamino)- und 2-(Ethylamino)-5-hydroxynaphthalin-7-sulfonsäure, 2-Amino-5-hydroxynaphthalin-1,7-disulfonsäure, 2-(Methylamino)- und 2-(Ethylamino)-8-hydroxynaphthalin-6-sulfonsäure, 2-Amino-8-hydroxynaphthalin-3,6-disulfonsäure, 2-(4'-Amino-3'-sulfophenylamino)-5-hydroxynaphthalin-7-sulfonsäure, 1-Amino-8-hydroxy-2-(phenylazo)-naphthalin-3,6-disulfonsäure, 1-Amino-8-hydroxy-2-(4'-sulfophenylazo)-naphthalin-3,6-disulfonsäure, 1-Amino-8-hydroxy-2-(2',5'-disulfophenylazo)-naphthalin-3,6-disulfonsäure, 1-(β-Aminoethyl)-3-cyano-4-methyl-6-hydroxy-pyrid-2-on, 1-(γ-Aminopropyl)-3-sulfomethyl-4-methyl-6-hydroxy-pyrid-2-on, 1,3-Diaminobenzol, 3-[N,N-Di-(β-hydroxyethyl)]-amino-anilin, 3-[N,N-Di-(β-sulfatoethyl)]-amino-4-methoxy-anilin, 3-(Sulfo-benzylamino)-anilin, 3-(Sulfobenzoylamino)-4-chlor-anilin und 3-[N,N-Di-(sulfobenzyl)]-amino-anilin, 2-Sulfo-5-acetylamino-anilin, 2-Amino-5-naphthol-7-sulfonsäure, 2-Amino-8-naphthol-6-sulfonsäure, 1-(4'-Aminobenzoyl)-amino-8-hydroxynaphthalin-3,6-disulfonsäure, 1-(4'-Aminobenzoyl)-amino-8-hydroxynaphthalin-4,6-disulfonsäure, 1-(3'-Aminobenzyl)-amino-8-hydroxynaphthalin-3,6-disulfonsäure, 1-(3'-Aminobenzoyl)-amino-8-hydroxynaphthalin-4,6-disulfonsäure, 1-(2'-Aminobenzoyl)-amino-8-hydroxy-naphthalin-3,6-disulfonsäure, 1-(2'-Aminobenzoyl)-amino-8-hydroxyphaphthalin-4,6-disulfonsäure, 2-(3'-Aminobenzoyl)-1mino-5-hydroxy-naphthalin-7-sulfonsäure, 2-(2'-Aminobenzoyl)-amino-5-hydroxy-naphthalin-7-sulfonsäure, 2-(4'-Aminobenzoyl)-amino-8-hydroxynaphthalin-6-sulfonsäure, 2-(3'-Aminobenzoyl)-amino-8-hydroxynaphthalin-6-sulfonsäure, 2-(2'-Aminobenzoyl)-amino-8-hydroxynaphthalin-6-sulfonsäure, 2-(4'-Aminobenzoyl)-amino-5-naphthol-7-sulfonsäure, 1-(4'-Amino- oder 1-(4'-Acetylamino-2-sulfophenyl)-3-methyl- oder -3-carboxy-5-pyrazolon, N-(3-Sulfo-4-amino)-acetoacetyl-anilid, 1-(3'-Aminobenzoyl)- oder 1-(4'-Aminobenzoyl)-amino-8-naphthol-3,6- oder -4,6-disulfonsäure, 1-Acetylamino-8-naphthol-3,6- oder -4,6-disulfonsäure, 2-Acetylamino-5-naphthol-7-sulfonsäure, 2-Acetylamino-8-naphthol-6-sulfonsäure, 3-Acetylamino-8-naphthol-6-sulfonsäure, 3-(N-Methyl-amino)-8-naphthol-6-sulfonsäure, 1-(3'-Amino- oder 1-(3'-Acetylamino-6'-sulfophenyl)-3-methyl- oder -3-carboxy-5-pyrazolon, 2-(N-Methyl-N-acetylamino)- oder 2-Methylamino-5-naphthol-7-sulfonsäure, N-Methyl-anilin und N-Propyl-m-toluidin.

Die Abscheidung der erfindungsgemäß hergestellten Azoverbindungen der allgemeinen Formel (1) aus den Syntheseansätzen erfolgt nach allgemein bekannten Methoden entweder durch Ausfällen aus dem Reaktionsmedium mittels Elektrolyten, wie beispielsweise Natriumchlorid oder Kaliumchlorid, oder durch Eindampfen der Reaktionslösung, beispielsweise durch Sprühtrocknung, wobei dieser Reaktionslösung eine Puffersubstanz zugeführt werden kann.

Die Azoverbindungen der allgemeinen Formel (1) - im nachfolgenden Farbstoffe (1) genannt - eignen sich zum Färben und Bedrucken der verschiedensten Materialien, wie Seide, Leder, Wolle, Polyamidfasern und Polyurethanen, und insbesondere cellulosehaltiger Fasermaterialien aller Art. Solche Fasermaterialien sind beispielsweise die natürlichen Cellulosefasern, wie Baumwolle, Leinen und Hanf, sowie Zellstoff und regenerierte Cellulose. Die Farbstoffe (1) sind auch zum Färben oder Bedrucken von hydroxygruppenhaltigen Fasern geeignet, die in Mischgeweben enthalten sind, z.B. von Gemischen aus Baumwolle mit Polyesterfasern oder Polyamidfasern.

Die Farbstoffe (1) lassen sich auf verschiedene Weise auf das Fasermaterial applizieren und auf der Faser fixieren, insbesondere in Form von wäßrigen Farbstofflösungen und -druckpasten. Sie eignen sich sowohl für das Ausziehverfahren als auch zum Färben nach dem Foulard-Färbeverfahren, wonach die Ware mit wäßrigen, gegebenenfalls salzhaltigen Farbstofflösungen imprägniert und der Farbstoff nach einer Alkalibehandlung oder in Gegenwart von Alkali, gegebenenfalls unter Wärmeeinwirkung, fixiert wird. Besonders geeignet sind die erfindungsgemäßen Farbstoffe für das sogenannte Kaltverweilverfahren, wonach der Farbstoff zusammen mit dem Alkali auf dem Foulard aufgebracht und danach durch mehrstündiges Lagern bei Raumtemperatur fixiert wird. Nach dem Fixieren werden die Färbungen oder Drucke mit kaltem und heißem Wasser, gegebenenfalls unter Zusatz von eines dispergierend wirkenden und die Diffusion der nicht fixierten Anteile fördernden Mittels, gründlich gespült. Diese Färbe- und Druckverfahren sind zahlreich in der allgemeinen Fachliteratur wie auch in der Patentliteratur, wie beispielsweise in den anfangs genannten Druckschriften, beschrieben.

Gegenstand der vorliegenden Erfindung ist deshalb auch die Verwendung der Farbstoffe (1) zum Färben (einschließlich des Bedrucken) dieser Materialien bzw. Verfahren zum Färben (und Bedrucken) solcher Materialien in an und für sich üblicher Verfahrensweise, bei welchen ein Farbstoff (1) als Farbmittel eingesetzt wird, indem man den Farbstoff (1) im wäßrigen Medium auf das Material appliziert und ihn mittels Wärme oder mittels einer alkalisch wirkenden Verbindung oder mittels beidem auf dem Material fixiert.

Die Farbstoffe (1) zeichnen sich durch hohe Reaktivität, gutes Fixiervermögen und ein sehr gutes Aufbauvermögen aus. Sie können daher nach dem Ausziehfärbeverfahren bei niedrigen Färbetemperaturen eingesetzt werden und erfordern bei Pad-Steam-Verfahren nur kurze Dämpfzeiten. Die Fixiergrade sind hoch, und die nicht fixierten Anteile können leicht ausgewaschen werden, wobei die Differenz zwischen Ausziehgrad und Fixiergrad bemerkenswert klein, d.h. der Seifverlust sehr gering ist. Die Farbstoffe (1) eignen sich auch besonders zum Druck, vor allem auf Baumwolle, ebenso aber auch zum Bedrucken von stickstoffhaltigen Fasern, z.B. von Wolle oder Seide oder von Mischgeweben, die Wolle oder Seide enthalten.

Die Farbstoffe (1) eigenen sich insbesondere auch für salzarme Auszieh-Färbeverfahren, die dadurch gekennzeichnet sind, daß die Färbeflotte ein oder mehrere Elektrolytsalze, wie Natriumchlorid, Kaliumchlorid und Natriumsulfat, von insgesamt 20 bis 40 g/l (anstelle der in der Technik üblichen 50 bis 80 g/l) enthalten. Trotz dieser geringen Elektrolytsalzkonzentration in der Auszieh-Färbeflotte ziehen die Farbstoffe (1) auf das Fasermaterial gleichmäßig und in hoher Farbstärke auf, weswegen egale und farbstarke Färbungen erhalten werden. Die hohe Egalität der Färbungen besteht beim salzarmen Färben auch dann, wenn helle Farbnuancen gewünscht und somit die Farbstoffe (1) nur in geringen Mengen in der Färbeflotte verwendet werden. Durch die Möglichkeit des salzarmen Färbens mit den Farbstoffen (1) ergibt sich ein anwendungstechnischer Vorteil insbesondere in ökologischer Sicht.

Die mit den Farbstoffen (1) hergestellten Färbungen und Drucke besitzen, insbesondere auf Cellulosefasermaterialien, eine hohe Farbstärke und eine hohe Faser-Farbstoff-Bindungsstabilität sowohl in saurem als auch in alkalischem Bereich, weiterhin eine gute Lichtechtheit und sehr gute Naßechtheitseigenschaften, wie Wasch-, Wasser-, Seewasser-, Ueberfärbe- und Schweißechtheiten, sowie eine gute Plissierechtheit, Bügelechtheit und Reibechtheit.

Die nachfolgenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in den Beispielen formelmäßig beschriebenen Verbindungen sind in Form der freien Säure angegeben; im allgemeinen werden sie in Form ihrer Alkalimetallsalze, wie Lithium-, Natrium- oder Kaliumsalze, hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, in die Synthese eingesetzt werden.

Die für die erfindungsgemäßen Farbstoffe angegebenen Absorptionsmaxima (λₘₐₓ) im sichtbaren Bereich wurden anhand ihrer Alkalimetallsalze in wäßriger Lösung ermittelt. In den Tabellenbeispielen sind die λₘₐₓ-Werte bei der Farbtonangabe in Klammern gesetzt; die Wellenlängenangabe bezieht sich auf nm.

### Beispiel 1

a) Eine Suspension von 37,6 Teilen Cyanurchlorid in 2000 Teilen Wasser wird bei einer Temperatur zwischen 0 und 3°C und einem pH-Wert zwischen 2 und 2,5 innerhalb von etwa 60 Minuten mit 50,6 Teilen Anilin-2,5-disulfonsäure versetzt. Man rührt den Ansatz noch etwa 30 Minuten nach und gibt sodann eine wäßrige Suspension von 50 Teilen 3-Amino-8-hydroxy-6-sulfo-naphthalin in 1000 Teilen Wasser hinzu, stellt einen pH-Wert von 4,5 ein, erwärmt auf 40°C und rührt den Ansatz unter Einhaltung dieser Bedingungen noch etwa zwei Stunden.
b) 29,8 Teile 5-Amino-benzimidazolin-2-on werden in 3000 Teilen Wasser bei 0°C bis 5°C unter gutem Rühren mit 60 Teilen einer 31 %igen wäßrigen Salzsäure und 14 Teilen Natriumnitrit versetzt. Man rührt den Ansatz noch etwa 30 Minuten bei dieser Temperatur nach und gibt danach die so erhaltene Diazoniumsalzlösung innerhalb von etwa 15 Minuten unter Einhaltung eines pH-Wertes zwischen 6 und 7 zu der unter a) hergestellten Lösung der als Kupplungskomponente dienenden Verbindung 3-N-[2'-(2'',5''-Disulfo-phenyl)-amino-4'-chlor-1',3',5'-triazin-6'-yl]amino-8-hydroxy-6-sulfo-naphthalin. Man rührt den Ansatz noch etwa 12 Stunden unter Einhaltung dieses pH-Wertes und bei einer Temperatur zwischen 20 und 25°C nach und isoliert sodann die erhaltene erfindungsgemäße Azoverbindung, die, in Form der freien Säure geschrieben, der Formel 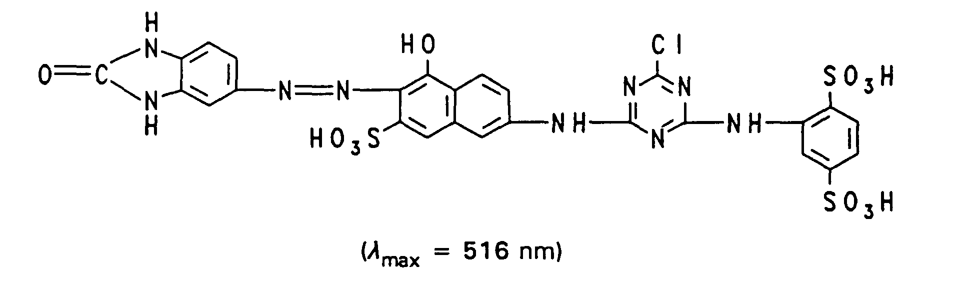 entspricht, als Alkalimetallsalz (Natriumsalz) in üblicher Weise, beispielsweise durch Aussalzen mit Natriumchlorid.

Die erfindungsgemäße Monoazoverbindung zeigt sehr gute faserreaktive Farbstoffeigenschaften und liefert nach den in der Technik für faserreaktive Farbstoffe üblichen Färbe- und Druckverfahren auf den in der Beschreibung genannten Fasermaterialien, insbesondere auf Cellulosefasermaterialien, wie Baumwolle, farbstarke rote Färbe- und Drucke mit guten Echtheitseigenschaften.

### Beispiel 2

14,9 Teile 5-Amino-benzimidazolin-2-on werden in 600 Teilen Wasser unter gutem Rühren bei einer Temperatur zwischen 0 und 5°C mit 30 Teilen einer wäßrigen 31 %igen Salzsäure und 6,9 Teilen Natriumnitrit versetzt. Man rührt den Ansatz noch 30 Minuten bei dieser Temperatur nach und gibt sodann die erhaltene Diazoniumsalzlösung innerhalb von 15 Minuten zu einer Lösung von 54,5 Teilen 1-N-(2'-Morpholino-4'-fluor-1',3',5'-triazin-6'-yl)-amino-8-hydroxy-3,6-disulfo-naphthalin (hergestellt gemäß der europäischen Patentanmeldungs-Veröffentlichung Nr. 0 542 082) in 1000 Teilen Wasser und rührt den Ansatz noch etwa 12 Stunden unter Einhaltung eines pH-Wertes von 7 und bei einer Temperatur zwischen 20 und 25°C nach.

Die erfindungsgemäße Azoverbindung, die, in Form der freien Säure geschrieben, die Formel 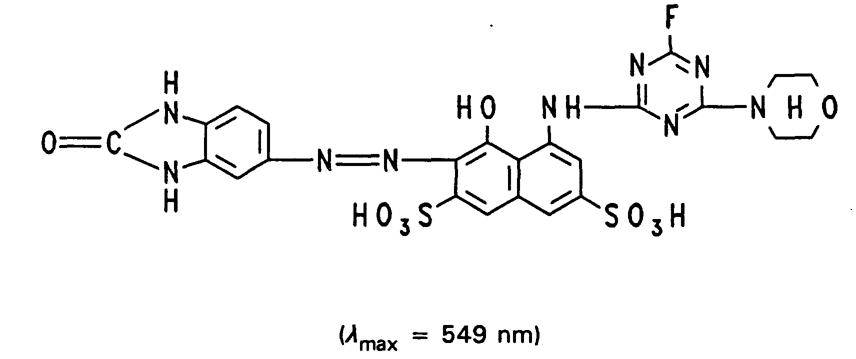 besitzt, wird aus der Syntheselösung in üblicher Weise, beispielsweise durch Aussalzen mit Natriumchlorid, als Alkalimetallsalz (Natriumsalz) isoliert.

Sie zeigt sehr gute faserreaktive Farbstoffeigenschaften und färbt beispielsweise Cellulosefasermaterialien nach den üblichen Anwendungsverfahren in kräftigen, brillanten violetten Tönen mit hohem Fixiergrad.

### Beispiel 3

30 Teile 4-Amino-benzimidazolin-2-on werden in einer Mischung aus 3000 Teilen Wasser und 60 Teilen einer 31 %igen wäßrigen Salzsäure bei einer Temperatur von 0 bis 5°C durch Zugabe von 14 Teilen Natriumnitrit diazotiert. Man rührt den Ansatz noch etwa 30 Minuten nach und gibt die erhaltene Diazoniumsalzlösung unter Einhaltung eines pH-Wertes von 6 bis 7 innerhalb von etwa 15 Minuten zu der gemäß Beispiel 1a) hergestellten Lösung der Kupplungskomponente. Man führt die Kupplungsreaktion innerhalb dieses pH-Bereiches unter weiterem Rühren bei 20 bis 25°C während etwa 12 Stunden zu Ende und isoliert sodann die erfindungsgemäße Azoverbindung der Formel (in Form der freien Säure geschrieben) 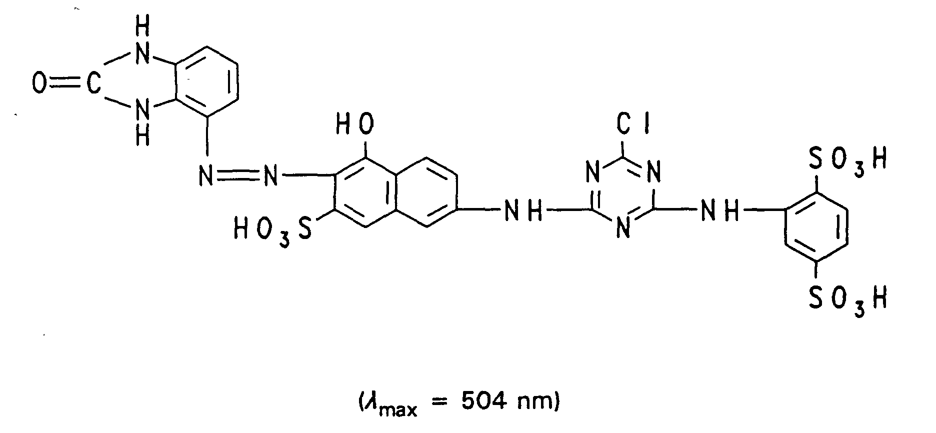 als Alkalimetallsalz (Natriumsalz) in üblicher Weise, beispielsweise durch Sprühtrocknen der wäßrigen Syntheselösung oder durch Aussalzen der Verbindung aus der Syntheselösung mit Natriumchlorid.

Die erfindungsgemäße Azoverbindung zeigt sehr gute faserreaktive Farbstoffeigenschaften und färbt die in der Beschreibung genannten Materialien, insbesondere cellulosehaltige Fasermaterialien, wie Baumwolle, nach den in der Technik für faserreaktive Farbstoffe üblichen Anwendungsverfahren in farbstarken, rotstichig braunen Tönen mit guten Echtheitseigenschaften.

### Beispiel 4

Man diazotiert 14,9 Teile 4-Amino-benzimidazolin-2-on in wäßriger salzsaurer Lösung (600 Teile Wasser und 30 Teile einer wäßrigen 31 %igen Salzsäure) bei 0 bis 5°C mittels 6,9 Teilen Natriumnitrit. Man rührt nach Zugabe des Natriumnitrits den Ansatz noch 30 Minuten bei 0 bis 5°C nach und gibt die erhaltene Diazoniumsalzlösung sodann zu der gemäß den Angaben des Beispieles 2 hergestellten Kupplungskomponente. Man rührt den Reaktionsansatz noch etwa 12 Stunden unter Einhaltung eines pH-Wertes von etwa 7 und einer Temperatur von 20 bis 25°C nach und isoliert sodann die erfindungsgemäße Azoverbindung in üblicher Weise.
Sie besitzt, in Form der freien Säure geschrieben, die Formel 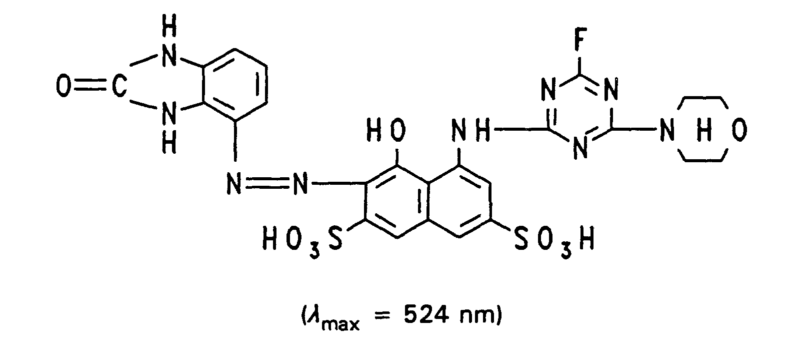 und zeigt gute faserreaktive Farbstoffeigenschaften. Sie färbt beispielsweise Baumwolle nach den in der Technik üblichen Färbe- und Druckverfahren in brillanten blaustichig roten Farbtönen mit guten Echtheitseigenschaften.

### Beispiel 5

59 Teile 2-(8'-Hydroxy-3',6'-disulfo-naphth-1'-yl-amino)-4-chlor-6-(3''-sulfophenyl-amino)-1,3,5-triazin werden mit 29 Teilen 4-(β-Sulfatoethylsulfonyl)-anilin in 1000 Teilen Wasser bei einem pH-Wert zwischen 3 und 4 und einer Temperatur zwischen 85 und 95°C innerhalb von vier Stunden miteinander umgesetzt. Anschließend kühlt man die Reaktionslösung auf 20 bis 25°C ab und gibt bei einem pH-Wert zwischen 6 und 7 die gemäß den Angaben des Beispiels 1 hergestellte Diazoniumsalzlösung aus 14,9 Teilen 5-Amino-benzoimidazolidin-2-on hinzu. Man rührt noch eine Weile zur Beendigung der Kupplungsreaktion nach.

Der erfindungsgemäße Azofarbstoff, der, in Form der freien Säure geschrieben, die Formel 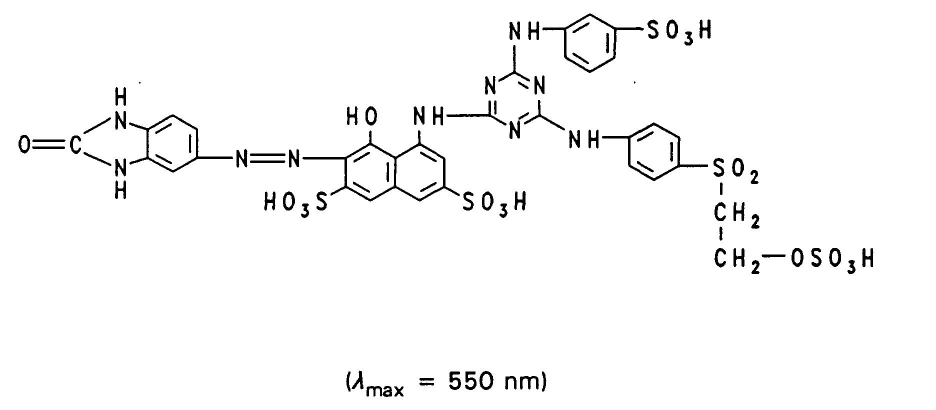 besitzt, wird in üblicher Weise als Alkalimetallsalz isoliert. Er zeigt sehr gute faserreaktive Eigenschaften und färbt beispielsweise Baumwolle in brillanten violetten Farbtönen mit guten Echtheitseigenschaften.

### Beispiel 6

31,7 Teile 3-Amino-4,6-disulfo-8-hydroxy-naphthalin werden in 500 Teilen Wasser bei einem pH-Wert zwischen 2 und 2,5 und bei einer Temperatur zwischen 0 und 5°C innerhalb von 2 bis 3 Stunden mit 19 Teilen Cyanurchlorid umgesetzt. Sodann gibt man zu dem Reaktionsansatz 36 Teile Bis-(β-chlorethylsulfonyl-ethyl)-amin-hydrochlorid hinzu und führt die Umsetzung bei einem pH-Wert zwischen 6 und 6,5 und einer Temperatur zwischen 40 und 50°C unter Rühren zu Ende. Man läßt danach den Ansatz auf 20 bis 25°C abkühlen und gibt sodann unter Einhaltung eines pH-Wertes zwischen 6 und 7 eine gemäß Beispiel 1 hergestellte Diazoniumsalzlösung aus 14 Teilen 5-Amino-benzimidazolin-2-on hinzu. Man rührt noch etwa 12 Stunden weiter und isoliert sodann den erfindungsgemäßen Azofarbstoff der, in Form der freien Säure geschriebenen, die Formel 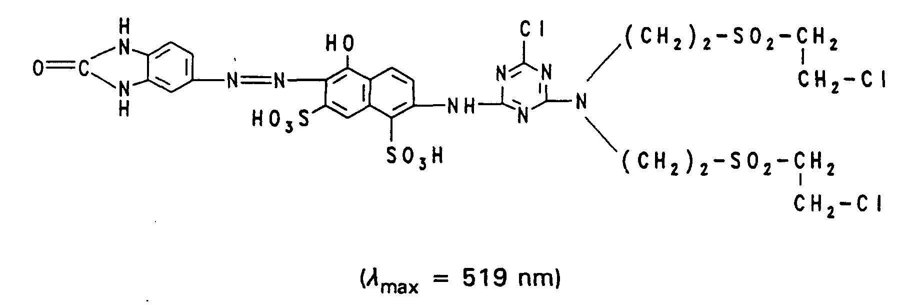 besitzt, in üblicher Weise als Alkalimetallsalz. Er zeigt sehr gute faserreaktive Farbstoffeigenschaften und färbt beispielsweise Baumwolle in roten Farbtönen mit guten Echtheitseigenschaften.

### Beispiel 7

23 Teile 7-Methoxy-6-amino-chinoxalin-2,3-dion werden in 300 Teilen Wasser bei 0 bis 5°C mit 30 Teilen einer 31 %igen wäßrigen Salzsäure und 7 Teilen Natriumnitrit versetzt. Man rührt bis zur Beendigung der Diazotierung nach und gibt sodann die Suspension des Diazoniumsalzes zu 1050 Teilen einer wäßrigen Lösung von 54,5 Teilen der im Beispiel 2 angegebenen Kupplungskomponente. Man führt die Kupplungsreaktion bei einer Temperatur zwischen 18 und 25°C und einem pH-Wert zwischen 5 und 6 durch und isoliert die erhaltene Azoverbindung, die, in Form der freien Säure geschrieben, die Formel 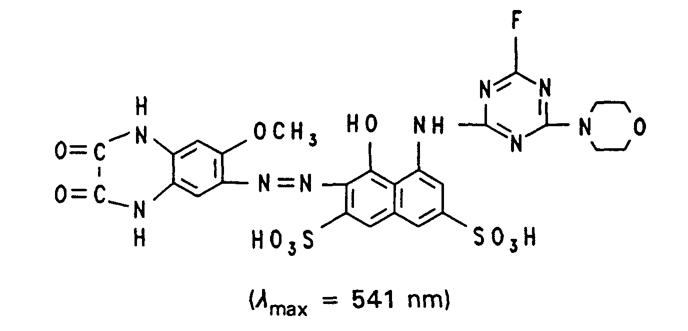 besitzt, in üblicher Weise aus der Syntheselösung durch Aussalzen mit Natriumchlorid. Sie besitzt sehr gute Farbstoffeigenschaften und liefert beispielsweise auf Cellulosefasermaterialien farbstarke, rotstichig blaue Färbungen bei hohem Fixiergrad.

### Beispiel 8

Eine gemäß Beispiel 7 hergestellte Diazoniumsalzsuspension aus 23 Teilen 7-Methoxy-6-amino-chinoxalin-2,3-dion rührt man in eine Lösung von 51,1 Teilen des Natriumsalzes der Verbindung 1-N-(2',4'-Dichlor-1',3',5'-triazin-6'-yl)-amino-8-hydroxy-3,6-disulfo-naphthalin in 1000 Teilen Wasser und führt die Kupplungsreaktion bei einer Temperatur zwischen 18 und 25°C und einem pH-Wert von 5 bis 6 durch. Die erfindungsgemäße Azoverbindung wird in üblicher Weise aus der Syntheselösung isoliert, beispielsweise durch Aussalzen mit Natriumchlorid. Sie besitzt, in Form der freien Säure geschrieben, die Formel 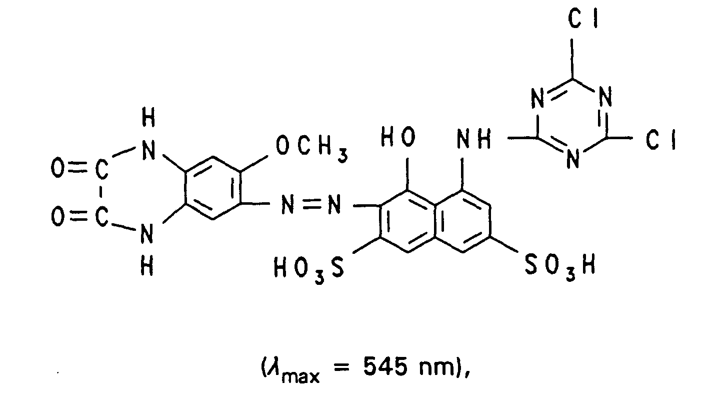 zeigt sehr gute faserreaktive Farbstoffeigenschaften und färbt beispielsweise Cellulosefasermaterialien nach den in der Technik üblichen Färbeverfahren in farbstarken, rotstichig blauen Tönen mit hohem Fixiergrad.

### Beispiel 9

Man versetzt 35,7 Teile des Natriumsalzes von 4-(4'-Sulfophenyl)-amino-3-amino-nitrobenzol in 200 Teilen Wasser bei 20 bis 25°C mit 9 Teilen Dimethylcarbonat und rührt den Ansatz einige Zeit bei einem pH-Wert zwischen 9 und 10 und einer Temperatur von 80 bis 90°C. Die erhaltene heterocyclische Verbindung wird unter Zusatz von in üblicher Weise aktiviertem Raney-Nickel bei einem Wasserstoffdruck von 2 bar bei einer Temperatur von 60°C und einem pH-Wert von 9,5 hydriert.

Die erhaltene Aminoverbindung dient nunmehr als Diazokomponente zur Herstellung einer erfindungsgemäßen Azoverbindung. Ohne sie aus der wäßrigen Syntheslösung zu isolieren, wird diese Lösung nach Filtration mit 30 Teilen einer 31 %igen wäßrigen Salzsäure versetzt und die Aminoverbindung mittels 7 Teilen Natriumnitrit in üblicher Weise diazotiert. Sodann gibt man diese Diazoniumsalzsuspension unter Einhaltung eines pH-Wertes von 5 bis 6 zu einer Lösung von 77,8 Teilen des Natriumsalzes der Verbindung 1-N-[2'-(4''-ß-Sulfatoethylsulfonyl)-4'-chlor-1',3',5'-triazin-6'-yl]-amino-8-hydroxy-3,6-disulfo-naphthalin in 500 Teilen Wasser. Man führt die Kupplungsreaktion bei einem pH-Wert von 5 bis 6 und einer Temperatur zwischen 18 und 25°C zu Ende und isoliert die erhaltene erfindungsgemäße Azoverbindung der Formel (in Form der freien Säure geschrieben) 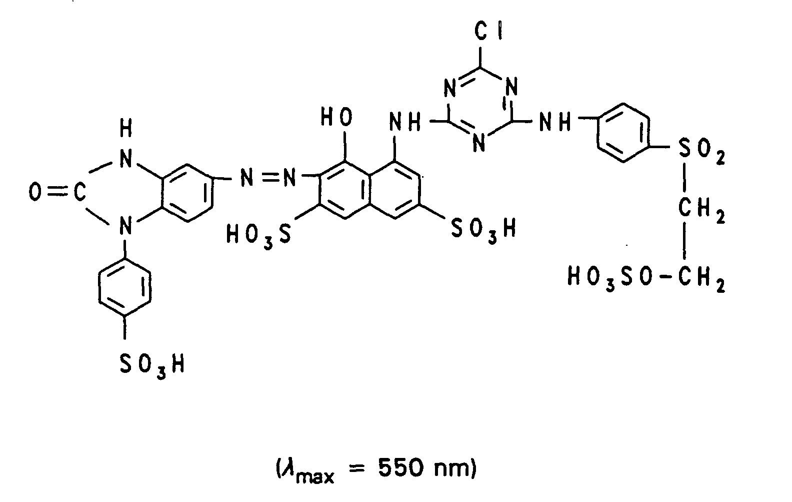 beispielsweise durch Aussalzen mit Natriumchlorid.
Die erfindungsgemäße Azoverbindung liefert als Farbstoff mit guten faserreaktiven Farbstoffeigenschaften beispielsweise auf Cellulosefasermaterialien farbstarke Färbungen und Drucke von violetten Farbtönen bei hohem Fixiergrad.

### Beispiel 10

51,9 Teile des Natriumsalzes der Verbindung 4-(6',8'-Disulfo-naphth-2'-yl)-amino-3-amino-nitrobenzol werden in 200 Teilen Wasser bei 20 bis 25°C mit 9 Teilen Dimethylcarbonat versetzt. Die Umsetzung (Cyclisierung) und anschließende Hydrierung der Nitrogruppe zur Aminogruppe erfolgt analog den Angaben des Beispiels 9. Die erhaltene Lösung wird filtriert und das Filtrat bei 0 bis 5°C mit 30 Teilen einer 31 %igen wäßrigen Salzsäure versetzt; die Diazotierung der Verbindung erfolgt sodann in üblicher Weise mittels 7 Teilen Natriumnitrit. Man gibt die erhaltene Diazoniumsalzsuspension sodann unter Einhaltung eines pH-Wertes von 5 bis 6 zu einer Lösung von 23,0 Teilen 3-Amino-8-hydroxy-6-sulfo-naphthalin und führt die Kupplungsreaktion bei 18 bis 25°C und einem pH-Wert von 5 bis 6 zu Ende.

Die so erhaltene Azoverbindung wird durch Aussalzen mit Natriumchlorid isoliert und anschließend in 500 Teilen Wasser gelöst. Man gibt unter gutem Rühren bei einer Temperatur von 0 bis 5°C eine Suspension von 18 Teilen Cyanurchlorid in 150 Teilen Eiswasser hinzu und hält den pH bei einem Wert von 5 bis 6. Nach Beendigung der Umsetzung gibt man 27 Teile 4-(β-Sulfatoethylsulfonyl)-anilin hinzu, stellt einen pH-Wert von 6 ein und führt die Umsetzung bei 50°C unter Beibehaltung dieses pH-Wertes zu Ende.

Die erhaltene erfindungsgemäße Azoverbindung, die der Formel (in Form der freien Säure geschrieben) 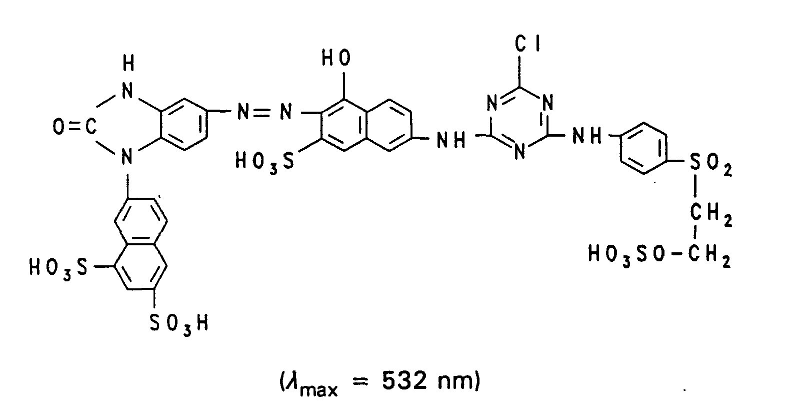 entspricht, wird in üblicher Weise, beispielsweise durch Sprühtrocknung der Syntheselösung, isoliert. Sie besitzt sehr gute faserreaktive Farbstoffeigenschaften und färbt beispielsweise Cellulosefasermaterialien in kräftigen roten Farbtönen bei hohem Fixiergrad.

### Beispiel 11

35,7 Teile des Natriumsalzes der Verbindung 4-(3'-Sulfophenyl)-amino-3-aminonitrobenzol in 200 Teilen Wasser werden bei einer Temperatur von 10 bis 15°C mit 12,6 Teilen Oxalsäuredichlorid versetzt. Man rührt den Ansatz noch einige Zeit bei einem pH-Wert zwischen 6 und 7 und einer Temperatur von zunächst 30 bis 40°C, sodann von 80 bis 90°C, weiter und reduziert in der erhaltenen heterocyclischen Verbindung die Nitrogruppe zur Aminogruppe in üblicher Weise mittels aktiviertem Raney-Nickel bei einem Wasserstoffdruck von 2 bar und einer Temperatur von 40 bis 60°C und einem pH-Wert von 9.

Die erhaltene Lösung wird von dem Raney-Nickel durch Filtration befreit und die in Lösung befindliche Aminoverbindung nach Zugabe von 30 Teilen einer 31 %igen wäßrigen Salzsäure in üblicher Weise mit 7 Teilen Natriumnitrit diazotiert. Die erhaltene Diazoniumsalzsuspension gibt man unter Einhaltung eines pH-Wertes von 5 bis 6 zu einer Lösung von 67,5 Teilen des Natriumsalzes der Verbindung 3-N-[2'-(4"-β-Sulfatoethylsulfonyl)-phenylamino-4'-chlor-1',3',5'-triazin-6'-yl]-amino-8-hydroxy-6-sulfo-naphthalin in 500 Teilen Wasser. Man führt die Kupplungsreaktion bei einem pH-Wert von 5 bis 6 und einer Temperatur von 18 bis 25°C zu Ende und isoliert sodann die erfindungsgemäße Azoverbindung in üblicher Weise, beispielsweise durch Aussalzen mit Natriumchlorid.

Sie besitzt, in Form der freien Säure geschrieben, die Formel

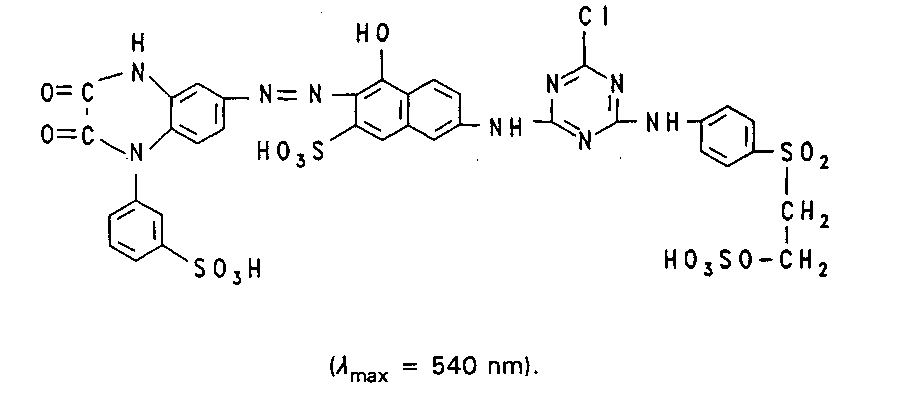

Sie zeigt sehr gute Farbstoffeigenschaften und färbt beispielsweise Cellulosefasermaterialien nach den in der Technik für faserreaktive Farbstoffe üblichen Färbe- und Druckverfahren in kräftigen roten Farbtönen bei hohem Fixiergrad.

### Beispiel 12

17,7 Teile 7-Amino-chinazolin-2,4-dion (bekannt aus Egypt. J. Chem. 20, 427 (1980)) werden in 300 Teilen Wasser nach Zugabe von 30 Teilen einer 31 %igen wäßrigen Salzsäure bei einer Temperatur von 0 bis 5°C in üblicher Weise mittels 7 Teilen Natriumnitrit diazotiert. Die erhaltene Diazoniumsalzsuspension gibt man sodann zu 1050 Teilen einer wäßrigen Lösung von 54,5 Teilen der in Beispiel 2 genannten Kupplungskomponente und führt die Kupplungsreaktion bei 18 bis 25°C und einem pH-Wert von 5 bis 6 durch.

Man isoliert die erfindungsgemäße Azoverbindung aus der Syntheselösung in üblicher Weise, beispielsweise durch Aussalzen mit Natriumchlorid. Sie besitzt, in Form der freien Säure geschrieben, die Formel 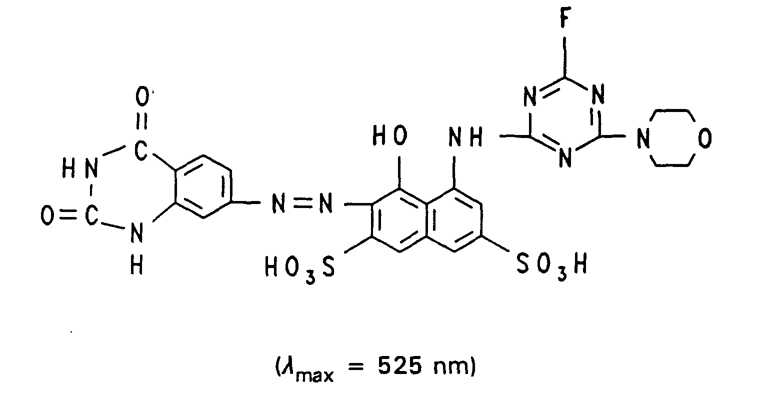 und liefert beispielsweise auf Cellulosefasermaterialien, wie beispielsweise Baumwolle, nach den für faserreaktive Farbstoffe üblichen Färbeweisen farbstarke, brillante rote Färbungen bei hohem Fixiergrad.

### Beispiel 13

Man diazotiert analog den Angaben des Beispieles 12 17,7 Teile 6-Amino-chinazolin-2,4-dion (bekannt aus Egypt. J. Chem., loc. cit.) und gibt die erhaltene Diazoniumsalzsuspension zu einer Lösung von 77,8 Teilen der Verbindung 1-N-[2'-(3''-β-Sulfatoethylsulfonyl)-phenylamino-4'-chlor-1',3',5'-triazin-6'-yl]-amino-8-hydroxy-3,6-disulfo-naphthalin in 1000 Teilen Wasser. Die Kupplungsreaktion erfolgt bei 18 bis 25°C und einem pH-Wert zwischen 5 und 6. Man isoliert die erfindungsgemäße Azoverbindung aus der wäßrigen Syntheselösung beispielsweise durch Aussalzen. Sie besitzt, in Form der freien Säure geschrieben, die Formel 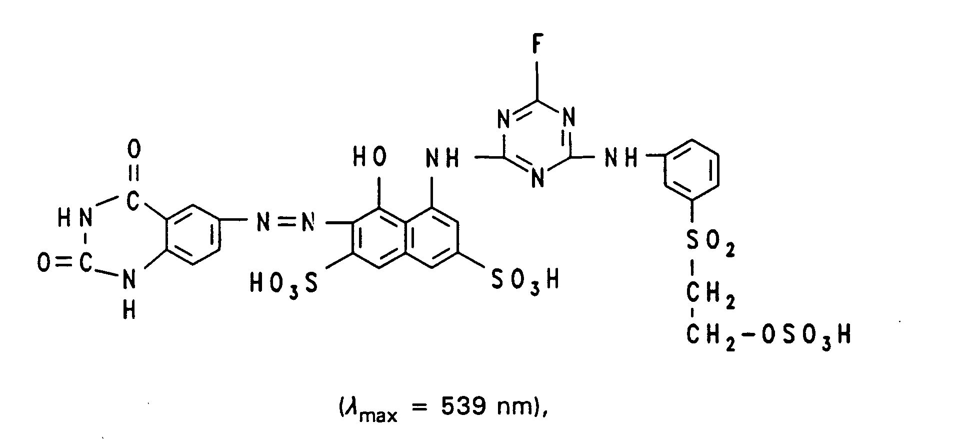 zeigt sehr gute faserreaktive Farbstoffeigenschaften und färbt beispielsweise Cellulosefasermaterialien, wie Baumwolle, nach den in der Technik für faserreaktive Farbstoffe üblichen Anwendungsverfahren in kräftigen, brillanten blaustichig roten Tönen bei hohem Fixiergrad.

### Beispiel 14

16,7 Teile 7-Amino-3,4-dihydro-chinolin-2-on (bekannt aus J. Chem. Soc. C 1969, 183) werden analog den Angaben des Beispieles 12 diazotiert. Man rührt sodann die erhaltene Diazoniumsalzsuspension bei einem pH-Wert von 5 bis 6 in eine Lösung von 23 Teilen 3-Amino-8-hydroxy-6-sulfo-naphthalin in 500 Teilen Wasser und führt die Kupplungsreaktion in diesem pH-Bereich und bei 18 bis 25°C durch. Die erhaltene Aminoazoverbindung wird durch Aussalzen mit Natriumchlorid isoliert und in 500 Teilen Wasser gelöst. Unter intensivem Rühren gibt man bei 0 bis 3°C und unter Einhaltung eines pH-Wertes von oberhalb 4 15 Teile Cyanurfluorid hinzu und führt die Umsetzung sodann bei 0 bis 3°C und einem pH-Wert von 5 zu Ende. Die zweite Kondensationsreaktion mit der erhaltenen Difluortriazinylamino-Verbindung erfolgt sodann nach Einstellen des pH-Wertes auf 6 durch Zugabe von 27 Teilen 3-(β-Chlorethylsulfonyl)-propylamin bei einem pH-Wert von 8 bis 9 und einer Temperatur von 10 bis 15°C.

Die erfindungsgemäße Azoverbindung der Formel (in Form der freien Säure geschrieben) 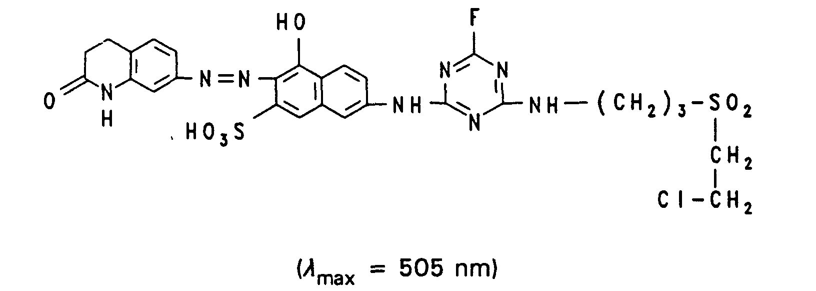 wird in üblicher Weise aus der wäßrigen Syntheselösung isoliert, beispielsweise durch Aussalzen mit Natriumchlorid. Sie zeigt sehr gute Farbstoffeigenschaften und liefert beispielsweise nach den in der Technik für faserreaktive Farbstoffe üblichen Färbe- und Druckverfahren auf den in der Beschreibung genannten Materialien, wie insbesondere Cellulosefasermaterialien, wie beispielsweise Baumwolle, kräftige Färbungen und Drucke in brillanten roten Tönen bei hohem Fixiergrad.

### Beispiel 15

14,8 Teile 6-Amino-1,3-dihydro-indol-2-on (bekannt aus EP-A-0 549 892) werden analog den Angaben des Beispieles 12 diazotiert. Die Diazoniumsalzsuspension gibt man sodann zu 1050 Teilen einer wäßrigen Lösung von 54,5 Teilen der Verbindung 2-N-{2'-[N'-Phenyl-N'-γ-(β'-sulfatoethylsulfonyl)-propyl]-amino-4'-fluor-1',3',5'-triazin-6'-yl}-amino-8-hydroxy-6-sulfo-naphthalin und führt die Kupplungsreaktion unter gutem Rühren bei 18 bis 25°C und einem pH-Wert von 5 bis 6 zu Ende.

Die erfindungsgemäße Azoverbindung der Formel (in Form der freien Säure geschrieben) 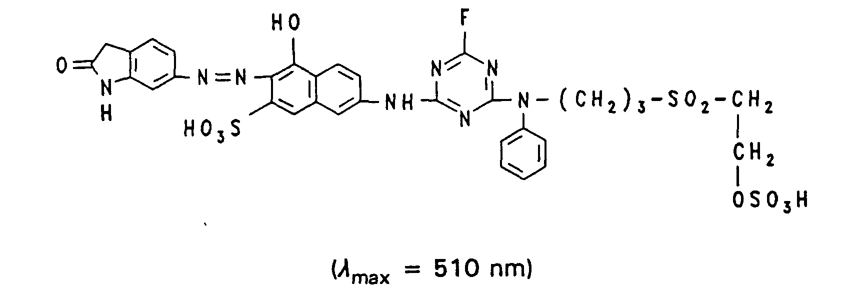 wird in üblicher Weise durch Aussalzen mit Natriumchlorid isoliert. Sie zeigt gute Farbstoffeigenschaften und färbt beispielsweise Cellulosefasermaterialien, wie Baumwolle, in farbstarken, brillanten roten Töne bei hohem Fixiergrad.

### Beispiel 16

Man diazotiert analog den Angaben des Beispieles 12 19,8 Teile 5-Amino-1,2-benzisothiazolin-3-on-1,1-dioxid(bekannt aus J. Macromol. Sci. 3, 941 (1969)) und rührt die erhaltene Diazoniumsalzsuspension in 500 Teile einer wäßrigen Lösung von 24 Teilen 3-Amino-8-hydroxy-6-sulfo-naphthalin, wobei man mittels Natriumcarbonat einen pH-Wert zwischen 5 und 6 einstellt und die Kupplungsreaktion innerhalb dieses pH-Bereiches und bei einer Temperatur zwischen 18 und 25°C zu Ende führt.

Die erhaltene Aminoazo-Verbindung wird durch Aussalzen mit Natriumchlorid aus der wäßrigen Syntheselösung isoliert und sodann unter Zusatz eines handelsüblichen Dispergiermittels in 1000 Teilen Wasser unter gutem Rühren gelöst. Man gibt 42 Teile der Verbindung N-Phenyl-N-(2,4-difluor-1,3,5-triazin-6-yl)-N-[β-(β'-sulfatoethylsulfonyl)-ethyl]-amin (bekannt aus EP-A-0 568 876) versetzt; die Umsetzung erfolgt bei einer Temperatur von 10 bis 15°C und einem pH-Wert von 6.

Man isoliert die erfindungsgemäße Azoverbindung der Formel (in Form der freien Säure geschrieben) 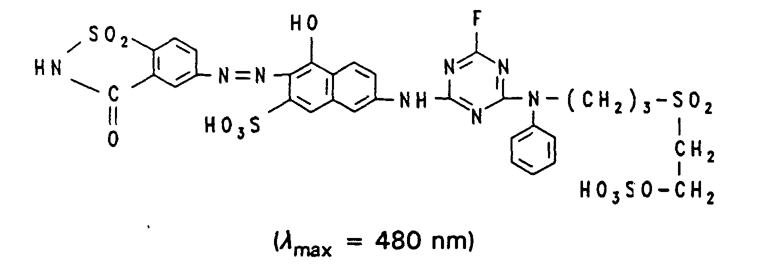 aus der wäßrigen Syntheselösung durch Aussalzen mit Natriumchlorid. Die Azoverbindung liefert nach den in der Technik für faserreaktive Farbstoffe üblichen Anwendungsverfahren beispielsweise auf Cellulosefasermaterialien, wie Baumwolle, farbstarke orange Färbungen bei hohem Fixiergrad.

### Beispiel 17

Analog den Angaben des Beispieles 12 diazotiert man 19,8 Teile 6-Amino-1,2-benzoisothiazolin-3-on-1,1-dioxid(bekannt aus J. Chem. Educ. 47, 649 (1970)) und rührt die erhaltene Diazoniumsalzsuspension in 1050 Teilen einer wäßrigen Lösung von 57,5 Teilen des Natriumsalzes der Verbindung 3-N-{2'-[N'-Phenyl-N'-β-(β'-sulfatoethylsulfonyl)-ethyl]-amino-4'-fluor-1',3',5'-triazin-6'-yl}-amino-8-hydroxy-6-sulfo-naphthalin und führt die Umsetzung unter weiterem Rühren bei einem pH-Wert zwischen 5 und 6 und einer Temperatur von 18 bis 20°C zu Ende.

Die erhaltene erfindungsgemäße Azoverbindung der Formel (in Form der freien Säure geschrieben) 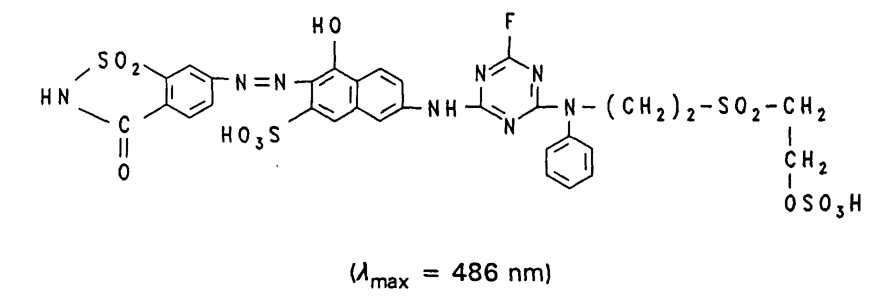 kann in üblicher Weise, beispielsweise durch Aussalzen mit Natriumchlorid, isoliert werden. Sie zeigt gute Farbstoffeigenschaften und liefert auf den in der Beschreibung genannten Materialien, wie insbesondere Cellulosefasermaterialien, wie beispielsweise Baumwolle, farbstarke brillante orange Färbungen bei hohem Fixiergrad.

### Beispiel 18

18,5 Teile 5-Chlor-6-amino-benzoxazolin-2-on (bekannt aus DE-PS 439 606) versetzt man in 300 Teilen Wasser mit 30 Teilen einer 31 %igen wäßrigen Salzsäure und diazotiert in üblicher Weise mittels 7 Teilen Natriumnitrit. Man rührt die erhaltene Diazoniumsalzsuspension sodann in 1050 Teilen einer wäßrigen Lösung von 65 Teilen der Verbindung 3-N-{2'-[3''-(β-Sulfatoethyl)-sulfonyl)-phenyl]-amino-4'-cyanoamino-1',3',5'-triazin-6'-yl}-amino-8-hydroxy-6-sulfo-naphthalin und führt die Kupplungsreaktion unter Rühren bei 18 bis 25°C und einem pH-Wert von 5 bis 6 durch.

Man isoliert die erfindungsgemäße Azoverbindung der Formel (in Form der freien Säure geschrieben) 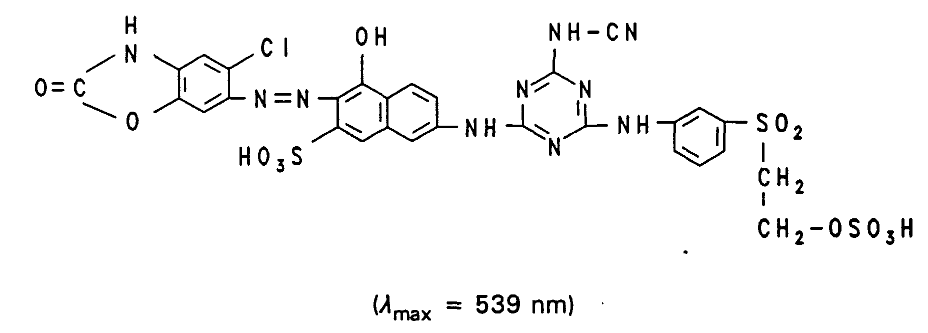 mittels Natriumchlorid. Man erhält mit ihr nach den in der Technik für faserreaktive Farbstoffe üblichen Färbe- und Druckverfahren auf den in der Beschreibung genannten Fasermaterialien, wie beispielsweise Cellulosefasermaterialien, wie Baumwolle, farbstarke brillante rote Färbungen und Drucke bei hohem Fixiergrad.

### Beispiel 19

Man diazotiert analog den Angaben des Beispieles 12 16,2 Teile 5-Amino-phthalimid in 530 Teilen einer salzsauren wäßrigen Lösung mittels 7 Teilen Natriumnitrit und rührt die erhaltene Diazoniumsalzsuspension unter Einhaltung eines pH-Wertes von 5 bis 6 in 1050 Teilen einer wäßrigen Lösung von 75,5 Teilen der Verbindung 3-N-{2'-[3''-(ß-Sulfatoethylsulfonyl)-phenyl]-amino-4'-chlor-1',3',5'-triazin-6'-yl]-amino-8-hydroxy-4,6-disulfo-naphthalin und führt die Kupplungsreaktion innerhalb dieses pH-Bereiches und bei 18 bis 25°C zu Ende. Man isoliert den erfindungsgemäßen Azofarbstoff aus der Syntheselösung durch Aussalzen mit Natriumchlorid. Sie besitzt, in Form der freien Säure geschrieben, die Formel

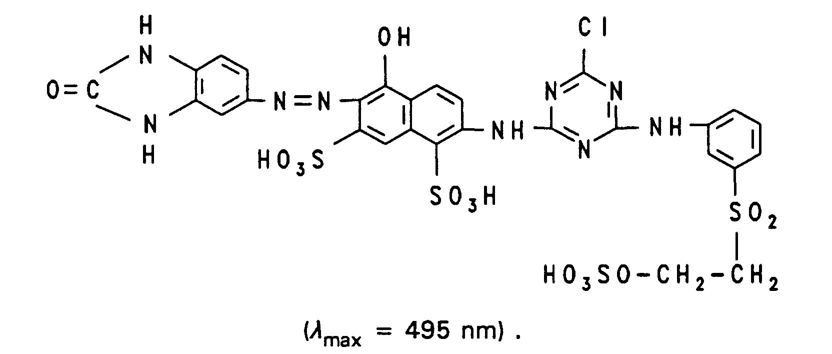

Sie liefert nach den in der Technik für faserreaktive Farbstoffe üblichen Färbe- und Druckverfahren beispielsweise auf Cellulosefasermaterialien farbstarke, brillante gelbstichig rote Färbungen und Drucke bei hohem Fixiergrad.

### Beispiele 20 bis 36

In den nachfolgenden Tabellenbeispielen sind weitere erfindungsgemäße Azoverbindungen entsprechend der allgemeinen Formel (A) 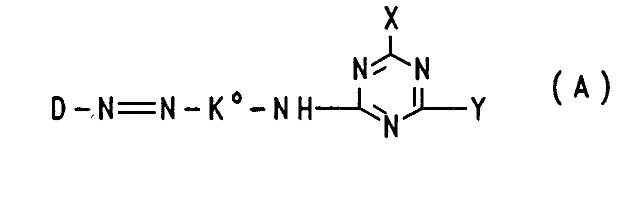 mit Hilfe deren Formelkomponenten beschrieben. Sie lassen sich in erfindungsgemäßer Weise, beispielsweise nach einem der obigen Beispiele, mittels der aus dem jeweiligen Tabellenbeispiel in Verbindung mit der Formel (A) ersichtlichen Komponenten (der Diazokomponente D-NH₂ , der Kupplungskomponente H-K⁰-NH₂ , Cyanurfluorid oder Cyanurchlorid, gegebenenfalls einer Aminoverbindung H-X und einer Verbindung H-Y) herstellen. Sie besitzen sehr gute faserreaktive Farbstoffeigenschaften und liefern auf den in der Beschreibung genannten Materialien, insbesondere Cellulosefasermaterialien, wie Baumwolle, nach den bekannten Färbe- und Druckverfahren farbstarke, echte Färbungen und Drucke in dem in dem jeweiligen Tabellenbeispiel für Färbungen auf Baumwolle angegebenen Farbton.

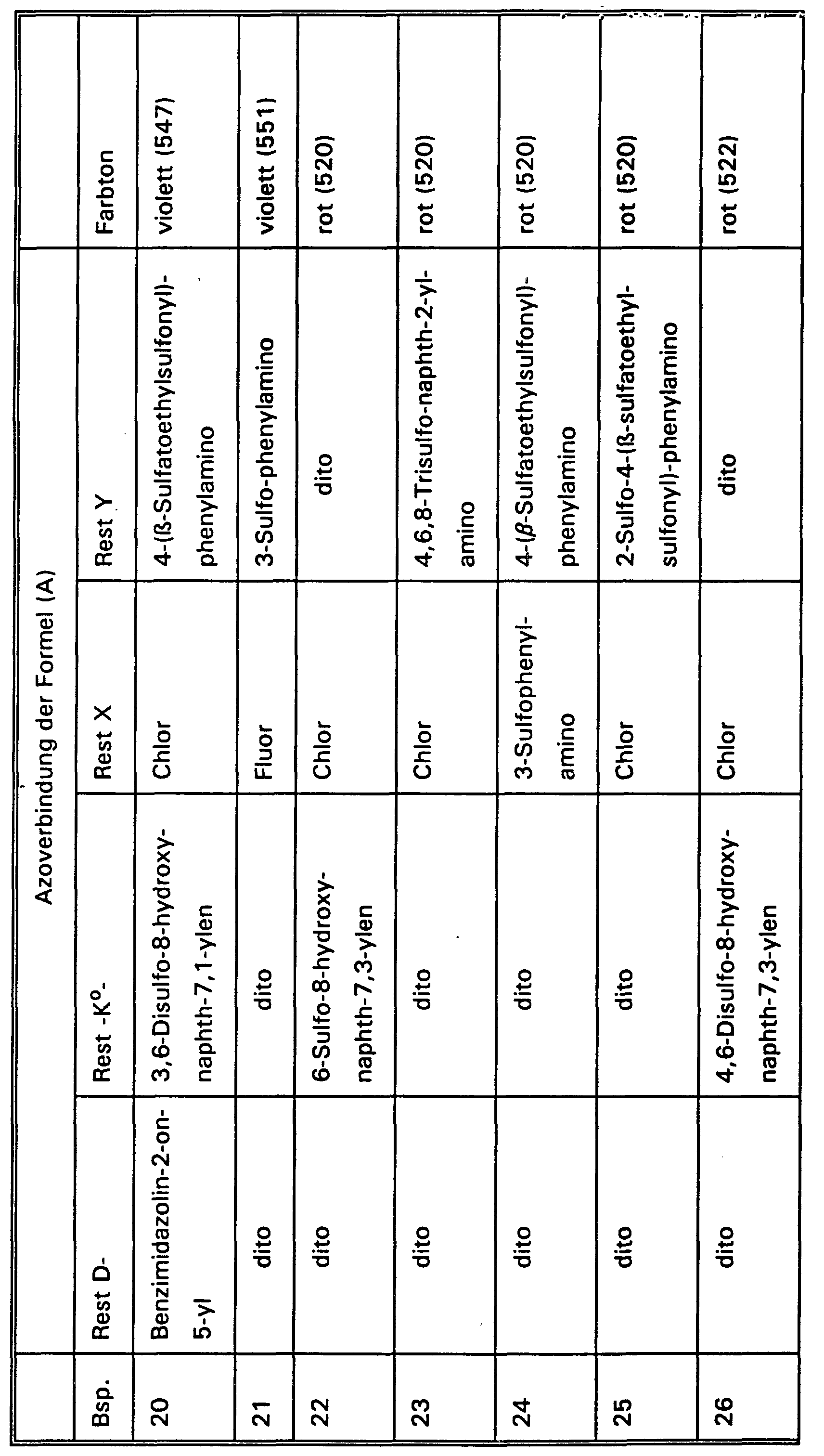

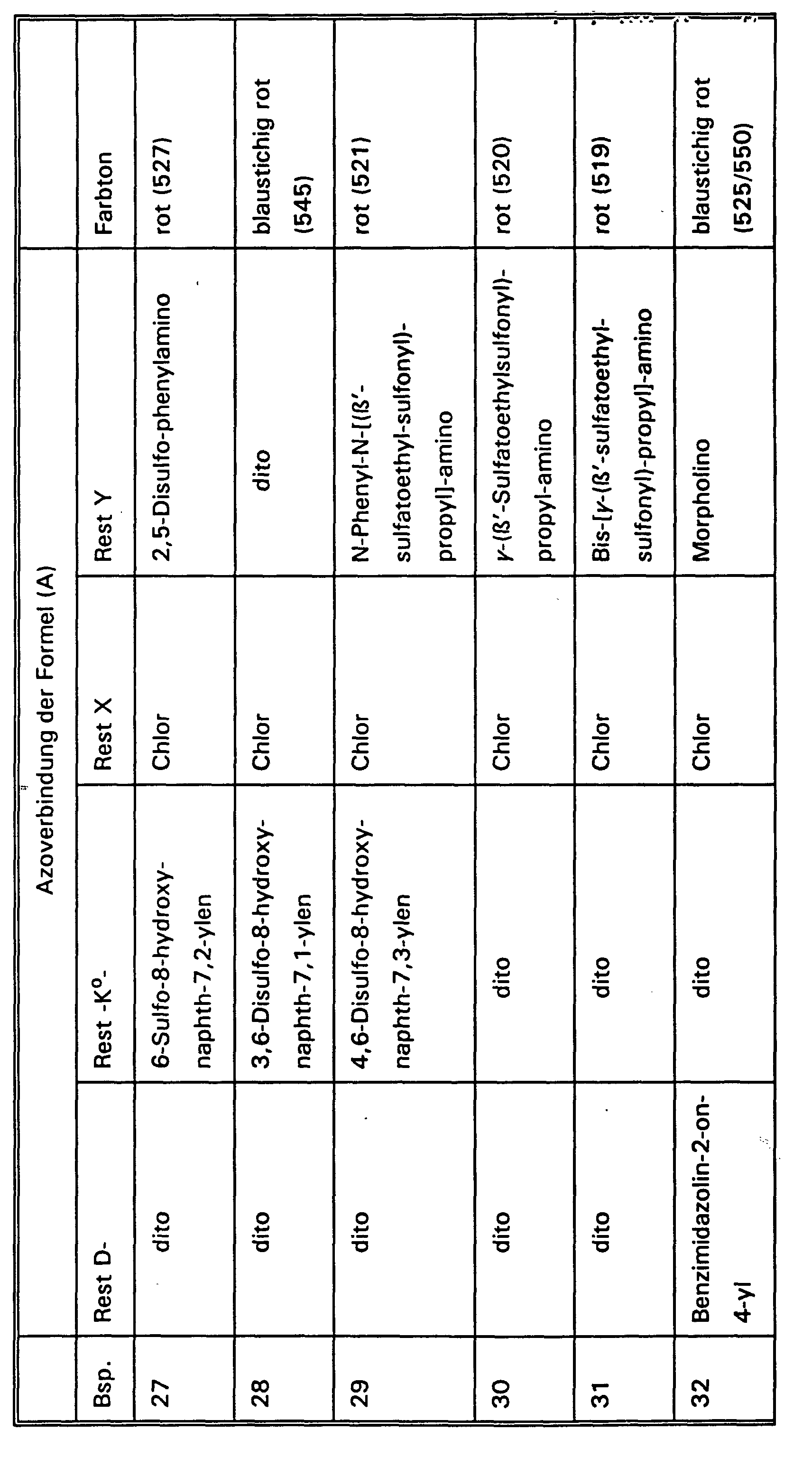

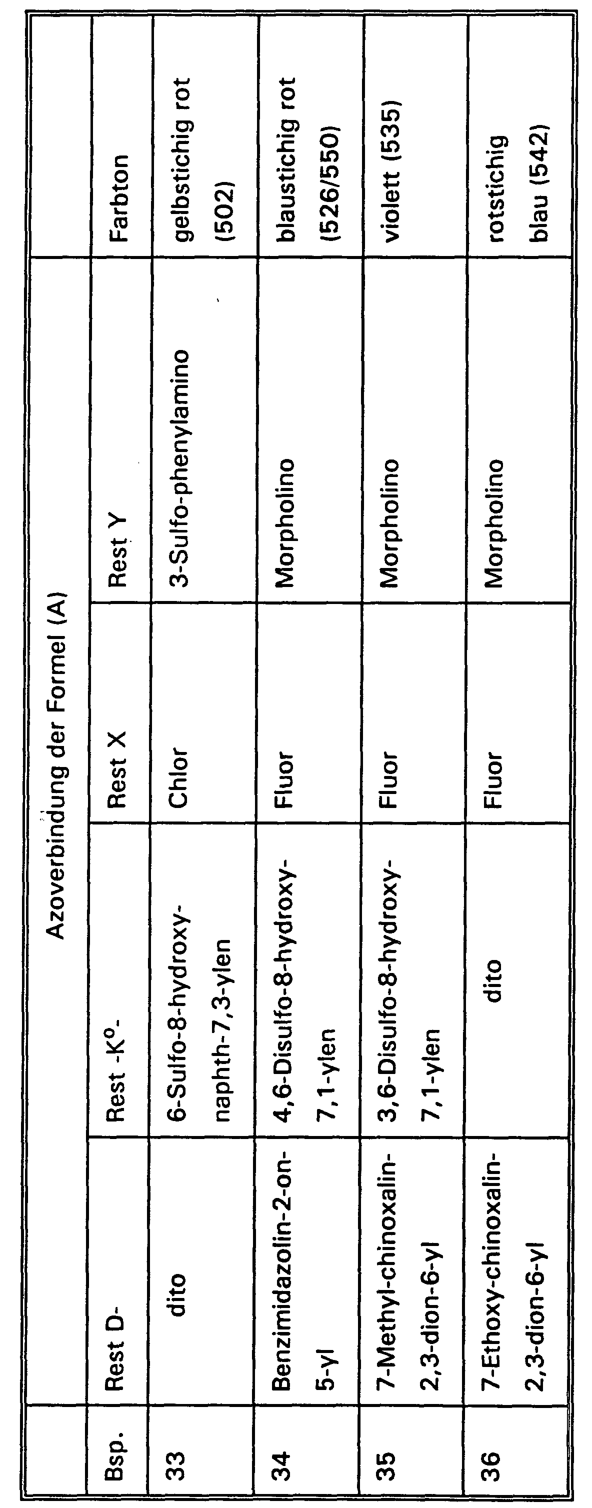

## Patentansprüche

1. Azoverbindung entsprechend der allgemeinen Formel (1) 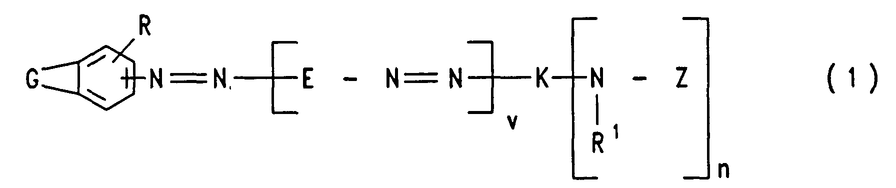 in welcher bedeuten:
G bildet zum Benzolring den Rest eines von olefinischen Doppelbindungen freien Heterocyclus, der mindestens eine Carbonamidgruppe der Formel -CO-N(R¹⁰)- enthält, in welcher R¹⁰ Wasserstoff, Alkyl von 1 bis 4 C-Atomen oder gegebenenfalls durch 1 bis 3 Substituenten aus der Gruppe Sulfo, Carboxy, Alkoxy von 1 bis 4 C-Atomen, Alkyl von 1 bis 4 C-Atomen, Halogen, Cyano, Nitro und Amino substituiertes Aryl von 6 bis 10 C-Atomen ist, und der gegebenenfalls 1 oder 2 weitere Heterogruppen aus der Reihe -O- , -S- und -N(R¹⁰)- mit R¹⁰ der oben genannten Bedeutung besitzt;
R ist Wasserstoff, Alkyl von 1 bis 4 C-Atomen, Alkoxy von 1 bis 4 C-Atomen, Halogen oder Sulfo:
R¹ ist Wasserstoff, Alkyl von 1 bis 4 C-Atomen oder Alkyl von 1 bis 4 C-Atomen, das durch Hydroxy, Cyano, Alkoxy von 1 bis 4 C-Atomen, Carboxy, Sulfo, Sulfato oder Phosphato substituiert ist, oder ist Phenyl oder Naphthyl, die beide durch 1, 2 oder 3 Substituenten aus der Gruppe Halogen, Hydroxy, Cyano, Alkoxy von 1 bis 4 C-Atomen, Alkyl von 1 bis 4 C-Atomen, Alkoxycarbonyl von 2 bis 5 C-Atomen, Carboxy, Sulfamoyl, Sulfo und Alkyl Alkylsulfonyl mit einem Alkylrest von 1 bis 4 C-Atomen substituiert sein können;
E ist der bivalente, von der Aminogruppe freie Rest einer kupplungsfähigen und diazotierbaren Verbindung aus der Anilin- oder Naphthylamin-Reihe;
v steht für die Zahl Null, 1 oder 2;
K ist der bivalente, von der Aminogruppe freie Rest einer Kupplungskomponente der Anilin- oder Naphthylamin-Reihe oder der bivalente Rest einer Kupplungskomponente der heterocyclischen Reihe;
n ist die Zahl 1, 2, 3 oder 4;
Z ist der faserreaktive Rest einer faserreaktiven Gruppierung oder Gruppe -N(R¹)-Z, wobei im Falle von n größer als 1 die Reste -N(R¹)-Z voneinander verschiedene Bedeutungen haben können;
die Verbindungen der Formel (1) besitzen mindestens eine Sulfogruppe.

2. Azoverbindung nach Anspruch 1, dadurch gekennzeichnet, daß G ein Rest der Formel (2a), (2b), (2c), (2d), (2e), (2f), (2g) oder (2h) 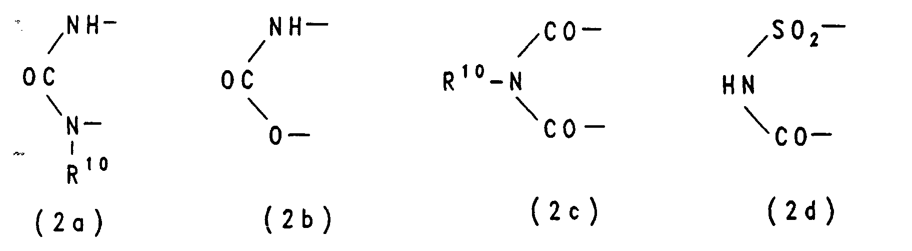 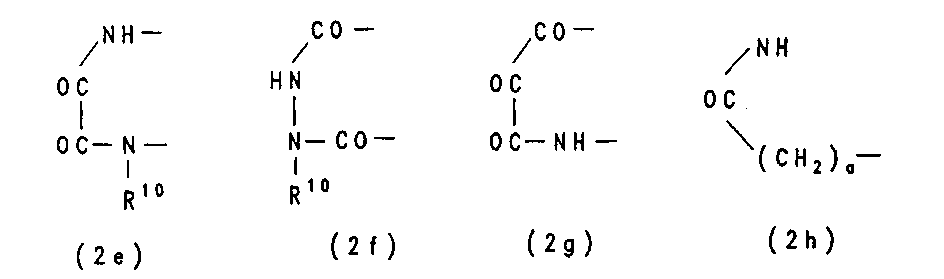 ist, in welchen R¹⁰ die in Anspruch 1 genannten Bedeutung hat und in Formel (2h) der Index a für die Zahl 1 oder 2 steht.

3. Azoverbindung nach Anspruch 1, dadurch gekennzeichnet, daß Z ein Rest der allgemeinen Formel (3) 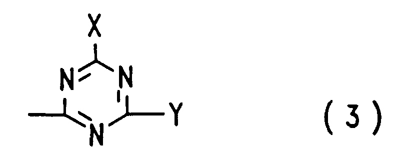 ist, in welcher bedeuten:
X ist Halogen oder eine Gruppe der allgemeinen Formel (4) 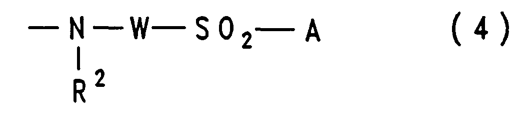 in welcher
R² Wasserstoff oder Alkyl von 1 bis 4 C-Atomen ist oder Alkyl von 1 bis 4 C-Atomen bedeutet, das durch Hydroxy, Cyano, Alkoxy von 1 bis 4 C-Atomen, Carboxy, Sulfo, Sulfato oder Phosphato substituiert ist, oder Phenyl oder Naphthyl ist, die beide durch 1, 2 oder 3 Substituenten aus der Gruppe Halogen, Hydroxy, Cyano, Alkoxy von 1 bis 4 C-Atomen, Alkyl von 1 bis 4 C-Atomen, Alkoxycarbonyl von 2 bis 5 C-Atomen, Carboxy, Sulfamoyl, Sulfo und Alkylsulfonyl mit einem Alkylrest von 1 bis 4 C-Atomen, substituiert sein können,
W Alkylen von 2 bis 4 C-Atomen ist oder Alkylen von 3 bis 6 C-Atomen ist, das durch 1 oder 2 Heterogruppen aus der Gruppe der Formeln -O- , -NH- , -SO₂- , -CO- und -N(R¹⁰)- mit R¹⁰ der in Anspruch 1 genannten Bedeutung unterbrochen ist, oder Phenylen oder Naphthylen bedeutet, die beide durch 1, 2 oder 3 Substituenten aus der Gruppe Halogen, Hydroxy, Cyano, Nitro, Alkoxy von 1 bis 4 C-Atomen, Alkyl von 1 bis 4 C-Atomen, Alkoxycarbonyl von 2 bis 5 C-Atomen, Carboxy, Sulfamoyl und Sulfo substituiert sein können, oder Phenylenalkylen oder Alkylenphenylen mit einem Alkylenrest von jeweils 1 bis 4 C-Atomen ist und
A Vinyl ist oder Ethyl bedeutet, das in β-Stellung durch einen mittels Alkali unter Bildung der Vinylgruppe abspaltbaren Substituenten substituiert ist;
Y ist Chlor, Cyanoamino oder eine Gruppe der allgemeinen Formel (5) 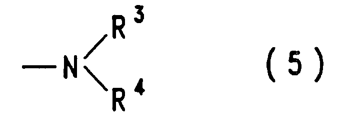 in welcher
R³ Wasserstoff oder Alkyl von 1 bis 4 C-Atomen ist oder Alkyl von 1 bis 4 C-Atomen bedeutet, das durch Halogen, Hydroxy, Cyano, Alkoxy von 1 bis 4 C-Atomen, Alkoxycarbonyl von 2 bis 5 C-Atomen, Carboxy, Sulfo, Sulfato oder Phosphato substituiert ist, oder eine Gruppe der Formel -W-SO₂-A mit W und A einer der obengenannten Bedeutungen ist und
R⁴ Wasserstoff oder Alkyl von 1 bis 4 C-Atomen ist oder Alkyl von 1 bis 4 C-Atomen bedeutet, das durch Halogen, Hydroxy, Cyano, Alkoxy von 1 bis 4 C-Atomen, Alkoxycarbonyl von 2 bis 5 C-Atomen, Carboxy, Sulfo, Sulfato oder Phosphato substituiert ist, oder Cycloalkyl von 5 bis 8 C-Atomen oder eine Gruppe der Formel -W-SO₂-A mit W und A einer der obengennanten Bedeutungen ist oder Phenyl oder Naphthyl ist, die beide durch 1, 2 oder 3 Substituenten aus der Gruppe Halogen, Hydroxy, Cyano, Alkoxy von 1 bis 4 C-Atomen, Alkyl von 1 bis 4 C-Atomen, Alkoxycarbonyl von 2 bis 5 C-Atomen, Carboxy, Sulfamoyl, Sulfo und Alkylsulfonyl mit einem Alkylrest von 1 bis 4 C-Atomen, substituiert sein können, oder
R³ und R⁴ gemeinsam einen Alkylenrest von 3 bis 6 C-Atomen oder einen durch eine Gruppe -NH- , -O- , -CO- , -S- , -SO₂- oder -N(R⁵)-(worin R⁵ durch Sulfo oder Sulfato substituiertes Alkyl von 1 bis 4 C-Atomen, wie Ethyl, ist) unterbrochenen Alkylenrest von 3 bis 6 C-Atomen darstellen, die zusammen mit dem N-Atom einen
heterocyclischen Rest bilden.

4. Azoverbindung nach mindestens einem der Ansprüche 1 bis 3, dadurch gekennzeichnet, daß R¹ Wasserstoff, Methyl oder Ethyl ist.

5. Azoverbindung nach Anspruch 3 oder 4, dadurch gekennzeichnet, daß X Chlor oder Fluor ist.

6. Azoverbindung nach mindestens einem der Ansprüche 3 bis 5, dadurch gekennzeichnet, daß Y eine Gruppe der allgemeinen Formel (4) 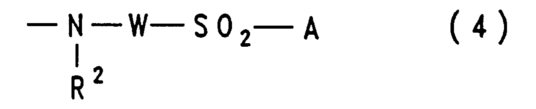 ist, in welcher
R² Wasserstoff, Alkyl von 1 bis 4 C-Atomen oder Alkyl von 1 bis 4 C-Atomen C-Atomen, ist, das durch Hydroxy, Cyano, Alkoxy von 1 bis 4 Carboxy, Sulfo, Sulfato oder Phosphato substituiert ist, oder Phenyl oder Naphthyl ist, die beide durch 1, 2 oder 3 Substituenten aus der Gruppe Halogen, Hydroxy, Cyano, Alkoxy von 1 bis 4 C-Atomen, Alkyl von 1 bis 4 C-Atomen, Alkoxycarbonyl von 2 bis 5 C-Atomen, Carboxy, Sulfamoyl, Sulfo und Alkylsulfonyl mit einem Alkylrest von 1 bis 4 C-Atomen substituiert sein können,
W Alkylen von 2 bis 4 C-Atomen ist oder Alkylen von 3 bis 6 C-Atomen ist, das durch 1 oder 2 Heterogruppen aus der Gruppe der Formeln -O- , -NH- , -SO₂- , -CO- und -N(R¹⁰)- mit R¹⁰ der in Anspruch 1 genannten Bedeutung unterbrochen ist, oder Phenylen oder Naphthylen bedeutet, die beide durch 1, 2 oder 3 Substituenten aus der Gruppe Halogen, Hydroxy, Cyano, Nitro, Alkoxy von 1 bis 4 C-Atomen, Alkyl von 1 bis 4 C-Atomen, Alkoxycarbonyl von 2 bis 5 C-Atomen, Carboxy, Sulfamoyl und Sulfo substituiert sein können, oder Phenylenalkylen oder Alkylenphenylen mit einem Alkylenrest von jeweils 1 bis 4 C-Atomen ist und
A Vinyl ist oder Ethyl bedeutet, das in β-Stellung durch einen mittels Alkali unter Bildung der Vinylgruppe abspaltbaren Substituenten substituiert ist.

7. Azoverbindung nach mindestens einem der Ansprüche 3 bis 5, dadurch gekennzeichnet, daß Y Morpholino ist.

8. Azoverbindung nach mindestens einem der Ansprüche 3 bis 5, dadurch gekennzeichnet, daß Y Mono- oder Disulfophenyl oder Mono-, Di- oder Trisulfonaphthyl ist.

9. Azoverbindung nach Anspruch 3, dadurch gekennzeichnet, daß Z ein Rest der allgemeinen Formel (8a) 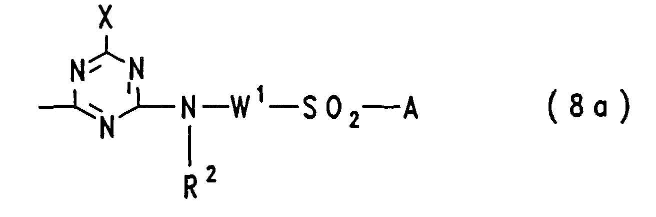 ist, in welcher X Chlor, Fluor oder Cyanoamino ist, A eine der in Anspruch 2 genannten, insbesonders bevorzugten, Bedeutungen hat, R² Wasserstoff, Ethyl, Methyl, β-Hydroxyethyl, β-Sulfatoethyl, Phenyl, 3-Sulfophenyl oder 4-Sulfophenyl ist und W¹ 1,2-Ethylen, 1,3-Propylen, 2-Methyl-1,2-ethylen, 2-Methyl-1,3-propylen, 1,4-Phenylen, 1,3-Phenylen, 2-Methoxy-1,5-phenylen, 2,5-Dimethoxy-1,4-phenylen, 2-Methoxy-5-methyl-1,4-phenylen, 2-Sulfo-1,4-phenylen, 2-Hydroxy-1,5-phenylen, 2-Brom-1,5-phenylen oder 4-(2'-Eth)-phen-1,1'-ylen, wobei in diesen Gruppen die 1-Position mit dem N-Atom verbunden ist, oder 1-Sulfonaphth-2,6-ylen, 6-Sulfo-naphth-2,8-ylen, Naphth-2,6-ylen oder Naphth-2,8-ylen ist, wobei in diesen Gruppen die 2-Position mit dem N-Atom verbunden ist.

10. Azoverbindung nach Anspruch 1 der allgemeinen Formel (1A) 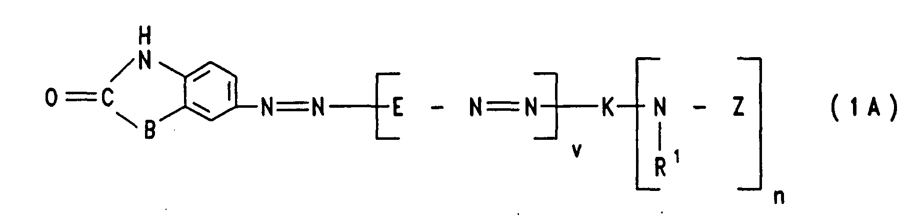 in welcher E, v, K, R¹ und n die in Anspruch 1 genannten Bedeutungen haben, Z eine der in mindestens einem der Ansprüche 1 bis 9 genannten Bedeutungen besitzt und B für -O- oder -N(R¹⁰)- steht, worin R¹⁰ Wasserstoff, Phenyl, Monosulfophenyl, Disulfophenyl, Trisulfophenyl, Naphthyl, Monosulfonaphthyl, Disulfonaphthyl oder Trisulfonaphthyl ist.

11. Azoverbindung nach Anspruch 1 der allgemeinen Formel (1B) 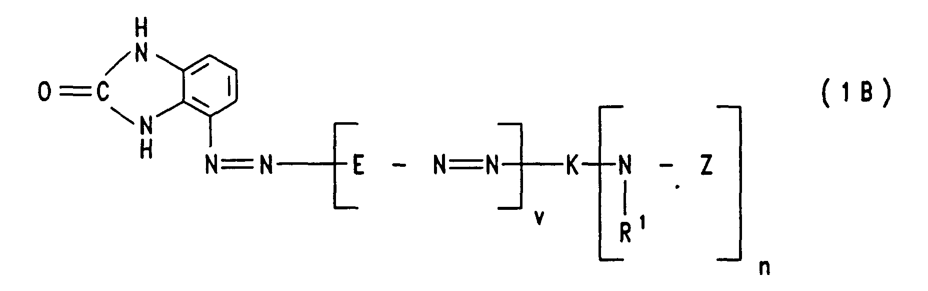 in welcher E, v, K, R¹ und n die in Anspruch 1 genannten Bedeutungen haben und Z eine der in mindestens einem der Ansprüche 1 bis 9 genannten Bedeutungen besitzt.

12. Azoverbindung nach Anspruch 1 der allgemeinen Formel (1C) 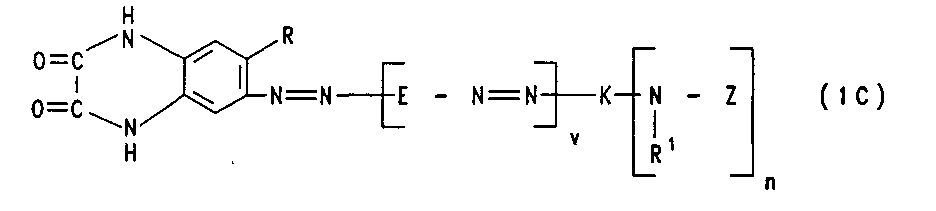 in welcher E, v, K, R¹ und n die in Anspruch 1 genannten Bedeutungen haben und Z eine der in mindestens einem der Ansprüche 1 bis 9 genannten Bedeutungen besitzt und R Wasserstoff, Methyl oder Methoxy ist.

13. Azoverbindung nach Anspruch 1 der allgemeinen Formel (1E) 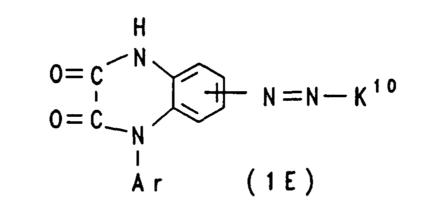 in welcher Ar durch 1 oder 2 Sulfogruppen substituiertes Phenyl oder durch 1, 2 oder 3 Sulfogruppen substituiertes Naphthyl ist und K¹⁰ eine Gruppe der allgemeinen Formel (8A) 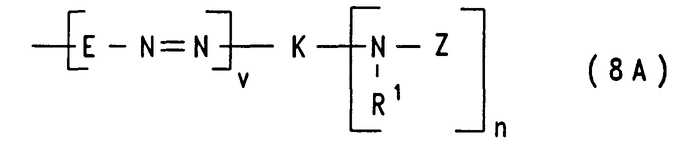 ist, in welcher E, v, K, R¹ und n die in Anspruch 1 genannten Bedeutungen haben und Z eine der in mindestens einem der Ansprüche 1 bis 9 genannten Bedeutungen besitzt.

14. Azoverbindung nach Anspruch 1 der allgemeinen Formel (1F) 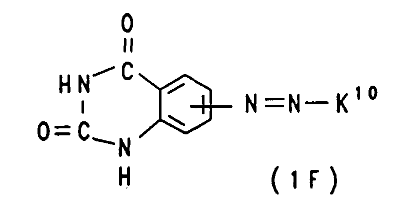 in welcher K¹⁰ eine Gruppe der allgemeinen Formel (8A) 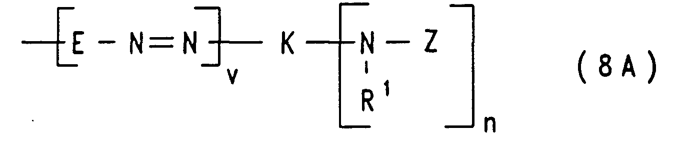 ist, in welcher E, v, K, R¹ und n die in Anspruch 1 genannten Bedeutungen haben und Z eine der in mindestens einem der Ansprüche 1 bis 9 genannten Bedeutungen besitzt.

15. Azoverbindung nach Anspruch 1 der allgemeinen Formel (1G) 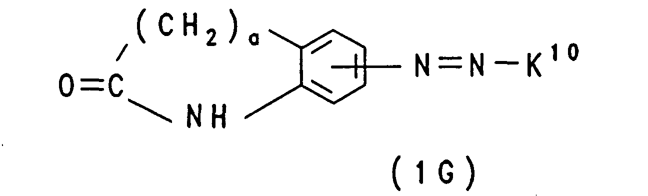 in welcher a die Zahl 1 oder 2 und K¹⁰ eine Gruppe der allgemeinen Formel (8A) 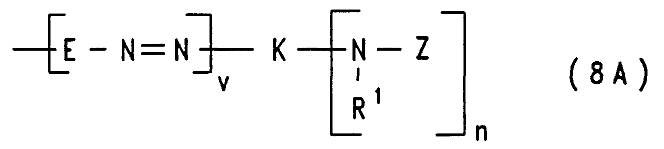 ist, in welcher E, v, K, R¹ und n die in Anspruch 1 genannten Bedeutungen haben und Z eine der in mindestens einem der Ansprüche 1 bis 9 genannten Bedeutungen besitzt.

16. Azoverbindung nach Anspruch 1 der allgemeinen Formel (1H) 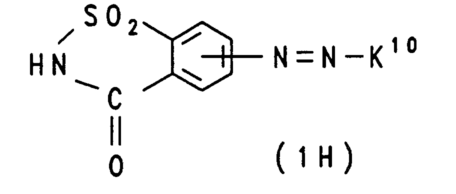 in welcher K¹⁰ eine Gruppe der allgemeinen Formel (8A) 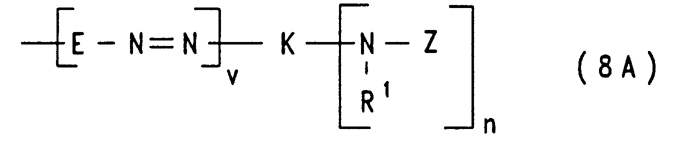 ist, in welcher E, v, K, R¹ und n die in Anspruch 1 genannten Bedeutungen haben und Z eine der in mindestens einem der Ansprüche 1 bis 9 genannten Bedeutungen besitzt.

17. Verfahren zur Herstellung einer Azoverbindung der in Anspruch 1 genannten und definierten allgemeinen Formel (1), dadurch gekennzeichnet, daß man eine Verbindung der allgemeinen Formel (12) 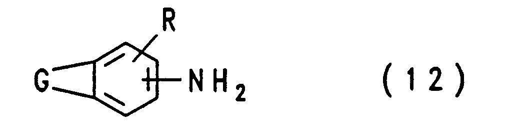 in welcher R und G eine der in Anspruch 1 genannten Bedeutungen haben, diazotiert und mit einer Verbindung der allgemeinen Formel (13) 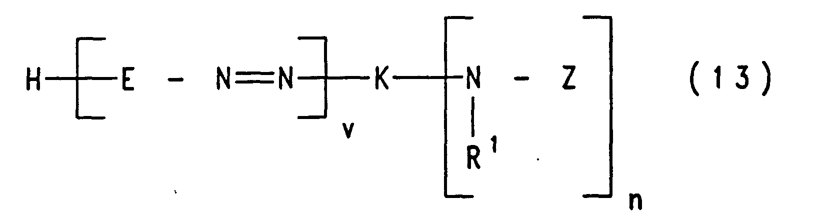 in welcher E, v, K, R¹, Z und n eine der in Anspruch 1 genannten Bedeutungen haben, kuppelt,
oder daß man eine Verbindung der allgemeinen Formel (14) 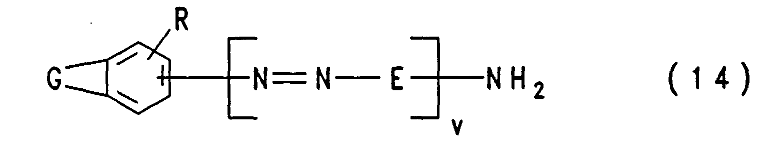 mit G, R, E und v einer der in Anspruch 1 genannten Bedeutungen diazotiert und mit einer Verbindung der allgemeinen Formel H-K-N(R¹)-Z mit K, R¹ und Z der in Anspruch 1 genannten Bedeutung kuppelt, oder daß man eine Verbindung der allgemeinen Formel (15) 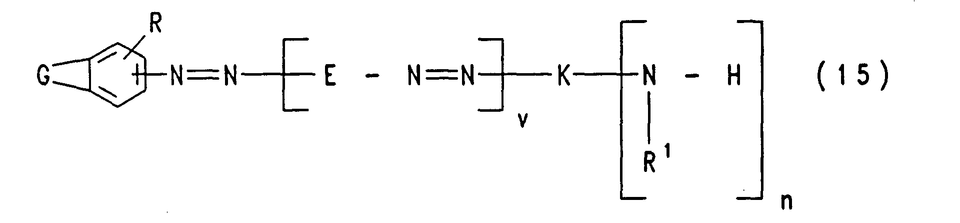 in welcher G, R, v, K, R¹ und n eine der in Anspruch 1 genannten, Bedeutungen haben, mit einer Verbindung der allgemeinen Formel X⁰-Z, in welcher X⁰ für Chlor, Brom oder Fluor steht und Z die in Anspruch 1 genannte Bedeutung besitzt, umsetzt.

18. Verwendung eines Farbstoffes von Anspruch 1 oder eines nach Anspruch 17 hergestellten Farbstoffes zum Färben (einschließlich Bedrucken) von hydroxy- und/oder carbonamidgruppenhaltigem Material, insbesondere Fasermaterial.

19. Verfahren zum Färben (einschließlich Bedrucken) von hydroxy- und/oder carbonamidgruppenhaltigem Material, insbesondere Fasermaterial, bei welchem man einen Farbstoff auf das Material aufbringt und den Farbstoff auf dem Material mittels Wärme oder mit Hilfe eines alkalisch wirkenden Mittels oder mittels beider Maßnahmen fixiert, dadurch gekennzeichnet, daß man als Farbstoff einen Farbstoff von Anspruch 1 oder einen nach Anspruch 17 hergestellten Farbstoff einsetzt.

## Claims

1. An azo compound of the formula (1) 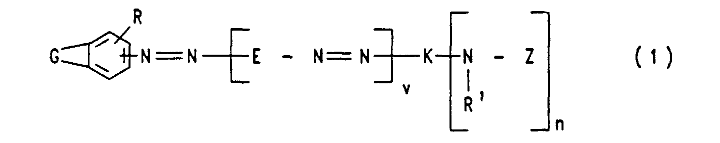 in which:
G forms to the benzene ring the radical of a heterocycle which is free from olefinic double bonds and which contains at least one carboxamide group of the formula -CO-N(R¹⁰)- in which R¹⁰ is hydrogen, alkyl of 1 to 4 carbon atoms, or aryl of 6 to 10 carbon atoms which may be substituted by from 1 to 3 substituents from the group consisting of sulfo, carboxyl, alkoxy of 1 to 4 carbon atoms, alkyl of 1 to 4 carbon atoms, halogen, cyano, nitro and amino, and which if desired possesses 1 or 2 further hetero-groups from the series consisting of -O-, -S- and -N(R¹⁰)- where R¹⁰ is as defined above;
R is hydrogen, alkyl of 1 to 4 carbon atoms, alkoxy of 1 to 4 carbon atoms, halogen or sulfo;
R¹ is hydrogen, alkyl of 1 to 4 carbon atoms or alkyl of 1 to 4 carbon atoms which is substituted by hydroxyl, cyano, alkoxy of 1 to 4 carbon atoms, carboxyl, sulfo, sulfato or phosphato, or is phenyl or naphthyl, each of which may be substituted by 1, 2 or 3 substituents from the group consisting of halogen, hydroxyl, cyano, alkoxy of 1 to 4 carbon atoms, alkyl of 1 to 4 carbon atoms, alkoxycarbonyl of 2 to 5 carbon atoms, carboxyl, sulfamoyl, sulfo and alkylsulfonyl having an alkyl radical of 1 to 4 carbon atoms;
E is the bivalent radical, free from the amino group, of a compound from the aniline or naphthylamine series which is capable of coupling and can be diazotized;
v is the number zero, 1 or 2;
K is the bivalent radical, free from the amino group, of a coupling component from the aniline or naphthylamine series or the bivalent radical of a coupling component from the heterocyclic series;
n is the number 1, 2, 3 or 4;
z is the fiber-reactive radical of a fiber-reactive grouping or group -N(R¹)-Z, in which case, if n is greater than 1, it is possible for the radicals -N(R¹)-Z to have definitions which are different from one another;
the compound of the formula (1) possesses at least one sulfo group.

2. An azo compound as claimed in claim 1, wherein G is a radical of the formula (2a), (2b), (2c), (2d), (2e), (2f), (2g) or (2h) 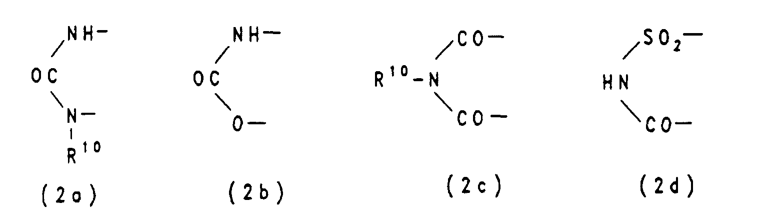 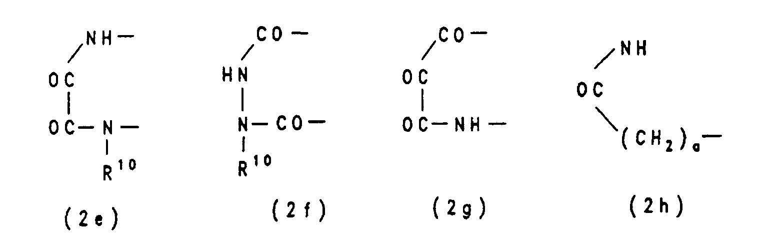 in which R¹⁰ is as defined in claim 1 and in formula (2h) the index a is the number 1 or 2.

3. An azo compound as claimed in claim 1, wherein Z is a radical of the formula (3) 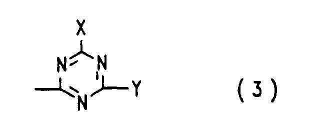 in which:
X is halogen or a group of the formula (4) 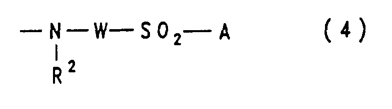
in which
R² is hydrogen or alkyl of 1 to 4 carbon atoms or alkyl of 1 to 4 carbon atoms which is substituted by hydroxyl, cyano, alkoxy of 1 to 4 carbon atoms, carboxyl, sulfo, sulfato or phosphato, or is phenyl or naphthyl, each of which may be substituted by 1, 2 or 3 substituents from the group consisting of halogen, hydroxyl, cyano, alkoxy of 1 to 4 carbon atoms, alkyl of 1 to 4 carbon atoms, alkoxycarbonyl of 2 to 5 carbon atoms, carboxyl, sulfamoyl, sulfo and alkylsulfonyl having an alkyl radical of 1 to 4 carbon atoms,
W is alkylene of 2 to 4 carbon atoms or is alkylene of 3 to 6 carbon atoms which is interrupted by 1 or 2 hetero-groups from the group consisting of the formulae -O-, -NH-, -SO₂-, -CO- and -N(R¹⁰)- where R¹⁰ is as defined in claim 1, or is phenylene or naphthylene, each of which may be substituted by 1, 2 or 3 substituents from the group consisting of halogen, hydroxyl, cyano, nitro, alkoxy of 1 to 4 carbon atoms, alkyl of 1 to 4 carbon atoms, alkoxycarbonyl of 2 to 5 carbon atoms, carboxyl, sulfamoyl and sulfo, or is phenylenealkylene or alkylenephenylene having an alkylene radical of in each case 1 to 4 carbon atoms, and
A is vinyl or is ethyl which is substituted in the β position by a substituent which can be eliminated using alkali to form the vinyl group;
Y is chlorine, cyanoamino or a group of the formula (5) 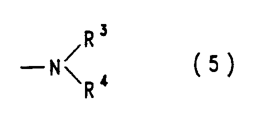 in which
R³ is hydrogen or alkyl of 1 to 4 carbon atoms or alkyl of 1 to 4 carbon atoms which is substituted by halogen, hydroxyl, cyano, alkoxy of 1 to 4 carbon atoms, alkoxycarbonyl of 2 to 5 carbon atoms, carboxyl, sulfo, sulfato or phosphato, or is a group of the formula -W-SO₂-A where W and A are as defined above, and
R⁴ is hydrogen or alkyl of 1 sulfonyl having an alkyl radical of 1 to 4 carbon atoms, or
R³ and R⁴ together are an alkylene radical of 3 to 6 carbon atoms or an alkylene radical of 3 to 6 carbon atoms which is interrupted by a group -NH-, -O-, -CO-, -S-, -SO₂- or -N(R⁵)- (in which R⁵ is sulfo- or sulfato-substituted alkyl of 1 to 4 carbon atoms such as ethyl) which together with the nitrogen atom form a heterocyclic radical.

4. An azo compound as claimed in at least one of claims 1 to 3, wherein R¹ is hydrogen, methyl or ethyl.

5. An azo compound as claimed in claim 3 or 4, wherein X is chlorine or fluorine.

6. An azo compound as claimed in at least one of claims 3 to 5, wherein Y is a group of the formula (4) 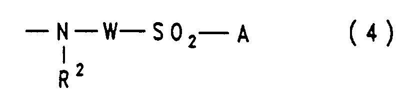 in which
R² is hydrogen, alkyl of 1 to 4 carbon atoms or alkyl of 1 to 4 carbon atoms which is substituted by hydroxyl, cyano, alkoxy of 1 to 4 carbon atoms, carboxyl, sulfo, sulfato or phosphato, or is phenyl or naphthyl, each of which may be substituted by 1, 2 or 3 substituents from the group consisting of halogen, hydroxyl, cyano, alkoxy of 1 to 4 carbon atoms, alkyl of 1 to 4 carbon atoms, alkoxycarbonyl of 2 to 5 carbon atoms, carboxyl, sulfamoyl, sulfo and alkylsulfonyl having an alkyl radical of 1 to 4 carbon atoms,
w is alkylene of 2 to 4 carbon atoms or is alkylene of 3 to 6 carbon atoms which is interrupted by 1 or 2 hetero-groups from the group consisting of the formulae -O-, -NH-, -SO₂-, -CO- and -N(R¹⁰)- where R¹⁰ is as defined in claim 1, or is phenylene or naphthylene, each of which may be substituted by 1, 2 or 3 substituents from the group consisting of halogen, hydroxyl, cyano, nitro, alkoxy of 1 to 4 carbon atoms, alkyl of 1 to 4 carbon atoms, alkoxycarbonyl of 2 to 5 carbon atoms, carboxyl, sulfamoyl and sulfo, or is phenylenealkylene or alkylenephenylene having an alkylene radical of in each case 1 to 4 carbon atoms, and
A is vinyl or is ethyl which is substituted in the β position by a substituent which can be eliminated using alkali to form the vinyl group.

7. An azo compound as claimed in at least one of claims 3 to 5, wherein Y is morpholino.

8. An azo compound as claimed in at least one of claims 3 to 5, wherein Y is mono- or disulfophenyl or mono-, di- or trisulfonaphthyl.

9. An azo compound as claimed in claim 3, wherein Z is a radical of the formula (8a) 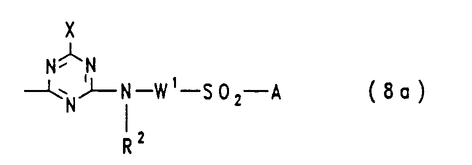 in which X is chlorine, fluorine or cyanoamino, A has one of the meanings given in claim 2, in particular one of the preferred definitions, R² is hydrogen, ethyl, methyl, β-hydroxyethyl, β-sulfatoethyl, phenyl, 3-sulfophenyl or 4-sulfophenyl and W¹ is 1,2-ethylene, 1,3-propylene, 2-methyl-1,2-ethylene, 2-methyl-l,3-propylene, 1,4-phenylene, 1,3-phenylene, 2-methoxy-1,5-phenylene, 2,5-dimethoxy-1,4-phenylene, 2-methoxy-5-methyl-1,4-phenylene, 2-sulfo-1,4-phenylene, 2-hydroxy-1,5-phenylene, 2-bromo-1,5-phenylene or 4-(2'-eth)phen-1,1'-ylene, in which groups position 1 is attached to the nitrogen atom, or is 1-sulfonaphth-2,6-ylene, 6-sulfonaphth-2,8-ylene, naphth-2,6-ylene or naphth-2,8-ylene, in which groups position 2 is attached to the nitrogen atom.

10. An azo compound as claimed in claim 1 of the formula (1A) 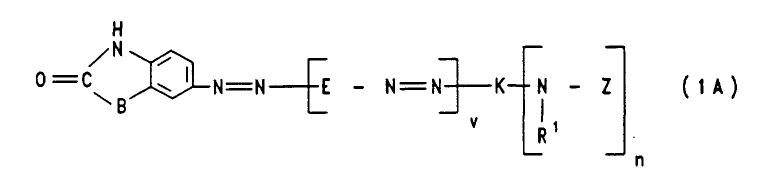 in which E, v, K, R¹ and n are as defined in claim 1, Z has one of the meanings given in at least one of claims 1 to 9 and B is -O- or -N(R¹⁰)-, in which R¹⁰ is hydrogen, phenyl, monosulfophenyl, disulfophenyl, trisulfophenyl, naphthyl, monosulfonaphthyl, disulfonaphthyl or trisulfonaphthyl.

11. An azo compound as claimed in claim 1 of the formula (1B) 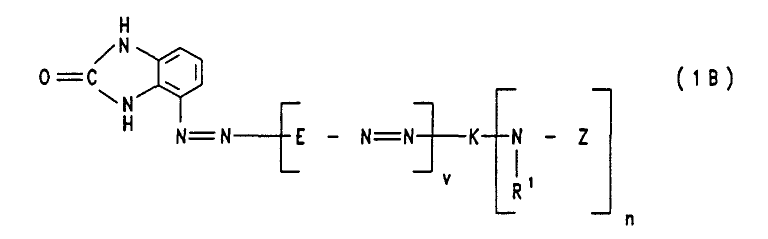 in which E, v, K, R¹ and n are as defined in claim 1 and Z has one of the meanings given in at least one of claims 1 to 9.

12. An azo compound as claimed in claim 1 of the formula (1C) 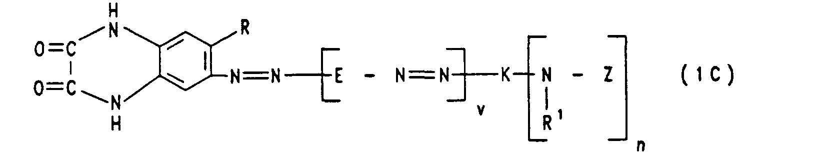 in which E, v, K, R¹ and n are as defined in claim 1 and Z has one of the meanings given in at least one of claims 1 to 9 and R is hydrogen, methyl or methoxy.

13. An azo compound as claimed in claim 1 of the formula (1E) 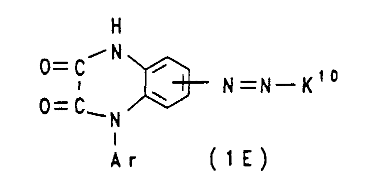 in which Ar is phenyl substituted by 1 or 2 sulfo groups or is naphthyl substituted by 1, 2 or 3 sulfo groups and K¹⁰ is a group of the formula (8A) 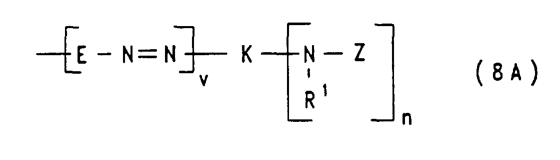 in which E, v, K, R¹ and n are as defined in claim 1 and Z has one of the meanings given in at least one of claims 1 to 9.

14. An azo compound as claimed in claim 1 of the formula (1F) 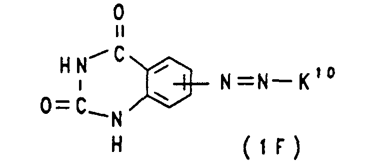 in which K¹⁰ is a group of the formula (8A) 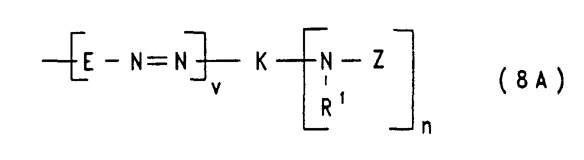 in which E, v, K, R¹ and n are as defined in claim 1 and Z has one of the meanings given in at least one of claims 1 to 9.

15. An azo compound as claimed in claim 1 of the formula (1G) 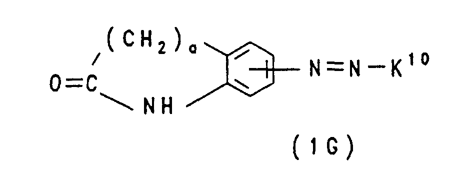 in which a is the number 1 or 2 and K¹⁰ is a group of the formula (8A) 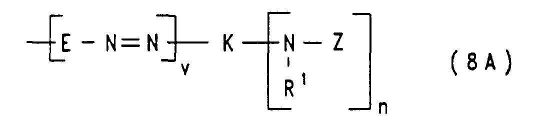 in which E, v, K, R¹ and n are as defined in claim 1 and Z has one of the meanings given in at least one of claims 1 to 9.

16. An azo compound as claimed in claim 1 of the formula (1H) 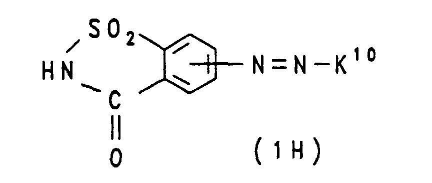 in which K¹⁰ is a group of the formula (8A) 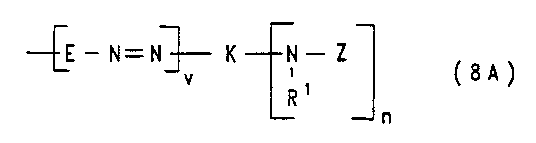 in which E, v, K, R¹ and n are as defined in claim 1 and Z has one of the meanings given in at least one of claims 1 to 9.

17. A process for the preparation of an azo compound of the formula (1) mentioned and defined in claim 1, which comprises diazotizing a compound of the formula (12) 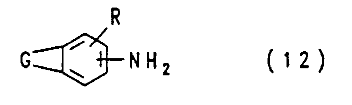 in which R and G are as defined in claim 1 and coupling the diazotization product with a compound of the formula (13) 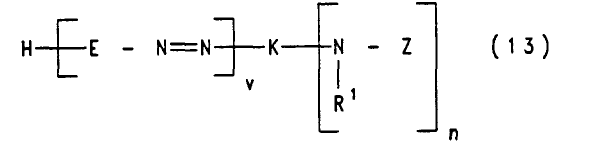 in which E, v, K, R¹, Z and n are as defined in claim 1, or comprises diazotizing a compound of the formula (14) 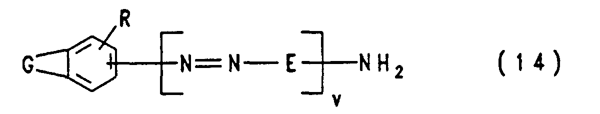 where G, R, E and v are as defined in claim 1, and coupling the diazotization product with a compound of the formula H-K-N(R¹)-Z where K, R¹ and Z are as defined in claim 1, or comprises reacting a compound of the formula (15) 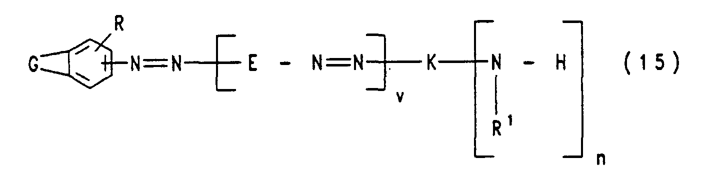 in which G, R, v, K, R¹ and n are as defined in claim 1 with a compound of the formula X⁰-Z in which X⁰ is chlorine, bromine or fluorine and Z is as defined in claim 1.

18. The use of a dye of claim 1 or a dye prepared as claimed in claim 17 for the dyeing (including printing) of material, in particular fiber material, which contains hydroxyl and/or carboxamido groups.

19. A process for the dyeing (including printing) of material, especially fiber material, which contains hydroxyl and/or carboxamido groups, in which a dye is applied to the material and the dye is fixed on the material by means of heat or by means of an alkaline agent or by means of both measures, wherein the dye employed is a dye of claim 1 or a dye prepared as claimed in claim 17.

## Revendications

1. Composé azoïque correspondant à la formule générale (1) 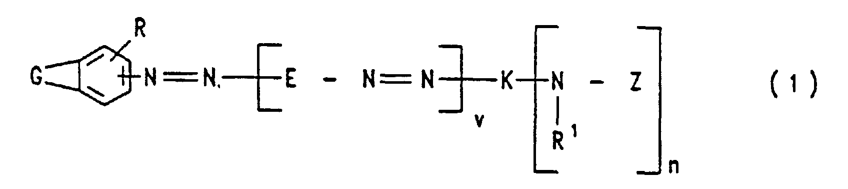 dans laquelle :
G forme, à côté du cycle benzénique, le radical d'un hétérocycle exempt de doubles liaisons oléfiniques, qui contient au moins un groupement carbonamide de formule -CO-N(R¹⁰)-, dans laquelle R¹⁰ est un hydrogène, un alkyle de 1 à 4 atomes de carbone ou, le cas échéant, un aryle de 6 à 10 atomes de carbone substitué par 1 à 3 substituants parmi le groupe des sulfo, carboxy, alcoxy de 1 à 4 atomes de carbone, alkyle de 1 à 4 atomes de carbone, halogène, cyano, nitro et amino et qui possède, le cas échéant, 1 ou 2 autres groupements à hétéroatome de la série-O-, -S- et -N(R¹⁰)- avec R¹⁰ ayant la signification susmentionnée;
R est un hydrogène, un alkyle de 1 à 4 atomes de carbone, un alcoxy de 1 à 4 atomes de carbone, un halogène ou un sulfo;
R¹ est un hydrogène, un alkyle de 1 à 4 atomes de carbone ou un alkyle de 1 à 4 atomes de carbone qui est substitué par un hydroxy, un cyano, un alcoxy de 1 à 4 atomes de carbone, un carboxy, un sulfo, un sulfato ou un phosphato, ou est un phényle ou un naphtyle, qui peuvent tous deux être substitués par 1, 2 ou 3 substituants parmi le groupe des halogène, hydroxy, cyano, alcoxy de 1 à 4 atomes de carbone, alkyle de 1 à 4 atomes de carbone, alcoxycarbonyle de 2 à 5 atomes de carbone, carboxy, sulfamoyle, sulfo et alkylsufonyle avec un radical alkyle de 1 à 4 atomes de carbone;
E est le radical divalent exempt de groupement amino d'un composé pouvant être diazoté et copulé, de la série aniline ou naphtylamine;
V signifie le nombre zéro, 1 ou 2;
K est le radical divalent exempt de groupement aminé d'un composant de copulation de la série aniline ou naphtylamine ou le radical divalent d'un composant de copulation de la série hétérocyclique;
n est le nombre 1, 2, 3 ou 4;
Z est le radical réagissant avec des fibres d'un groupement réagissant avec des fibres ou d'un groupement -N(R¹)-Z, les radicaux -N(R¹)-Z pouvant avoir des significations différentes les unes des autres dans le cas où n est plus grand que 1;
les composés de formule (1) possèdent au moins un groupement sulfo.

2. Composé azoïque suivant la revendication 1, caractérisé en ce que G est un radical de formule (2a), (2b), (2c), (2d), (2e), (2f), (2g) ou (2h) 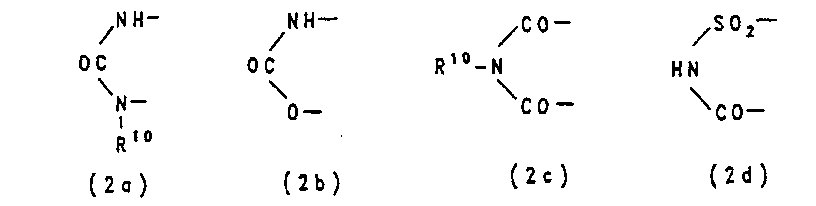 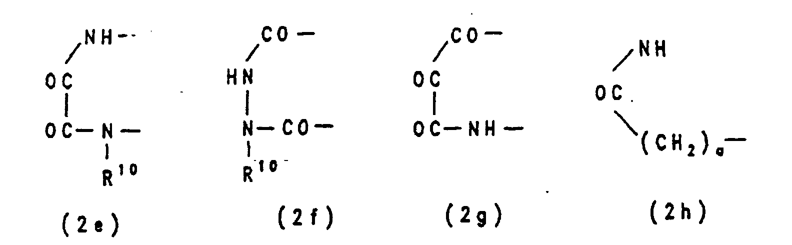 dans lesquelles R¹⁰ a la signification citée à la revendication 1 et, à la formule (2h), l'indice a signifie le nombre 1 ou 2.

3. Composé azoïque suivant la revendication 1, caractérisé en ce que Z est un radical de formule générale (3) 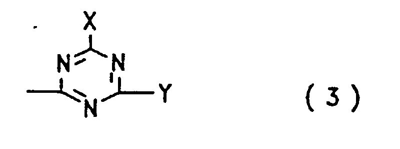 dans laquelle :
X est un halogène ou un groupement de formule générale (4) 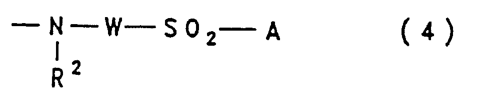
dans laquelle
R² signifie un hydrogène ou un alkyle de 1 à 4 atomes de carbone ou un alkyle de 1 à 4 atomes de carbone qui est substitué par un hydroxy, un cyano, un alcoxy de 1 à 4 atomes de carbone, un carboxy, un sulfo, un sulfato ou un phosphato, ou est un phényle ou un naphtyle qui peuvent tous deux être substitués par 1, 2 ou 3 substituants parmi le groupe des halogène, hydroxy, cyano, alcoxy de 1 à 4 atomes de carbone, alkyle de 1 à 4 atomes de carbone, alcoxycarbonyle de 2 à 5 atomes de carbone, carboxy, sulfamoyle, sulfo et alkylsulfonyle avec un radical alkyle de 1 à 4 atomes de carbone;
W est un alkylène de 2 à 4 atomes de carbone ou est un alkylène de 3 à 6 atomes de carbone qui est interrompu par 1 ou 2 groupements à hétéroatome parmi le groupe des formules -O-, -NH-, -SO₂-, -CO- et-N(R¹⁰)- avec R¹⁰ ayant la signification citée à la revendication 1, ou signifie un phénylène ou un naphtylène, qui peuvent tous deux être substitués par 1, 2 ou 3 substituants parmi le groupe des halogène, hydroxy, cyano, nitro, alcoxy de 1 à 4 atomes de carbone, alkyle de 1 à 4 atomes de carbone, alcoxycarbonyle de 2 à 5 atomes de carbone, carboxy, sulfamoyle et sulfo, ou est un phénylènealkylène ou un alkylènephénylène avec un radical alkylène de, chaque fois, 1 à 4 atomes de carbone, et
A est un vinyle ou signifie un éthyle, qui est substitué en position β par un substituant séparable au moyen d'un alcali avec formation d'un groupement vinyle;
Y est un chlore, un cyanoamino ou un groupement de formule générale (5) 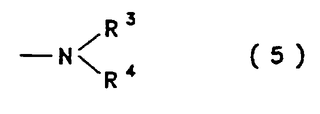
dans laquelle
R³ signifie un hydrogène ou un alkyle de 1 à 4 atomes de carbone ou un alkyle de 1 à 4 atomes de carbone qui est substitué par un halogène, un hydroxy, un cyano, un alcoxy de 1 à 4 atomes de carbone, un alcoxycarbonyle de 2 à 5 atomes de carbone, un carboxy, un sulfo, un sulfato ou un phoshato, ou est un groupement de formule -W-SO₂-A- avec W et A ayant l'une des significations susmentionnées;
R⁴ est un hydrogène ou un alkyle de 1 à 4 atomes de carbone ou signifie un alkyle de 1 à 4 atomes de carbone qui est substitué par un halogène, un hydroxy, un cyano, un alcoxy de 1 à 4 atomes de carbone, un alcoxycarbonyle de 2 à 5 atomes de carbone, un carboxy, un sulfo, un sulfato ou un phosphato, ou est un cycloalkyle de 5 à 8 atomes de carbone ou un groupement de formule -W-SO₂-A avec W et A ayant l'une des significations susmentionnées, ou est un phényle ou un naphtyle qui peuvent tous deux être substitués par 1, 2 ou 3 substituants parmi le groupe des halogène, hydroxy, cyano, alcoxy de 1 à 4 atomes de carbone, alkyle de 1 à 4 atomes de carbone, alcoxycarbonyle de 2 à 5 atomes de carbone, carboxy, sulfamoyle, sulfo et alkylsulfonyle avec un radical alkyle de 1 à 4 atomes de carbone, ou
R³ et R⁴ représentent ensemble un radical alkylène de 3 à 6 atomes de carbone ou un radical alkylène de 3 à 6 atomes de carbone interrompu par un groupement -NH-, -O-, -CO-, -S-, -SO₂- ou -N(R⁵)- (dans lequel R⁵ est un alkyle de 1 à 4 atomes de carbone substitué par un sulfo ou un sulfato, comme un éthyle), qui forment, avec l'atome d'azote, un radical hétérocyclique.

4. Composé azoïque suivant au moins l'une des revendications 1 à 3, caractérisé en ce que R¹ est un hydrogène, un méthyle ou un éthyle.

5. Composé azoïque suivant la revendication 3 ou 4, caractérisé en ce que X est un chlore ou un fluor.

6. Composé azoïque suivant au moins l'une des revendications 3 à 5, caractérisé en ce que Y est un groupement de formule générale (4) 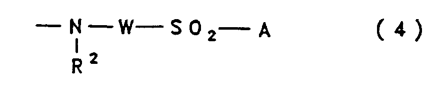 dans laquelle
R² est un hydrogène, un alkyle de 1 à 4 atomes de carbone ou un alkyle de 1 à 4 atomes de carbone qui est substitué par un hydroxy, un cyano, un alcoxy de 1 à 4 atomes de carbone, un carboxy, un sulfo, un sulfato ou un phosphato, ou est un phényle ou un naphtyle qui peuvent tous deux être substitués par 1, 2 ou 3 substituants parmi le groupe des halogène, hydroxy, cyano, alcoxy de 1 à 4 atomes de carbone, alkyle de 1 à 4 atomes de carbone, alcoxycarbonyle de 2 à 5 atomes de carbone, carboxy, sulfamoyle, sulfo et alkylsulfonyle avec un radical alkyle de 1 à 4 atomes de carbone;
W est un alkylène de 2 à 4 atomes de carbone ou est un alkylène de 3 à 6 atomes de carbone qui est interrompu par 1 ou 2 groupements à hétéroatome parmi le groupe des formules -O-, -NH-, -SO₂-, -CO- et-N(R¹⁰)- avec R¹⁰ ayant la signification citée à la revendication 1, ou signifie un phénylène ou un naphtylène, qui peuvent tous deux être substitués par 1, 2 ou 3 substituants parmi le groupe des halogène, hydroxy, cyano, nitro, alcoxy de 1 à 4 atomes de carbone, alkyle de 1 à 4 atomes de carbone, alcoxycarbonyle de 2 à 5 atomes de carbone, carboxy, sulfamoyle et sulfo, ou est un phénylènealkylène ou un alkylènephénylène avec un radical alkylène de, chaque fois, 1 à 4 atomes de carbone, et
A est un vinyle ou signifie un éthyle, qui est substitué en position β par un substituant séparable au moyen d'un alcali avec formation d'un groupement vinyle.

7. Composé azoïque suivant au moins l'une des revendications 3 à 5, caractérisé en ce que Y est un morpholino.

8. Composé azoïque suivant au moins l'une des revendications 3 à 5, caractérisé en ce que Y est un mono- ou un disulfophényle ou un mono-, di- ou trisulfonaphtyle.

9. Composé azoïque suivant la revendication 3, caractérisé en ce que Z est un radical de formule générale (8a) 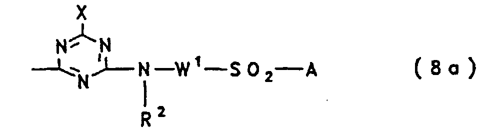 dans laquelle X est un chlore, un fluor ou un cyanoamino, A a, en particulier de préférence, l'une des significations citées à la revendication 2, R² est un hydrogène, un éthyle, un méthyle, un β-hydroxyéthyle, un β-sulfatoéthyle, un phényle, un 3-sulfophényle ou un 4-sulfophényle et W¹ est un 1,2-éthylène, un 1,3-propylène, un 2-méthyl-1,2-éthylène, un 2-méthyl-1,3-propylène, un 1,4-phénylène, un 1,3-phénylène, un 2-méthoxy-1,5-phénylène, un 2,5-diméthoxy-1,4-phénylène, un 2-méthoxy-5-méthyl-1,4-phénylène, un 2-sulfo-1,4-phénylène, un 2-hydroxy-1,5-phénylène, un 2-bromo-1,5-phénylène ou un 4-(2'-éthyl)-phén-1,1'-ylène, la position 1 étant liée à l'atome d'azote dans ces groupements, ou est un 1-sulfonapht-2,6-ylène, un 6-sulfonapht-2,8-ylène, un napht-2,6-ylène ou un napht-2,8-ylène, la position 2 étant liée à l'atome d'azote dans ces groupements.

10. Composé azoïque suivant la revendication 1 de formule générale (1A) 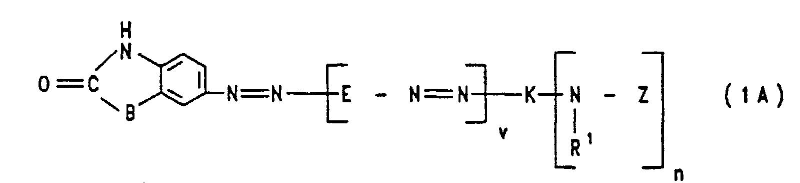 dans laquelle E, v, K, R¹ et n ont les significations citées à la revendication 1, Z possède l'une des significations citées dans au moins l'une des revendications 1 à 9 et B signifie -O- ou -N(R¹⁰)-, R¹⁰ étant un hydrogène, un phényle, un monosulfophényle, un disulfophényle, un trisulfophényle, un naphtyle, un monosulfonaphtyle, un disulfonaphtyle ou un trisulfonaphtyle.

11. Composé azoïque suivant la revendication 1 de formule générale (1B) 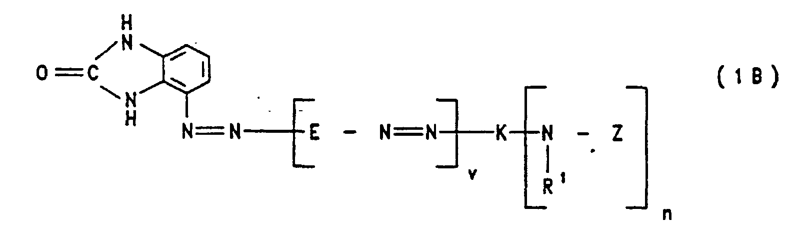 dans laquelle E, v, K, R¹ et n ont les significations citées à la revendication 1 et Z possède l'une des significations citées dans au moins l'une des revendications 1 à 9.

12. Composé azoïque suivant la revendication 1 de formule générale (1C) 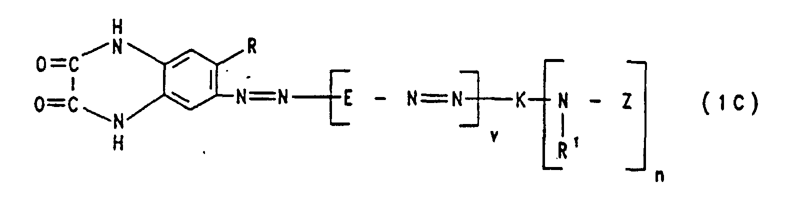 dans laquelle E, v, K, R¹ et n ont les significations citées à la revendication 1 et Z possède l'une des significations citées dans au moins l'une des revendications 1 à 9 et R est un hydrogène, un méthyle ou un méthoxy.

13. Composé azoïque suivant la revendication 1 de formule générale (1E) 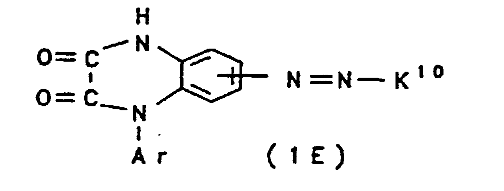 dans laquelle Ar est un phényle substitué par 1 ou 2 groupements sulfo ou un naphtyle substitué par 1, 2 ou 3 groupements sulfo et K¹⁰ est un groupement de formule générale (8A) 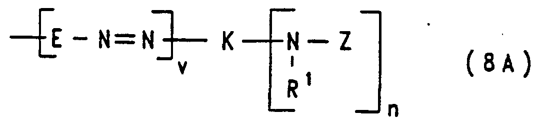 dans laquelle E, v, K, R¹ et n ont les significations citées à la revendication 1 et Z possède l'une des significations citées dans au moins l'une des revendications 1 à 9.

14. Composé azoïque suivant la revendication 1 de formule générale (1F) 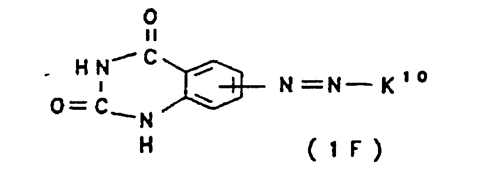 dans laquelle K¹⁰ est un groupement de formule générale (8A) 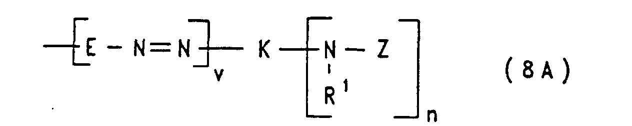 dans laquelle E, v, K, R¹ et n ont les significations citées à la revendication 1 et Z possède l'une des significations citées dans au moins l'une des revendications 1 à 9.

15. Composé azoïque suivant la revendication 1 de formule générale (1G) 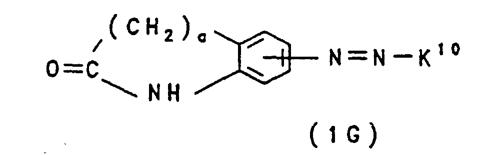 dans laquelle a est le nombre 1 ou 2 et K¹⁰ un groupement de formule générale (8A) 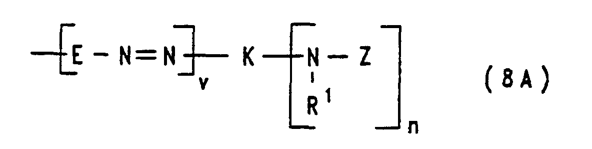 dans laquelle E, v, K, R¹ et n ont les significations citées à la revendication 1 et Z possède l'une des significations citées dans au moins l'une des revendications 1 à 9.

16. Composé azoïque suivant la revendication 1 de formule générale (1H) 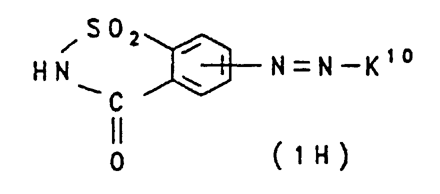 dans laquelle K¹⁰ est un groupement de formule générale (8A) 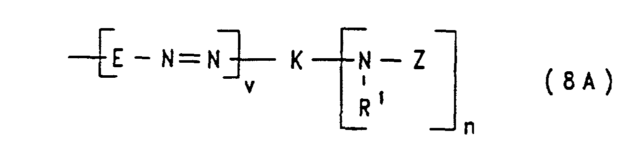 dans laquelle E, v, K, R¹ et n ont les significations citées à la revendication 1 et Z possède l'une des significations citées dans au moins l'une des revendications 1 à 9.

17. Procédé de préparation d'un composé azoïque de formule générale (1) définie et citée à la revendication 1, caractérisé en ce qu'un composé de formule générale (12) 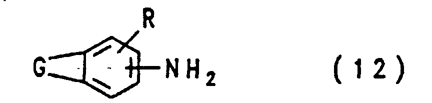 dans laquelle R et G ont l'une des significations citées à la revendication 1, est diazoté et est soumis à la copulation avec un composé de formule générale (13) 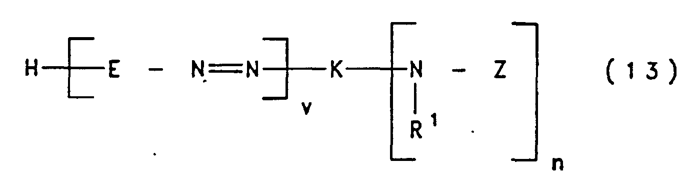 dans laquelle E, v, K, R¹, Z et n ont l'une des significations citées à la revendication 1, ou en ce qu'un composé de formule générale (14) 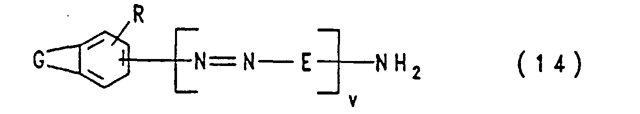 avec G, R, E et v ayant l'une des significations citées à la revendication 1, est diazoté et est soumis à la copulation avec un composé de formule générale H-K-N(R¹)-Z avec K, R¹ et Z ayant la signification citée à la revendication 1, ou en ce qu'un composé de formule générale (15) 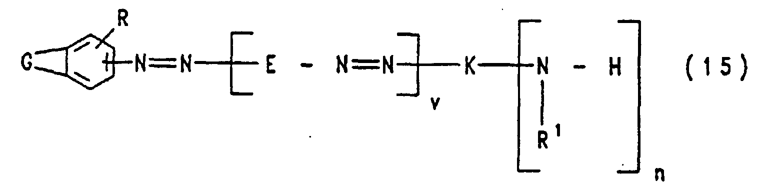 dans laquelle G, R, v, K, R¹ et n ont l'une des significations citées à la revendication 1, est transformé avec un composé de formule générale X⁰-Z, dans laquelle X⁰ signifie un chlore, un brome ou un fluor et Z possède la signification citée à la revendication 1.

18. Utilisation d'un colorant de la revendication 1 ou d'un colorant préparé suivant la revendication 17, pour teindre (y compris imprimer) du matériau contenant des groupements hydroxy et/ou carbonamide, en particulier un matériau fibreux.

19. Procédé pour teindre (y compris imprimer) du matériau contenant des groupements hydroxy et/ou carbonamide, en particulier un matériau fibreux, dans lequel on applique un colorant sur le matériau et on fixe thermiquement le colorant sur le matériau ou à l'aide d'un agent à action alcaline ou au moyen des deux mesures, caractérisé en ce que l'on utilise, comme colorant, un colorant de la revendication 1 ou un colorant préparé suivant la revendication 17.
